# EUROPEAN PATENT SPECIFICATION

(11) **EP 2 003 205 B1**
(45) Date of publication and mention of the grant of the patent: **01.05.2013**
(21) Application number: 08013040.4
(22) Date of filing: 28.12.2004
(51) Int. Cl.: C12N 15/82, C07K 14/415, A01H 5/00, A01H 5/10

(54) **Improved grain quality through altered expression of seed proteins**
Verbesserte Kornqualität durch Expressionsveränderung der Saatproteine
Qualité de grain améliorée par l'expression altérée des protéines des graines

(43) Date of publication of application: 17.12.2008
(62) Divisional of application: 04815600.4
(73) Proprietor: PIONEER HI-BRED INTERNATIONAL, INC., Johnston, Iowa 50131-1014 (US)
(72) Inventor: Jung, Ruldolf, Des Moines, IA 50311 (US); Hu, Wang-Nan, Johnston, IA 50131 (US); Meeley, Robert B., Des Moines, IA 50312 (US); Sewalt, Vincent J H, West Des Moines IA 50266 (US); Nair, Ramesh, Ankeny IA 50021 (US)
(74) Representative: Bentham, Andrew

(56) References cited:
- WO-A-98/26064
- WO-A-03/027249
- WO-A-2004/022759
- WO-A-2007/024207
- US-A- 5 850 024
- US-A- 5 929 311
- US-A1- 2004 194 171
- US-B1- 6 329 574
- AZEVEDO RICARDO A ET AL: "Regulation of maize lysine metabolism and endosperm protein synthesis by opaque and floury mutations." EUROPEAN JOURNAL OF BIOCHEMISTRY, vol. 270, no. 24, December 2003 (2003-12), pages 4898-4908, XP002502337 ISSN: 0014-2956
- DANNENHOFFER JOANNE M ET AL: "Opaque-15, a maize mutation with properties of a defective opaque-2 modifier" PROCEEDINGS OF THE NATIONAL ACADEMY OF SCIENCES OF THE UNITED STATES OF AMERICA, vol. 92, no. 6, 1995, pages 1931-1935, XP002518903 ISSN: 0027-8424
- HUANG S ET AL: "Improving nutritional quality of maize proteins by expressing sense and antisense zein genes" JOURNAL OF AGRICULTURAL AND FOOD CHEMISTRY, AMERICAN CHEMICAL SOCIETY. WASHINGTON, US, vol. 52, no. 7, 7 April 2004 (2004-04-07), pages 1958-1964, XP002339176 ISSN: 0021-8561
- LANDRY J ET AL: "Protein distribution pattern in floury and vitreous endosperm of maize grain." CEREAL CHEMISTRY, vol. 81, no. 2, March 2004 (2004-03), pages 153-158, XP009110813 ISSN: 0009-0352
- SEGAL G ET AL: "A new opaque variant of maize by a single dominant RNA-interference-inducing transgene" GENETICS, GENETICS SOCIETY OF AMERICA, AUSTIN, TX, US, vol. 165, no. 1, 1 September 2003 (2003-09-01), pages 387-397, XP002334753 ISSN: 0016-6731
- WU SHAOWEN ET AL: "Effects of maize hybrid and meal drying conditions on yield and quality of extracted zein" CEREAL CHEMISTRY, vol. 74, no. 3, 1997, pages 268-273, XP009108163 ISSN: 0009-0352
- WOO YOUNG-MIN ET AL: "Genomics analysis of genes expressed in maize endosperm identifies novel seed proteins and clarifies patterns of zein gene expression" PLANT CELL, vol. 13, no. 10, October 2001 (2001-10), pages 2297-2317, XP002518904 ISSN: 1040-4651

## Description

### FIELD OF THE INVENTION

The invention relates to the field of plant molecular biology and the use of genetic modification to improve the quality of crop plants, more particularly to improving the nutritional value of grain and the efficiency of grain processing.

### DETAILED DESCRIPTION OF THE INVENTION

The present invention is directed to maize plants with altered levels of seed proteins in plant seed, particularly reducing the levels of gamma-zein proteins in maize. Modification of seed protein composition causes changes in the physical and/or chemical properties of the grain.

Corn is a major crop used as a human food source, an animal feed, and as a source of carbohydrate, oil, protein and fiber. It is principally used as an energy source in animal feeds.

Most corn grain is handled as a commodity, since many of the industrial and animal feed requirements for corn can be met by common varieties of field corn which are widely grown and produced in volume. However, there exists at present a growing market for corn with special end-use properties which are not met by corn grain of standard composition.

The invention is directed to the alteration of protein composition and levels in plant seed, resulting in grain with increased digestibility, increased energy availability, improved amino acid composition, increased response to feed processing, improved silage quality, increased efficiency of wet or dry milling, and decreased anti-nutritional properties. The sequences disclosed herein encode proteins preferentially expressed during seed development.

Typically, "grain," means the mature kernel produced by commercial growers for purposes other than growing or reproducing the species, and "seed" means the mature kernel used for growing or reproducing the species. For the purposes of the present invention, "grain", "steed", and "kernel", will be used interchangeably.

Additionally, the invention is directed to altering seed hardness, decreasing seed caloric value for use in diet foods and other food for human use, pet food, increasing the antioxidant properties of seed, and taking advantage of the metal chelating properties of the corn legumin 1 protein to purify other polypeptides of interest and to increase iron and zinc content and bioavailability in the grain.

Nucleotides used in the invention comprise sequences encoding maize seed proteins and variants and fragments thereof. Inhibiting a seed protein may be achieved co-suppression methods including: RNA interference, gene activation or suppression using transcription factors and/or repressors to manipulate, in plants and plant seeds and grains, the expression of seed proteins, including, but not limited to, those encoded by the sequences disclosed herein.

Transgenic plants producing seeds and grain with altered seed protein content are provided.

The 50 kD gamma-zein relating to the instant invention maps to chromosome 7, bin 7.03, the 18 kD alpha-globulin to chromosome 6, bin 6.05, and the 50 kD legumin 1 to chromosome 6, Bin 6.01. This information, as well as the map location for 15kD beta zein, for 16kD and 27kD gamma-zein, the map location of members of the alpha-zein gene families, the map location for the 10kD and 18kD delta zeins (see Table 1 in Woo et al., 2001, Plant Cell 13:2297-2317) enables one of skill in the art to employ these map locations to generate improved maize lines with altered seed protein profiles and levels.

The proteins relating to the present invention have been designated for the purposes of this invention as "abundant seed proteins". In maize, a single species (that is a polypeptide encoded by a specific gene) or a group of similar species (that are protein family members with similar molecular properties like size) of these proteins make up 1% or more by dry weight of the total protein of seed and a single protein species or a group of similar species can be visualized by commonly used protein analytical methods such as gel electrophoresis and detection of proteins by staining of protein bands with Coomassie Blue or by liquid chromatography and detection of protein peaks by means of a UV detector (ref: Walker, JM, (2002) The Protein Protocol Handbook, second edition, Humana Press, Totowa, New Jersey). Although these proteins are "abundant" in the majority of maize lines (hereafter referred to simply as maize) they may be of low abundance or even absent in specific maize lines or can be transgenically manipulated to become suppressed.

Additionally, these proteins may originally not be "abundant" in grain from wild-type maize but be structurally related to proteins found abundantly in seeds of other plant species. For example, legumins are abundant proteins in legumes and rice but corn legumin is a protein of lower abundance in grain from common maize. Thus we refer to corn legumin as an "abundant seed protein". Traditionally "abundant seed proteins" as defined herein have also been called "seed storage proteins" as they are the major source for nitrogen and amino acids to provide nutrients for seedling growth and development.

These abundant seed proteins can occur as major seed proteins and minor seed proteins.

In maize, the major seed proteins are the alpha-zeins such as the 19 kDa and 22kDa alpha-zeins, and the gamma-zeins such as the 27kDa gamma-zein protein.

Zeins are typically prolamins; that is they are typically characterized by being extractable in 70% ETOH and a reducing agent (see Woo et al., 2001, Plant Cell 13:2297-2317, and Shewry and Casey (eds.) (1999) Seed Proteins 141-157, Academic Publishers, Dordrecht.). A zein can also be identified phylogenetically through the use of sequence analysis.

The alpha-zeins are a family of related proteins that typically comprise 10-50% of the total protein (based on dry weight) in the grain, i.e. they are major seed proteins. This protein family is further comprised of two 19kD alpha-zein protein subfamilies and one 22kD alpha-zein protein subfamily (Woo *et al*). The chromosomal loci (genomic sequence) of the alpha-zein gene subfamilies have been sequenced in their entirety for a common maize inbred line and are known to the art (ref. Song R., Messing J, (2002) Plant Physiol, Vol. 130, pp. 1626-1635, Song R, Llaca V, Linton E, Messing J. (2001) Genome Res. 11, pp. 1817-25).

The gamma-zeins are a family of related proteins that typically make up 10-15% of the total seed proteins (based on dry weight). The structure and characteristics of this family are exemplified by the 16kD gamma-zein, the 27 kD gamma-zein - which are major seed proteins - and the 50kDa gamma-zein - a minor seed protein. The 15kD beta-zein is a minor seed protein and belongs to this family as well (Woo et al).

Non-zein abundant seed proteins in maize include the corn legumins and alpha-globulins. The corn legumins and the corn alpha-globulins are minor seed proteins in maize; the name designation of both proteins are based on their phylogentic relationship to seed proteins from other species (Woo et al). Seed proteins have been traditionally characterized based on solubility characteristics (Shewry and Casey (eds.) (1999) Seed Proteins, 141-157, Academic Publishers, Dordrecht). Thus most seed proteins are either extractable in aqueous alcoholic solutions (prolamins), extractible in aqueous solutions of low ionic strength (albumins), or extractable in aqueous solutions of high ionic strength (globulins). The classification of seed proteins by extraction methods is well known in the art (Shewry and Casey (1999)). However it is also common to designate seed proteins with unknown extraction characteristics as globulins, albumins, or prolamins if they are phylogenetically or sequence-related to proteins that have originally been classified based on extraction experiments. Therefore, it is a common practice to name seed proteins based on their phylogenetic association rather then their extraction properties. The name of a seed protein gene may therefore not reflect the properties of the encoded protein in a strict sense.

It has been recently discovered that down regulation or inhibition of the zein proteins alone or in combination increases digestibility and the energy availability of cereal grain such as corn and sorghum.

Additionally, the novel discovery has been made that the up regulation (or overexpression) of the non-zein proteins increases digestibility of cereal grain.

Accordingly, the present invention provides:
(1) A genetically modified maize plant wherein said genetic modification is selected from:
   (a) co-suppression or homology-dependent gene silencing of a 27 kD gamma-zein and said plant has a stably decreased level of a 27 kD and 16 kD gamma-zein protein in the grain of the modified maize plant due to said modification; and
   (b) co-suppression or homology-dependent gene silencing of a 50 kD gamma-zein and said plant has a stably decreased level of a 27 kD, 50 kD and 16 kD gamma-zein protein in the grain of the modified maize plant due to said modification;
      and wherein the grain of the genetically modified maize plant has a hard, vitreous endosperm.
(2) Seed or grain of the plant of (1) having decreased level of a 27 kD and 16 kD gamma-zein protein compared to seed or grain of the corresponding unmodified plant.
(3) Seed or grain of the plant of (1) having decreased level of 27 kD, 50 kD gamma-zein protein, and 16 kD gamma-zein protein compared to seed or grain of the corresponding unmodified plant.
   The invention further provides:
(4) A plant according to (1) further comprising increased expression, compared to the corresponding unmodified plant, of a nucleotide sequence that encodes the maize 18 kD alpha-globulin or a 50 kD legumin protein.
(5) A plant according to (1) or (4) further comprising increased expression, compared to the corresponding unmodified plant, of a nucleotide sequence that encodes the maize 18 kD delta zein protein.
(6) Seed or grain of the plant of (4) having increased levels of maize 18 kD alpha-globulin or maize 50 kD legumin protein compared to seed or grain of the corresponding unmodified plant.
(7) Seed or grain of the plant of (5) having increased levels of maize 18 kD delta zein protein compared to seed or grain of the corresponding unmodified plant.

In one embodiment of the present invention, zein proteins are down regulated in combination with over expression of non-zein proteins to produce a synergistic effect of increased digestibility of cereal grain.

The present invention may use isolated nucleic acid molecules comprising a nucleotide sequence encoding a maize protein, designated herein as the 50 kD gamma-zein, having the amino acid sequence shown in SEQ ID NO:2. The nucleic acid molecules of SEQ ID NO:1 and fragments and variants thereof may also be used.

The present invention may also use isolated nucleic acid molecules comprising a nucleotide sequence encoding a maize protein, herein designated as the 18 kD alpha-globulin, having the amino acid sequence shown in SEQ ID NO:4. Further described is a polypeptide having an amino acid sequence encoded by the nucleic acid molecules described herein, for example that set forth in SEQ ID NO:3, and fragments and variants thereof.

The present invention may also use isolated nucleic acid molecules comprising a nucleotide sequence encoding a maize protein, herein designated as the 50 kD legumin 1 protein, having the amino acid sequence shown in SEQ ID NO:6. Further described is a polypeptide having an amino acid sequence encoded by the nucleic acid molecules described herein, for example that set forth in SEQ ID NO:5, and and fragments and variants thereof.

A plasmid containing the nucleotide sequence encoding the 50 kD gamma-zein protein was deposited with the Patent Depository of the American Type Culture Collection (ATCC), Manassas, Virginia, on July 26, 2000 and assigned Patent Deposit No. PTA-2272. A plasmid containing the nucleotide sequence encoding the 18 kD alpha-globulin protein was deposited with the Patent Depository of the American Type Culture Collection (ATCC), Manassas, Virginia, on July 26, 2000 and assigned Patent Deposit No. PTA-2274. A plasmid containing the nucleotide sequence encoding the 50 kD legumin 1 protein was deposited with the Patent Depository of the American Type Culture Collection (ATCC), Manassas, Virginia, on July 26, 2000 and assigned Patent Deposit No. PTA-2273. These deposits will be maintained under the terms of the Budapest Treaty on the International Recognition of the Deposit of Microorganisms for the Purposes of Patent Procedure. These deposits were made merely as a convenience for those of skill in the art and are not an admission that a deposit is required under 35 U.S.C. §112.

A comparison of the amino acid content of cereal grains shows that 18 kD alpha-globulin is an excellent source of tryptophan and methionine for amino acid balance in all cereals and that 50 kD corn legumin 1 is an excellent source of methionine for all cereals and a good source of lysine and tryptophan for the amino acid balance of most cereals.

The present invention may use isolated nucleotide sequences comprising transcriptional units for gene over -expression and gene-suppression that have been used either as single units or in combination as multiple units to transform plant cells.

As used herein in connection with abundant seed proteins, "biologically active" means a protein that folds, assemble and interacts with other proteins, is available as a nitrogen source for seed germination and accumulates (ie: synthesis exceeds deposition) during seed development.

An "isolated" or "purified" nucleic acid molecule or protein, or biologically active portion thereof, is substantially or essentially free from components that normally accompany or interact with the nucleic acid molecule or protein as found in its naturally occurring environment. Thus, an isolated or purified nucleic acid molecule or protein is substantially free of other cellular material, or culture medium when produced by recombinant techniques, or substantially free of chemical precursors or other chemicals when chemically synthesized. Preferably, an "isolated" nucleic acid is free of sequences (preferably protein encoding sequences) that naturally flank the nucleic acid (i.e., sequences located at the 5' and 3' ends of the nucleic acid) in the genomic DNA of the organism from which the nucleic acid is derived. For example, the isolated nucleic acid molecule can contain less than about 5 kb, 4 kb, 3 kb, 2 kb, 1 kb, 0.5 kb, or 0.1 kb of nucleotide-sequences that naturally flank the nucleic acid molecule in genomic DNA of the cell from which the nucleic acid is derived.

Fragments and variants of the disclosed nucleotide sequences may also be used in the present invention. By "fragment" is intended a portion of the nucleotide sequence. Fragments of a nucleotide sequence may encode protein fragments that retain the biological activity of the targeted gene. Alternatively, fragments of a nucleotide sequence that are useful as hybridization probes generally do not encode fragment proteins retaining biological activity. In addition, fragments may be used to inhibit the expression of a targeted gene product of interest. Thus, fragments of a nucleotide sequence may range from at least about 10 nucleotides, about 50 nucleotides, about 100 nucleotides, and up to the full-length nucleotide sequence-encoding native alpha-zein proteins, native-gamma-zein proteins, native delta-zein proteins, the native 50 kD gamma-zein, the 18 kD alpha-globulin, or the 50 kD legumin 1 protein of the invention. Similarly, fragments of a nucleotide sequence that are useful for generating cells, tissues or plants transiently or permanently suppressing a gene or genes may not encode fragment proteins retaining biological activity. Fragments may be in sense or antisense or reverse orientation or a combination thereof. Thus, for example, fragments of such nucleotide sequence may range from at least about 10 nucleotides, at least about 20 nucleotides, about 50 nucleotides, about 100 nucleotides, and up to the full-length nucleotide sequence encoding native alpha-zein proteins, native gamma-zein proteins, native delta-zein proteins, the 50 kD gamma-zein, the 18 kD alpha-globulin, or the 50 kD legumin 1 protein.

Fragments of the maize nucleotide sequences used in the invention (SEQ ID NO:1, SEQ ID NO:3, or SEQ ID NO:5) that encode a biologically active portion of the 50 kD gamma-zein protein, the 18 kD alpha-globulin protein, or the 50 kD legumin 1 protein, respectively, will encode at least 15, 25, 30, 50, 100, 150, or 200 contiguous amino acids, or up to the total number of amino acids present in the full-length 50 kD gamma-zein protein, the 18 kD alpha-globulin protein, or the 50 kD legumin 1 protein (for example, 295 amino acids for SEQ ID NO:1; 206 amino acids for SEQ ID NO:3; and 483 amino acids for SEQ ID NO:5). Fragments of SEQ ID NO:1, SEQ ID NO:3, and SEQ ID NO:5 that are useful as hybridization probes or PCR primers need not encode a biologically active portion of a prolamin protein.

Thus, a fragment of SEQ ID NO:1, SEQ ID NO:3, or SEQ ID NO:5 may encode a biologically active portion of a prolamin or globulin protein, or it may be a fragment that can be used as a hybridization probe or PCR primer using methods disclosed below or it may be used to inhibit the expression of the protein. A biologically active portion of the 50 kD gamma-zein protein, the 18 kD alpha-globulin protein, or the 50 kD legumin 1 protein can be prepared by isolating a portion of the disclosed nucleotide sequence that codes for a portion of the 50 kD gamma-zein protein, the 18 kD alpha-globulin protein, or the 50 kD legumin 1 protein (e.g., by recombinant expression *in vitro*), and assessing the activity of the encoded portion of the prolamin protein. Nucleic acid molecules that are fragments of SEQ ID NO:1 comprise at least 40, 50, 75, 100, 150, 200, 250, 300, 350, 400, 450, 500, 550, 600, 650, 700, 800, 900, 1000, or 1100 nucleotides, or up to the number of nucleotides present in the full-length gamma-zein cDNA (for example, 1129 nucleotides for SEQ ID NO:1). Nucleic acid molecules that are fragments of SEQ ID NO:3 comprise at least 40, 50, 75, 100, 150, 200, 250, 300, 350, 400, 450, 500, 550, 600, 650, 700, 800, or 900 nucleotides, or up to the number of nucleotides present in the full-length alpha-globulin cDNA (for example, 950 nucleotides for SEQ ID NO:3). Nucleic acid molecules that are fragments of SEQ ID NO:5 comprise at least 40, 50,-75, 100, 150, 200, 250, 300, 350, 400, 450, 500, 550, 600, 650, 700, 800, 1000, 1200, 1400, or 1600 nucleotides, or up to the number of nucleotides present in the full-length legumin 1 cDNA (for example, 1679 nucleotides for SEQ ID NO:5).

By "variants" is intended substantially similar sequences. For nucleotide sequences, conservative variants include those sequences that, because of the degeneracy of the genetic code, encode the amino acid sequence of 50 kD gamma-zein protein, the 18 kD alpha-globulin protein, or the 50 kD legumin 1 protein. Naturally occurring allelic variants such as these can be identified with the use of well-known molecular biology techniques, as, for example, with polymerase chain reaction (PCR) and hybridization techniques as outlined below. Variant nucleotide sequences also include synthetically derived nucleotide sequences, such as those generated, for example, by using site-directed mutagenesis but which still-encode a 50 kD gamma-zein protein, an 18 kD alpha-globulin protein, or an 50 kD legumin 1 protein. Generally, variants of a particular nucleotide sequence will have at least about 60%, 65%, 70%, 75%, 80%, 85%, 90%, 91%, 92%, 93%, 94%, 95%, 96%, 97%, 98%, or 99% or more sequence identity to that particular nucleotide sequence over a length of 20, 30, 50, or 100 nucleotides or less, as determined by sequence alignment programs described elsewhere herein using default parameters.

By "variant" protein is intended a protein derived from the native protein by deletion (so-called truncation) or addition of one or more amino acids to the N-terminal and/or C-terminal end of the native protein; deletion or addition of one or more amino acids at one or more sites in the native protein; or substitution of one or more amino acids at one or more sites in the native protein. Variant proteins relating to the present invention are biologically active, that is they continue to possess all or some of the activity of the native proteins as described herein. Such variants may result from, for example, genetic polymorphism or from human manipulation. Biologically active variants of the native 50 kD gamma-zein protein, the 18 kD alpha-globulin protein, or the 50 kD legumin 1 protein of the invention will have at least about 60%, 65%, 70%, 75%, 80%, 85%, 90%, 91%, 92%, 93%, 94%, 95%, 96%, 97%, 98%, or 99% or more sequence identity to the amino-acid sequence for the native protein over a length of 10, 30, 50, or 100 amino acid residues or less as determined by sequence alignment programs described elsewhere herein using default parameters. A biologically active variant of a protein may differ from that protein by as few as 1-15 amino acid residues, as few as 1-10, such as 6-10, as few as 5, as few as 4, 3, 2, or even 1 amino acid residue.

The proteins may be altered in various ways including amino acid substitutions, deletions, truncations, and insertions. Methods for such manipulations are generally known in the art. For example, amino acid sequence variants of the 50 kD gamma-zein protein, the 18 kD alpha-globulin protein, or the 50 kD legumin 1 protein can be prepared by mutations in the DNA. Methods for mutagenesis and nucleotide sequence alterations are well known in the art. See, for example, Kunkel (1985) Proc. Natl. Acad. Sci. USA 82:488-492; Kunkel et al. (1987) Methods in Enzymol. 154:367-382; US Patent No. 4,873,192; Walker and Gaastra, eds. (1983) Techniques in Molecular Biology (MacMillan Publishing Company, New York) and the references cited therein. Guidance as to appropriate amino acid substitutions that do not affect biological activity of the protein of interest may be-found in the model of Dayhoff et al. (1978) Atlas of Protein Sequence and Structure (Natl. Biomed. Res. Found., Washington, B.C.) .

Thus, the genes and nucleotide sequences used in the invention include both the naturally occurring sequences as well as mutant forms. Likewise, proteins relating to the invention encompass both naturally occurring variant proteins as well as variations and modified forms thereof. Such variants will continue to be biologically active as defined herein. Obviously, the mutations that will be made in the DNA encoding the variant must not place the sequence out of reading frame and preferably will not create complementary regions that could produce secondary mRNA structure. See EP Patent Application Publication No. 75,444.

Variant nucleotide sequences and proteins also encompass sequences and proteins derived from a mutagenic and recombinogenic procedure such as DNA shuffling. With such a procedure, one or more different alpha-zein, delta-zein, beta-zein, gamma-zein, alpha-globulin, or legumin protein coding sequences can be manipulated to create a new alpha-zein, delta-zein, beta-zein, gamma-zein, alpha-globulin, or legumin protein possessing the desired properties. In this manner, libraries of recombinant polynucleotides are generated from a population of related sequence polynucleotides comprising sequence regions that have substantial sequence identity and can be homologously recombined *in vitro* or *in vivo.* For example, using this approach, sequence motifs encoding a domain of interest may be shuffled between 50 kD gamma-zein coding sequence, the 18 kD alpha-globulin coding sequence, or the 50 kD legumin 1 protein coding sequence and other known gene coding sequences to obtain a new coding sequence for a protein with an improved property of interest. Strategies for such DNA shuffling are known in the art. See, for example, Stemmer (1994) Proc. Natl. Acad. Sci. USA 91:10747-10751; Stemmer (1994) Nature 370:389-391; Crameri et al. (1997) Nature Biotech. 15:436-438; Moore et al. (1997) J. Mol. Biol. 272:336-347; Zhang et al. (1997) Proc. Natl. Acad. Sci. USA 94:4504-4509; Crameri et al. (1998) Nature 391:288-291; and U.S. Patent Nos. 5,605,793 and 5,837,458.

The following terms are used to describe the sequence relationships between two or more nucleic acids or polynucleotides: (a) "reference sequence", (b) "comparison window", (c) "sequence identity", (d) "percentage of sequence identity", and (e) "substantial identity".
(a) As used herein, "reference sequence" is a defined sequence used as a basis for sequence comparison. A reference sequence may be a subset or the entirety of a specified sequence; for example, as a segment of a full-length cDNA or gene sequence, or the complete cDNA or gene sequence.
(b) As used herein, "comparison window" makes reference to a contiguous and specified segment of a polynucleotide sequence, wherein the polynucleotide sequence in the comparison window may comprise additions or deletions (i.e., gaps) compared to the reference sequence (which does not comprise additions or deletions) for optimal alignment of the two sequences. Generally, the comparison window is at least 20 contiguous nucleotides in length, and optionally can be 30, 40, 50, 100, or longer. Those of skill in the art understand that to avoid a high similarity to a reference sequence due to inclusion of gaps in the polynucleotide sequence a gap penalty is typically introduced and is subtracted from the number of matches.

Methods of alignment of sequences for comparison are well known in the art. Thus, the determination of percent sequence identity between any two sequences can be accomplished using a mathematical algorithm. Non-limiting examples of such mathematical algorithms are the algorithm of Myers and Miller (1988) CABIOS 4:11-17; the local homology algorithm of Smith et al. (1981) Adv. Appl. Math. 2:482; the homology alignment algorithm of Needleman and Wunsch (1970) J. Mol. Biol. 48:443-453; the search-for-similarity-method of Pearson and Lipman (1988) Proc. Natl. Acad. Sci. 85:2444-2448; the algorithm of Karlin and Altschul (1990) Proc. Natl. Acad. Sci. USA 872264, modified as in Karlin and Altschul (1993) Proc. Natl. Acad. Sci. USA 90:5873-5877.

Computer implementations of these mathematical algorithms can be utilized for comparison of sequences to determine sequence identity. Such implementations include, but are not limited to: CLUSTAL in the PC/Gene program (available from Intelligenetics, Mountain View, California); the ALIGN program (Version 2.0) and GAP, BESTFIT, BLAST, FASTA, and TFASTA in the Wisconsin Genetics Software Package, Version 8 (available from Genetics Computer Group (GCG), 575 Science Drive, Madison, Wisconsin, USA). Alignments using these programs can be performed using the default parameters. The CLUSTAL program is well described by Higgins et al. (1988) Gene 73:237-244 (1988); Higgins et al. (1989) CABIOS 5:151-153; Corpet et al. (1988) Nucleic Acids Res. 18:10881-90; Huang et al. (1992) CABIOS 8:155-65; and Pearson et al. (1994) Meth. Mol. Biol. 24:307-331. The ALIGN program is based on the algorithm of Myers and Miller (1988) supra. A PAM120 weight residue table, a gap length penalty of 12, and a gap penalty of 4 can be used with the ALIGN program when comparing amino acid sequences. The BLAST programs of Altschul et al (1990) J. Mol. Biol. 215:403 are based on the algorithm of Karlin and Altschul (1990) *supra.* BLAST nucleotide searches can be performed with the BLASTN program, score = 100, wordlength = 12, to obtain nucleotide sequences homologous to a nucleotide sequence encoding a protein of the invention. BLAST protein searches can be performed with the BLASTX program, score = 50, wordlength = 3, to obtain amino acid sequences homologous to a protein or polypeptide of the invention. To obtain gapped alignments for comparison purposes, Gapped BLAST (in BLAST 2.0) can be utilized as described in Altschul et al. (1997) Nucleic Acids Res. 25:3389. Alternatively, PSI-BLAST (in BLAST 2.0) can be used to perform an iterated search that detects distant relationships between molecules. See Altschul et al. (1997) *supra.* When utilizing BLAST, Gapped BLAST, PSI-BLAST, the default parameters of the respective programs (e.g., BLASTN for nucleotide sequences, BLASTX for proteins) can be used. Alignment may also be performed manually by inspection.

Unless otherwise stated, sequence identity/similarity values provided herein refer to the value obtained using GAP version 10 using the following parameters: % identity using GAP Weight of 50 and Length Weight of 3; % similarity using Gap Weight of 12 and Length Weight of 4, or any equivalent program, aligned over the full length of the sequence. By "equivalent program" is intended any sequence comparison program that, for any two sequences in question, generates an alignment having identical nucleotide or amino acid residue matches and an identical percent sequence identity when compared to the corresponding alignment generated by GAP Version 10.

GAP uses the algorithm of Needleman and Wunsch (1970) J. Mol. Biol. 48:443-453, to find the alignment of two complete sequences that maximizes the number of matches and minimizes the number of gaps. GAP considers all possible alignments and gap positions and creates the alignment with the largest number of matched bases and the fewest gaps. It allows for the provision of a gap creation penalty and a gap extension penalty in units of matched bases. GAP must make a profit of gap creation penalty number of matches for each gap it inserts. If a gap extension penalty greater than zero is chosen, GAP must, in addition, make a profit for each gap inserted of the length of the gap times the gap extension penalty. Default gap creation penalty values and gap extension penalty values in Version 10 of the Wisconsin Genetics Software Package for protein sequences are 8 and 2, respectively. For nucleotide sequences the default gap creation penalty is 50 while the default gap extension penalty is 3. The gap creation and gap extension penalties can be expressed as an integer selected from the group of integers consisting of from 0 to 200. Thus, for example, the gap creation and gap extension penalties can be 0, 1, 2, 3, 4, 5, 6, 7, 8, 9, 10, 15, 20, 25, 30, 35, 40, 45, 50, 55, 60, 65 or greater.

GAP presents one member of the family of best alignments. There may be many members of this family, but no other member has a better quality. GAP displays four figures of merit for alignments: Quality, Ratio, Identity, and Similarity. The Quality is the metric maximized in order to align the sequences. Ratio is the quality divided by the number of bases in the shorter segment. Percent Identity is the percent of the symbols that actually match. Percent Similarity is the percent of the symbols that are similar. Symbols that are across from gaps are ignored. A similarity is scored when the scoring matrix value for a pair of symbols is greater than or equal to 0.50, the similarity threshold. The scoring matrix used in Version 10 of the Wisconsin Genetics Software Package is BLOSUM62 (see Henikoff and Henikoff (1989) Proc. Natl. Acad. Sci. USA 89:10915).
(c) As used herein, "sequence identity" or "identity" in the context of two nucleic acid or polypeptide sequences makes reference to the residues in the two sequences that are the same when aligned for maximum correspondence over a specified comparison window. When percentage of sequence identity is used in reference to proteins it is recognized that residue positions which are not identical often differ by conservative amino acid substitutions, where amino acid residues are substituted for other amino acid residues with similar chemical properties (e.g., charge or hydrophobicity) and therefore do not change the functional properties of the molecule. When sequences differ in conservative substitutions, the percent sequence identity may be adjusted upwards to correct for the conservative nature of the substitution. Sequences that differ by such conservative substitutions are said to have "sequence similarity" or "similarity". Means for making this adjustment are well known to those of skill in the art. Typically this involves scoring a conservative substitution as a partial rather than a full mismatch, thereby increasing the percentage sequence identity. Thus, for example, where an identical amino acid is given a score of 1 and a non-conservative substitution is given a score of zero, a conservative substitution is given a score between zero and 1. The scoring of conservative substitutions is calculated, e.g., as implemented in the program PC/GENE (Intelligenetics, Mountain View, California).
(d) As used herein, "percentage of sequence identity" means the value determined by comparing two optimally aligned sequences over a comparison window, wherein the portion of the polynucleotide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison, and multiplying the result by 100 to yield the percentage of sequence identity.
(e)(i) The term "substantial identity" of polynucleotide sequences means that a polynucleotide comprises a sequence that has at least 60%, 65%, 70%, 75%, 80%, 85%, 90%, 91%, 92%, 93%, 94%, 95%, 96%, 97%, 98%, or 99% or more sequence identity compared to a reference sequence using one of the alignment programs described using standard parameters. One of skill in the art will recognize that these values can be appropriately adjusted to determine corresponding identity of proteins encoded by two nucleotide sequences by taking into account codon degeneracy, amino acid similarity, reading frame positioning, and the like. Substantial identity of amino acid sequences for these purposes normally means sequence identity of at least 60%, 65%, 70%, 75%, 80%, 85%, 90%, 91%, 92%, 93%, 94%, 95%, 96%, 97%, 98%, or 99% or more.

Another indication that nucleotide sequences are substantially identical is if two molecules hybridize to each other under stringent conditions. Generally, stringent conditions are selected to be about 5°C lower than the thermal melting point (Tₘ) for the specific sequence at a defined ionic strength and pH. However, stringent conditions encompass temperatures in the range of about 1°C to about 20°C lower than the Tₘ, depending upon the desired degree of stringency as otherwise qualified herein. Nucleic acids that do not hybridize to each other under stringent conditions are still substantially identical if the polypeptides they encode are substantially identical. This may occur, e.g., when a copy of a nucleic acid is created using the maximum codon degeneracy permitted by the genetic code. One indication that two nucleic acid sequences are substantially identical is when the polypeptide encoded by the first nucleic acid is immunologically cross reactive with the polypeptide encoded by the second nucleic acid.
(e)(ii) The term "substantial identity" in the context of a peptide indicates that a peptide comprises a sequence with at least 60%, 65%, 70%, 75%, 80%, 85%, 90%, 91%, 92%, 93%, 94%, 95%, 96%, 97%, 98%, or 99% or more sequence identity to the reference sequence over a specified comparison window. Alignment can be conducted using the homology alignment algorithm of Needleman and Wunsch (1970) J. Mol. Biol. 48:443-453. An indication that two peptide sequences are substantially identical is that one peptide is immunologically reactive with antibodies raised against the second peptide. Peptides that are "substantially similar" comprise a sequence with at least 60%, 65%, 70%, 75%, 80%, 85%, 90%, 91%, 92%, 93%, 94%, 95%, 96%, 97%, 98%, or 99% or more sequence identity or sequence similarity to the reference sequence over a specified comparison window. In this case residue positions that are not identical instead differ by conservative amino acid changes.

The 50 kD gamma-zein nucleotide sequence cloned from a maize endosperm cDNA library (Example 1) and disclosed herein (SEQ ID NO:1) displays sequence similarity to the other two described corn gamma-zein genes, 27 kD gamma-zein (represented herein by GenBank Accession No. P04706) and 16 kD gamma-zein (represented herein by GenBank Accession No. AAA33523). The 50 kD gamma-zein was named due to its apparent molecular weight by migration in SDS-PAGE. This cDNA encodes a 295 amino acid protein and also shows sequence similarity to seed proteins of other plant species. For example, wheat alpha-gliadin (GenBank -ID:TAU51305, Accession No. U51305). The 50 kD gamma-zein DNA sequences isolated from different inbred lines showed an unusually low level of polymorphism. Only one single nucleotide polymorphism (SNP) (a 3 bp insertion) was detected along the entire cDNA sequence from DNA isolated from the inbred lines Mo17 and B73. The 50 kD gamma-zein gene has been located on chromosome 7, bin 7.03.

The 18 kD alpha-globulin nucleotide sequence was also cloned from a maize endosperm cDNA library (Example 5) and is disclosed herein (SEQ ID NO:3). The 18 kD alpha-globulin was named due to its similarity to a rice seed globulin (rice alpha-globulin, GenBank Accession No. D50643). This cDNA encodes a 206 amino acid protein. Unlike the case of the 50 kD gamma-zein, there is no other abundantly expressed maize gene known closely related to alpha-globulin in maize. The 18 kD alpha-globulin cDNA shows sequence similarity to seed proteins from other cereals including rice, wheat, and oats and distantly to maize proteins with the conserved domain pfam00234.11 (see below). Different maize inbred lines showed considerable allelism in the 18 kD alpha-globulin gene including SNP's. The 18 kD alpha-globulin gene has been located on chromosome 6, bin 6.05.

Alpha-globulins are accumulated in seed storage tissues during seed development and serve as nitrogen reserves for the growing seedling during germination. They are homologues of abundant seed proteins in other plants (e.g. rice). These seed proteins can therefore be categorized as abundant seed proteins.

Corn alpha-globulin belongs to a plant protein family that is preferentially expressed in seed. This protein family contains the conserved domain pfam00234.11, Tryp_alpha_amyl.

Description: Trypsin-alpha-amylase inhibitor domain, Alpha-Amylase Inhibitor (AAI) subgroup. These cereal-type alpha-amylase inhibitors are composed of 120-160 residues, form 5 disulfide bonds and inhibit amylases from birds, bacilli, insects and mammals. They are related to the other members of the AAI family (plant lipid transfer proteins and seed storage proteins), the disulfide-bonding pattern varies between members.

Polypeptides belonging to this protein family contain three regions with conserved cysteine residues. Members of this gene family from other plant species (e.g. puroindulin from wheat, GenBank Account No. gi_509109) are only about 25% identical to alpha-globulin, but shares the conserved cysteines.

Corn alpha-globulin and rice alpha-globulin (Account No. JC4784) are 46% identical on the amino acid level, but are 80% -100% identical in the cysteine domains.

### Domain 1:

The first of these regions (SPLDACRQVLDRQLTG) is 100% identical between rice and corn over 16 aa residues. The Cys residue followed by an Arg residue is conserved also in other members of this protein family found in other plant species.

### Domain 2:

The second of these regions (CCQQLQDVSRECRCAAIR) is 100% identical between rice and corn over 18 aa residues. The two consecutive cysteine residues followed by 9 amino acids and a CysArgCys tripeptide are conserved in other members of this protein family.

The 50 kD legumin 1 nucleotide sequence was also cloned from a maize endosperm cDNA library (Example 8) and is disclosed herein (SEQ ID NO:5). The 50 kD legumin 1 was named due to its similarity to 11 S globulins found in other plant species: the so-called legumins. The 50 kD legumin 1 appears to be encoded by a single gene in the maize genome. It belongs to the 11S globulin superfamily and is closely related to legumins from other cereals (also called glutenins in rice and wheat or globulins in oat) and dicot plants. The 50 kD legumin polypeptide sequence is missing the evolutionary conserved 11 S globulin pro-protein proteolytic site (Asn-Gly bond between the acidic chain and basic chain legumin regions) which makes it unique among the legumin protein superfamily. This cDNA encodes a 483 amino acid protein with a predicted N-terminal endoplasmic reticulum import signal peptide of 36 amino acids. The 50 kD legumin 1 DNA sequences isolated from different inbred lines showed a considerable level of polymorphism. The 50 kD legumin 1 gene has been mapped to chromosome 6, Bin 6.01.

The chromosomal location of the genes corresponding to the known maize seed proteins and the cDNA's described above are known (see Woo et al, *et seq*) or have been determined as stated above. Knowing the map position of a gene is important and useful if it correlates with a trait, as is the case for the encoded polypeptides described herein. Certain alleles of these genes can, for instance, have an impact on seed hardness, starch extractability, energy availability, etc as is described in detail *infra*. Considerable knowledge has been accumulated regarding the so called Quantitative Trait Loci (QTL). Linkage of a gene to a QTL is of significance regarding the impact of this gene on the corresponding trait. Further, the map position can be used for marker assisted breeding, which is a very economical and time saving way to introduce alleles into elite germplasm. Alternatively, SNP's can also be used to screen a wide variety of germplasm for advantageous alleles.

The 50 kD gamma-zein protein relating to the present invention displays a high cysteine content and is therefore predicted to have a high number of disulfide bonds or high "disulfide status", as is observed for the other gamma-zein proteins. By "disulfide status" is intended the portion of cysteine residues within a protein that participate in disulfide bonds or disulfide bridges. Such disulfide bonds can be formed between the sulfur of a first cysteine residue and the sulfur of a second cysteine residue. It is recognized that such first and second cysteine residues can occur as part of a single polypeptide chain, or alternatively, can occur on separate polypeptide chains referred to herein as "inter-molecular disulfide bonds". When "disulfide status" is used in reference to a seed or part thereof, the "disulfide status" of such a seed or part thereof is the total disulfide status of the proteins therein.

The disulfide-rich, gamma-zein protein fraction in corn has been implicated as a major determinant of the poor amino acid content of this grain which contributes to its low nutrient content. In addition, as a result of the high-disulfide status of this gamma-zein fraction of corn endosperm it can also be a significant contributor to the wet-milling properties of corn grain. For example, in the wet-milling process, the higher the number of disulfide bonds, the greater the requirement for chemical reductants to break these bonds and to maximize the release of starch granules. It is believed that extensive disulfide bonding negatively impacts the process of wet-milling.

The intermolecular disulfide bridges of the gamma-zeins, along with the hydrophobic beta-zein, and alpha- and delta-zeins, are also important for the formation and maintenance of protein bodies. These protein bodies contribute to the physical properties of the grain that also affect the wet-milling process. In the wet-milling process, chemical reductants are required to break protein disulfide bonds to maximize starch yield and quality (Hoseney, R.C. (1994), Principles of Cereal Science and Tech., (Ed.2)). The use in wet mills of odorous chemical such as sulfur dioxide and bisulfite requires extensive precautions and poses significant environmental problems.

Similar to that described for a decrease in the number of disulfide bonds, a decrease in the number of protein bodies can also be expected to improve the efficiency of the wet-milling process. Zein proteins interact during formation of protein bodies (through intermolecular disulfide bonds and hydropobic interactions), and these interactions are important for the formation of proteolytically stable complexes. Though not limited by any theory of action, a decrease in the expression of two or three gamma-zein genes can be expected to have an additive effect on the reduction of protein bodies resulting in a corresponding improvement in wet-milling properties.

The wet-milling properties of the corn grain of the present invention can be analyzed using a small-scale simulated wet-milling process incorporating or leaving out a reducing agent (bisulfite) in the steep water as used by Eckhoff et al., (1996, Cereal Chem. 73:54-57).

In addition to the positive impact that reducing agents have on the release of starch granules in the wet-milling process, it has also been shown that reducing agents can increase the dry matter digestibility of sorghum and corn and, thus, improve their feed properties. This result is supported by the results of data from *in vitro* digestibility assays described in the present invention (Examples 2-4) that demonstrate that reducing agents increase the dry matter digestibility or energy availability of corn. See also: Hamaker, B.R., et al., 1987, Improving the in vitro protein digestibility of sorghum with reducing agents, Proc. Natl. Acad. Sci. USA 84:626-628.

The "energy value", or "caloric value" of a feed or food, which is determined by energy density or gross energy (GE) content and by energy availability, is also termed "metabolizable energy (ME) content." (see Wiseman, J., and Cole, D.J.A., (1987), Animal Production 45(1):117-122)

As used herein, "energy availability" means the degree to which energy-rendering nutrients are available to the animal, often referred to as energy conversion (ratio of metabolizable energy content to gross energy content). One way energy availability may be determined is with in vivo balance trials, in which excreta are collected by standard methodology (e.g., Sibbald, I.R., Poultry Science, 58(5):1325-29 (1979); McNab and Blair, British Poultry Science 29(4):697-708 (1988)). Energy availability is largely determined by food or feed digestibility in the gastro-intestinal tract, although other factors such as absorption and metabolic utilization also influence energy availability.

"Digestibility" is defined herein as the fraction of the feed or food that is not excreted as feces. Digestibility is a component of energy availability. It can be further defined as digestibility of specific constituents (such as carbohydrates or protein) by determining the concentration of these constituents in the foodstuff and in the excreta. Digestibility can be estimated using in vitro assays, which is routinely done to screen large numbers of different food ingredients and plant varieties. In vitro techniques, including assays with rumen inocula and/or enzymes for ruminant livestock (e.g. Pell and Schofield, Journal of Dairy Science 76(4):1063-1073 (1993)) and various combinations of enzymes for monogastric animals reviewed in Boisen and Eggum, Nutrition Research Reviews 4:141-162 (1991) are also useful techniques for screening transgenic materials for which only limited sample is available.

The enzyme digestible dry matter (EDDM) assay used in these experiments as an indicator of *in vivo* digestibility is known in the art and can be performed according to the methods described in Boisen and Fernandez (1997) Animal Feed Science and Technology 68:277-286, and Boisen and Fernandez (1995) Animal Feed Science and Technology 51:29-43 . The actual in vitro method used for determining EDDM in this patent application is a modified version of the above protocol as described in Example 2. These data indicate that reducing the number of disulfide bonds in the seed of corn can increase the dry matter digestibility of grain from this crop while retaining a "normal" i.e.: vitreous phenotype. It is also likely that a decrease in the disulfide-status of other grains would have a similar positive effect on their digestibility properties.

While seed with extensive disulfide bonding exhibits poor wet-milling properties and decreased dry matter digestibility, a high disulfide-status has also been correlated with increased seed hardness and improved dry-milling properties. In fact, the transcript level of the 50 kD maize gamma-zein gene has been shown to be largely affected in several opacity mutants (o2, o5, and o9) and in opaque hordothionin-12 (US Patent 5,990,389) corn. These data indicate that this 50 kD maize gamma-zein is a good gene candidate for altering other grain quality traits such as grain hardness. Assays for seed hardness are well known in the art and include such methods as those used in the present invention, described in Pomeranz et al. (1985) Cereal Chemistry 62:108-112 .

Based on its amino acid sequence, the 18 kD alpha-globulin can also be expected to have a high number of disulfide bonds and to participate in intermolecular protein cross-linking. For this reason, over-expression of the 18 kD alpha-globulin protein can be predicted to increase seed hardness. The ability to confer seed hardness is particularly useful in the case of soft kernel phenotypes that are induced by mutation or transgenic polypeptides. An increase in the levels of the 18 kD alpha-globulin can be used as a method for improving the dry-milling properties of soft kernel phenotypes.

In addition to its high cysteine content, the 18 kD alpha-globulin protein also possesses a relatively high percentage of the essential amino acids tryptophan (4.6% by weight, cysteine (5.1% by weight), and methionine (3.9% by weight). For this reason, transgenic over-expression of the 18 kD alpha-globulin protein can be expected to significantly increase the percentage of tryptophan and sulfur-containing amino acids in corn grain and, thus, increase the nutritional value of the grain.

The "nutritional value" of a feed or food is defined as the ability of that feed or food to provide nutrients to animals or humans. The nutritional value is determined by 3 factors: concentration of nutrients (protein & amino acids, energy, minerals, vitamins, etc.), their physiological availability during the processes of digestion, absorption and metabolism, and the absence (or presence) of anti-nutritional compounds.

Similar to the 18 kD alpha-globulin, the 50 kD legumin 1 protein also possesses a relatively high percentage of essential amino acids. This protein contains 6.7%, 0.7%, 2.2%, 1.1%, 3.6%, and 2.7% by weight of lysine, tryptophan, methionine, cysteine, isoleucine, and threonine, respectively. For this reason, transgenic over-expression of the 50 kD legumin 1 protein can also be expected to increase the nutritional value of the grain.

In addition to its desirable amino acid content, the 50 kD legumin 1 protein is assembled differently than other legumin polypeptides. As a result of the missing proteolytic cleavage site, the 50 kD legumin 1 protein is not cleaved into acidic and basic chains. Instead this legumin assembles into 9S polypeptide primers (presumably in the endoplasmic reticulum) and does not undergo assembly into 11S globulin hexamers. The assembly properties of this 50 kD legumin 1 polypeptide could contribute to unique food processing properties of protein extracts from seed expressing this protein. For example, the 50kD legumin 1 polypeptide could be ectopically expressed in soybean seed and protein isolates from corresponding soybean seed display altered functionalities such as solubility under acidic conditions, improved water-holding capacity and the like.

Another feature of the 50 kD legumin 1 polypeptide is a string of histidine residues that can function as a metal binding site. Native 50 kD legumin 1 polypeptide binds with high affinity to nickel chelation columns. This property can be used to purify corn legumin 1 in bulk from complex protein mixtures and to purify other polypeptides of interest through the production of fusion proteins. The metal chelation properties of the 50 kD legumin 1 polypeptide could also be of importance for bio-remediation or food health (antioxidant) applications. Additionally, in cereal grain such as maize transgenically overexpressing the 50 kD legumin 1 polypeptide, the Zn and Fe chelating properties of the 50 kD legumin 1 polypeptide may result in an increased concentrations of Zn and Fe in the grain and in increased bio-availability of these micro-nutrient from the diet.

Similar to the over expression of the 18 kD alpha-globulin maize grain, the over-expression of the 50 kD corn legumin 1 protein unexpectedly also resulted in a significant increase of grain digestibility (See Example 11). Endosperm from transgenic corn grain over-expressing corn either alpha-globulin or corn legumin was investigated by immuno- Electron Microscopy (EM). Both transgenic proteins were found to accumulate in non-zein storage organelles, which appear greatly enhanced in number and in size in the transgenic endosperm samples, compared to control EM images obtained from endosperm from non-transgenic corn. Thus the investigation of endosperm structure for similar changes in protein storage organelle number and sizes may be used to select transgenic corn lines transformed with unrelated seed proteins or foreign non-seed protein to screen for events carrying a highly digestible endosperm trait.

The invention relates to the modulation of the levels of at least one seed protein in seeds. By "modulate" is defined herein as an increase or decrease in the level of a seed protein within seed of a genetically manipulated plant relative to the level of that protein in seed from the corresponding wild-type plant (i.e., a plant not genetically manipulated in accordance with the methods of the present invention).

The terms "inhibit," "inhibition," "inhibiting", "reduced", "reduction" and the like as used herein refer to any decrease in the expression or function of a target gene product, including any relative decrement in expression or function up to and including complete abrogation of expression or function of the target gene product. The term "expression" as used herein in the context of a gene product refers to the biosynthesis of that gene product, including the transcription and/or translation of the gene product. Inhibition of expression or function of a target gene product (i.e., a gene product of interest) can be in the context of a comparison between any two plants, for example, expression or function of a target gene product in a genetically altered plant versus the expression or function of that target gene product in a corresponding wild-type plant. Alternatively, inhibition of expression or function of the target gene product can be in the context of a comparison between plant cells, organelles, organs, tissues, or plant parts within the same plant or between plants, and includes comparisons between developmental or temporal stages within the same plant or between plants.

The term "inhibitory sequence" encompasses any polynucleotide or polypeptide sequence that is capable of inhibiting the expression of a target gene product, for example, at the level of transcription or translation, or which is capable of inhibiting the function of a target gene product. Examples of inhibitory sequences include, but are not limited to, full-length polynucleotide or polypeptide sequences, truncated polynucleotide or polypeptide sequences, fragments of polynucleotide or polypeptide sequences, variants of polynucleotide or polypeptide sequences, sense-oriented nucleotide sequences, antisense-oriented nucleotide sequences, the complement of a sense- or antisense-oriented nucleotide sequence, inverted regions of nucleotide sequences, hairpins of nucleotide sequences, double-stranded nucleotide sequences, single-stranded nucleotide sequences, combinations thereof, and the like. The term "polynucleotide sequence" includes sequences of RNA, DNA, chemically modified nucleic acids, nucleic acid analogs, combinations thereof, and the like.

Inhibitory sequences are designated herein by the name of the target gene product. Thus, for example, an "27 kD gamma zein inhibitory sequence" would refer to an inhibitory sequence that is capable of inhibiting the expression of 27 kD gamma zein, for example, at the level of transcription and/or translation, or which is capable of inhibiting the function of 27 kD gamma zein. When the phrase "capable of inhibiting" is used in the context of a polynucleotide inhibitory sequence, it is intended to mean that the inhibitory sequence itself exerts the inhibitory effect; or, where the inhibitory sequence encodes an inhibitory nucleotide molecule (for example, hairpin RNA, miRNA, or double-stranded RNA polynucleotides), or encodes an inhibitory polypeptide (i.e., a polypeptide that inhibits expression or function of the target gene product), following its transcription (for example, in the case of an inhibitory sequence encoding a hairpin RNA, miRNA, or double-stranded RNA polynucleotide) or its transcription and translation (in the case of an inhibitory sequence encoding an inhibitory polypeptide), the transcribed or translated product, respectively, exerts the inhibitory effect on the target gene product (i.e., inhibits expression or function of the target gene product).

Conversely, the terms "increase, " "increased, " and "increasing" in the context of the present invention refer to any increase in the expression or function of a gene product, including any relative increment in expression or function. As with inhibition, increases in the expression or function of a gene product of interest (i.e., a target gene product) can be in the context of a comparison between any two plants, for example, expression or function of a target gene product in a genetically altered plant versus the expression or function of that target gene product in a corresponding wild-type plant. Alternatively, increases in the expression or function of the target gene product can be in the context of a comparison between plant cells, organelles, organs, tissues, or plant parts within the same plant or between plants, and includes comparisons between developmental or temporal stages within the same plant or between plants. Any composition that up-regulates expression of a target gene product, either at the level of transcription or translation, or up-regulates functional activity of the target gene product can be used to achieve increased expression or function of the target gene product. For example, a method that increases oil production in a plant can be any method that increases the total oil content or the percent oil content of that plant relative to that observed in another plant, for example a comparison between a genetically modified plant and a corresponding wild-type plant, any method that increases oil content of a cell, organelle, organ, tissue, or plant part relative to a different cell, organelle, organ, tissue, or plant part within the same plant or between two plants, or any method that increases oil content of a cell, organelle, organ, tissue, plant part, or whole plant relative to that observed during different developmental or temporal stages within the same plant or another plant.

The invention is particularly directed to reducing the level of the 16 kD, the 27 kD protein and the 50 kD gamma-zein proteins to improve the nutritional value and industrial use of grain. In one embodiment, the levels of alpha-zeins and of gamma-zeins are reduced in maize grain resulting in an increase in digestibility. Another embodiment is directed to the reduction of the gamma zein seed proteins and the concurrent increase of the levels of the alpha-globulin protein or the corn legumin 1 protein to incrementally or synergistically improve the grain digestibility.

Reduction of the level of the 16 kD, the 27 kD protein or the 50 kD gamma-zein proteins in plant seed can be used to improve the nutritional value and industrial use of such grain. The invention can be useful for producing grain that is more rapidly and extensively digested than grain with normal/wild-type gamma-zein protein levels.

Because the inhibition of the 27 kD gamma-zein trait is dominant or semi-dominant, improvements in grain digestibility can be obtained by introducing it into specific pollinators (i.e., high oil corn) using conventional methods and/or the top-cross technology found in US Patent No. 5,704,160. In addition, reducing the levels of other seed proteins, such as beta-zein, in conjunction with inhibition of one or more gamma-zein genes can result in further grain improvement including improved digestibility.

Thus, inhibition of gamma-zein genes can be used to increase the nutritional value of seed, particularly by increasing the energy availability of seed. Reduction in the gamma-zein levels in such seed can be at least about 20%, 30%, 40%, 50%, 60%, 70%, 80%, 90% and up to 100%. Energy availability can be improved by at least 3%, 6%, 9%, 12%, 15%, 20% and greater.

Reduction of the level of alpha-zein proteins in plant seed can be used to improve the nutritional value and industrial use of such grain. The methods are also useful for producing grain that is more rapidly and extensively digested than grain with normal gamma-zein protein levels.

Inhibition of alpha-zein genes can be used to increase the nutritional value of seed, particularly by increasing the energy availability of seed. Reduction in the alpha-zein levels in such seed can be at least about 20%, 30%, 40%, 50%, 60%, 70%, 80%, 90% and up to 100%. Energy availability can be improved by at least 3%, 6%, 9%, 12%, 15%, 20% and greater.

It has been discovered that the improvement in energy availability by inhibition of alpha-zeins is in part independent on the levels of gamma-zein or interaction with gamma-zein proteins. Thus the combined inhibition of alpha-zein and gamma-zein proteins (either in breeding crosses or in independent transgenic events and/or in mutants or by means of stacked transgenic constructs) shows an increase in digestibility greater than inhibition of either alpha-zein or gamma-zein protein alone.

Similarly, the combination of inhibiting gamma- zein proteins with/without alpha-zein inhibition, with over-expressing either, or both, 18kD alpha-globulin or 50kD corn legumin 1, leads to an increase in grain digestibility greater than inhibition of alpha- and gamma-zein proteins alone or increasing levels of 18kD alpha-globulin and/or 50kD corn legumin 1 alone.

It is noted that modifications made to the grain by the present invention typically do not compromise grain handling properties with respect to mechanical damage: taking into account that grain handling procedures are adapted to specific properties of the modified grain. Mechanical damage to grain is a well-described phenomenon (e.g., McKenzie, B.A., Am Soc Ag Engineers (No: 85-3510): 10pp, 1985) that contributes to dust in elevators and livestock facilities, and which may increase susceptibility to pests. Grain damage can be quantified and assessed by objective measures (e.g., Gregory, J.M., et al., Am Soc. Ag. Engineers (no.91-1608): 11pp, 1991) such as kernel density and test weight. See also: McKenzie, B.A. 1985, *supra.*

The invention also encompasses modulation of an 18 kD alpha-globulin protein or a corn legumin 1 protein (in addition to gamma-zein inhibition) to affect the nutritional value and/or the hardness of plant seed. An increase in the levels of these proteins in plant seed would result in an increase in the nutritional value of the seed. The levels of the maize 18 kD alpha-globulin protein (SEQ ID NO:4) can be increased in maize seed, resulting in seed that can be predicted to possess at least about 20%, 30%, 40%, 50%, 60%, 70%, 80%, 90%, 100%, 110%, 120%, 130%, 140%, 150%, and up to a 300% increase in tryptophan and sulfur-containing amino acids relative to grain of wild-type plants. The level of the corn legumin 1 protein can be similarly increased in maize seed to increase the level of essential amino acids in the grain. Food products and feed based on such seed will have a higher nutritional value based on the increased levels of essential amino acids.

In addition to the increase in nutritional value, an increase in the level of the 18 kD alpha-globulin protein in plant seed can be predicted to result in grain possessing altered hardness. This is due to an increase in non-zein protein accumulated in non-zein storage organelles relative to grain from wild-type plants, and has applications for improving the dry-milling properties of such modified grain. Introduction of this trait into corn plants with inferior kernel phenotypes, particularly inferior kernel phenotypes induced by the introduction of other transgenic polypeptides including, but not limited to, hordothionin 12 (US Patent No. 5,990,389), can ameliorate or eliminate the undesirable dry-milling properties of such grain by altering seed hardness.

In another embodiment, the levels of the 50 kD legumin 1 polypeptide are increased (in addition to gamma-zein inhibition) in cereal grain for the purpose of increasing the metal chelating properties of the grain. The unique string of histidine residues present in the 50 kD legumin 1 polypeptide function as a metal chelating site. Products produced from such grain could be used for bio-remediation, in food health (antioxidant) applications, or in biofortification (increase of zinc and iron bioavailability).

Accordingly, the invention relates to reducing the level of the 50 kD gamma-zein (SEQ ID NO:2),), the 27 kD gamma-zein (Accession No. P04706), the 16 kD gamma-zein (Accession No. AAA33523) in maize seed or grain, and may also include modulation of the15 kD beta-zein (Accession No. P06673) the delta zeins (Woo, et al) the alpha-zeins (Song et al, 2002, 2001), the 18 kD alpha-globulin (SEQ ID NO:4), and the corn legumin 1 (SEQ ID NO:6).

Methods for inhibiting gene expression are well known in the art. Although any method know in the art for reducing the level of protein in a plant could be used, possible methods for reducing protein include, but are not limited to, homology-dependent gene silencing, antisense technology, co-suppression including, for example, RNA interference (RNAi), micro RNA and the like, site-specific recombination, site-specific integration, mutagenesis including transposon tagging, and biosynthetic competition, homologous recombination, and gene targeting, alone or in combination. Depending upon the intended goal, the level of at least one seed protein may be increased, decreased, or eliminated entirely as described below.

According to the present invention, the 50 kD and 27 kD gamma-zeins are targeted by co-suppression or homology-dependent gene silencing methods. The level of other seed protein found within maize, including but not limited to, the alpha-, beta-, delta-zeins of maize, and alpha-globulins of maize, the legumin 1 and other seed proteins of maize may be altered by the methods disclosed herein.

In many instances the nucleotide sequences used in the present invention are provided in transcriptional units with for transcription in the plant of interest. A transcriptional unit is comprised generally of a promoter and a nucleotide sequence operably linked in the 3' direction of the promoter, optionally with a terminator.

By "operably linked" is intended a functional linkage between a promoter and a second sequence, wherein the promoter sequence initiates and mediates transcription of the DNA sequence corresponding to the second sequence. The expression cassette will include 5' and 3' regulatory sequences operably linked to at least one of the sequences of the invention.

Generally, in the context of an over expression cassette, operably linked means that the nucleotide sequences being linked are contiguous and, where necessary to join two or more protein coding regions, contiguous and in the same reading frame. In the case where an expression cassette contains two or more protein coding regions joined in a contiguous manner in the same reading frame, the encoded polypeptide is herein defined as a "heterologous polypeptide" or a "chimeric polypeptide" or a "fusion polypeptide". The cassette may additionally contain at least one additional coding sequence to be co-transformed into the organism. Alternatively, the additional coding sequence(s) can be provided on multiple expression cassettes.

The methods of transgenic expression can be used to increase the level of at least one seed protein in grain. The methods of transgenic expression comprise transforming a plant cell with at least one expression cassette comprising a promoter that drives expression in the plant operably linked to at least one nucleotide sequence encoding a seed protein. Methods for expressing transgenic genes in plants are well known in the art.

In other instances the nucleotide sequences for use in the invention are provided in transcriptional units as co-supression cassettes for transcription in the plant of interest. Transcription units can contain coding and/or non-coding regions of the genes of interest. Additionally, transcription units can contain promoter sequences with or without coding or non-coding regions. The co-suppression cassette may include 5' (but not necessarily 3') regulatory sequences, operably linked to at least one of the sequences of the genes of interest. Co-supression cassettes used in the methods can comprise sequences in so-called "inverted repeat" structures. The cassette may additionally contain a second copy of the fragment in opposite direction to form an inverted repeat structure: opposing arms of the structure may or may not be interrupted by any nucleotide sequence related or unrelated to the nucleotide sequences disclosed herein. (see Fiers et al. US Pat No: 6,506,559). The transcriptional units are linked to be co-transformed into the organism. Alternatively, additional transcriptional units can be provided on multiple over-expression and co-suppression cassettes.

The methods of transgenic co-suppression can be used to reduce or eliminate the level of at least one seed protein in grain. One method of transgenic co-suppression comprise transforming a plant cell with at least one transcriptional unit containing an expression cassette comprising a promoter that drives transcription in the plant operably linked to at least one nucleotide sequence transcript in the sense orientation encoding at least a portion of the seed protein of interest. Methods for suppressing gene expression in plants using nucleotide sequences in the sense orientation are known in the art. The methods generally involve transforming plants with a DNA construct comprising a promoter that drives transcription in a plant operably linked to at least a portion of a nucleotide sequence that corresponds to the transcript of the endogenous gene. Typically, such a nucleotide sequence has substantial sequence identity to the sequence of the transcript of the endogenous gene, at least about 65%, 70%, 75%, 80%, 85%, 90%, 91%, 92%, 93%, 94%, 95%, 96%, 97%, 98%, or 99% or more sequence identity over the entire length of the sequence. Furthermore, portions, rather than the entire nucleotide sequence, of the polynucleotides may be used to disrupt the expression of the target gene product. Generally, sequences of at least 10, 15, 20, 21, 22, 23, 24, 25, 26, 27, 28, 29, 30, 35, 40, 45, 50, 55, 60, 65, 70, 75, 80, 85, 90, 95, 100, 200 nucleotides, or greater may be used. See U.S. Patent Nos. 5,283,184 and 5,034,323.

The endogenous gene targeted for co-suppression may be a gene encoding any seed protein that accumulates as a seed protein in the plant species of interest, including, but not limited to, the seed genes noted above. For example, where the endogenous gene targeted for co-suppression is the 50 kD gamma-zein gene disclosed herein, co-suppression is achieved using an expression cassette comprising the 50 kD gamma-zein gene sequence, or variant or fragment thereof.

Additional methods of co-suppression are known in the art and can be similarly applied to the instant invention. These methods involve the silencing of a targeted gene by spliced hairpin RNA's and similar methods also called RNA interference and promoter silencing (see Smith et al. (2000) Nature 407:319-320, Waterhouse and Helliwell (2003)) Nat. Rev. Genet. 4:29-38; Waterhouse et al. (1998) Proc. Natl. Acad. Sci. USA 95:13959-13964; Chuang and Meyerowitz (2000) Proc. Natl. Acad. Sci. USA 97:4985-4990; Stoutjesdijk et al. (2002) Plant Phystiol. 129:1723-1731; and Patent Application WO 99/53050; WO 99/49029; WO 99/61631; WO 00/49035 and US Pat. No. 6,506,559 . For the purpose of this invention the term "co-suppression" is used to collectively designate gene silencing methods based on mechanisms involving the expression of sense RNA molecules, aberrant RNA molecules, double-stranded RNA molecules, micro RNA molecules and the like.

The expression cassette for co-suppression may also be designed such that the sense sequence and the antisense sequence do not correspond to an endogenous RNA. The sense and antisense sequence can flank a loop sequence that comprises a nucleotide sequence corresponding to all or part of the endogenous messenger RNA of the target gene. Thus, it is the loop region that determines the specificity of the RNA interference. See, for example, International Publication No. WO 02/00904. Inhibition of the expression of a protein of interest may also be obtained by RNA interference by expression of a gene encoding a micro RNA (miRNA). miRNAs are regulatory agents consisting of about 22 ribonucleotides. miRNA are highly efficient at inhibiting the expression of endogenous genes. See, for example Javier et al. (2003) Nature 425: 257-263.

For miRNA interference, the expression cassette is designed to express an RNA molecule that is modeled on an endogenous miRNA gene. The miRNA gene encodes an RNA that forms a hairpin structure containing a 22-nucleotide sequence that is complementary to another endogenous gene (target sequence). miRNA molecules are highly efficient at inhibiting the expression of endogenous genes, and the RNA interference they induce is inherited by subsequent generations of plants.

The polynucleotide to be introduced into the plant may comprise an inhibitory sequence that encodes a zinc finger protein that binds to a gene encoding a protein of the invention resulting in reduced expression of the gene. The zinc finger protein binds to a regulatory region of a zein gene, a legumin gene or a globulin gene. The zinc finger protein can also bind to a messenger RNA encoding a seed protein and prevents its translation. Methods of selecting sites for targeting by zinc finger proteins have been described, for example, in U.S. Patent No. 6,453,242, and methods for using zinc finger proteins to inhibit the expression of genes in plants are described, for example, in U.S. Patent Publication No. 20030037355.

Methods for antisense suppression can be used to reduce or eliminate the level of at least one seed protein in grain. The methods of antisense suppression comprise transforming a plant cell with at least one expression cassette comprising a promoter that drives expression in the plant cell operably linked to at least one nucleotide sequence that is antisense to a nucleotide sequence transcript of such a gamma-zein gene. By "antisense suppression" is intended the use of nucleotide sequences that are antisense to nucleotide sequence transcripts of endogenous plant genes to suppress the expression of those genes in the plant.

Methods for suppressing gene expression in plants using nucleotide sequences in the antisense orientation are known in the art. The methods generally involve transforming plants with a DNA construct comprising a promoter that drives expression in a plant operably linked to at least a portion of a nucleotide sequence that is antisense to the transcript of the endogenous gene. Antisense nucleotides are constructed to hybridize with the corresponding mRNA. Modifications of the antisense sequences may be made as long as the sequences hybridize to and interfere with expression of the corresponding mRNA. In this manner, antisense constructions having at least about 70%, 75%, 80%, 85%, 90%, 91%, 92%, 93%, 94%, 95%, 96%, 97%, 98%, or 99% or more sequence identity to the corresponding antisense sequences may be used. Furthermore, portions, rather than the entire nucleotide sequence, of the antisense nucleotides may be used to disrupt the expression of the target gene. Generally, sequences of at least 10 nucleotides, 50 nucleotides, 100 nucleotides, 200 nucleotides, or greater may be used.

Methods for transposon tagging can be used to reduce or eliminate the level of at least one seed protein in grain. The methods of transposon tagging comprise insertion of a transposon within an endogenous plant seed gene to reduce or eliminate expression of the seed protein.

Methods for transposon tagging of specific genes in plants are well known in the art (see for example, Maes et al. (1999) Trends Plant Sci. 4:90-96; Dharmapuri and Sonti (1999) FEMS Microbiol. Lett. 179:53-59; Meissner et al. (2000) Plant J. 22:265-274; Phogat et al. (2000) J. Biosci. 25:57-63; Walbot (2000) Curr. Opin. Plant Biol. 2:103-107; Gai et al. (2000) Nuc. Acids Res. 28:94-96; Fitzmaurice et al. (1999) Genetics 153:1919-1928). In addition, the TUSC process for selecting Mu-insertions in selected genes has been described (Bensen et al. (1995) Plant Cell 7:75-84; Mena et al. (1996) Science 274:1537-1540; U.S. Patent No. 5,962,764.

Other methods for inhibiting or eliminating the expression of endogenous genes are also known in the art and can be similarly applied to the instant invention. These methods include other forms of mutagenesis, such as ethyl methanesulfonate-induced mutagenesis, deletion mutagenesis, and fast neutron deletion mutagenesis used in a reverse genetics sense (with PCR) to identify plant lines in which the endogenous gene has been deleted (for examples of these methods see Ohshima et al. (1998) Virology 243:472-481; Okubara et al. (1994) Genetics 137:867-874; Quesada et al. (2000) Genetics 154:421-436. In addition, a fast and automatable method for screening for chemically induced mutations, TILLING, (Targeting Induced Local Lesions In Genomes), using a denaturing HPLC or selective endonuclease digestion of selected PCR products is also applicable to the instant invention (see McCallum et al. (2000) Nat. Biotechnol. 18:455-457).

Other methods for inhibiting or eliminating the expression of genes include the transgenic application of transcription factors (Pabo, C.O., et al. (2001) Annu Rev Biochem 70, 313-40.; and Reynolds, L., et al (2003), Proc Natl Acad Sci U S A 100, 1615-20.), and homologous recombination methods for gene targeting (see US Pat. No. 6,187,994).

Similarly, it is possible to eliminate the expression of a single gene by replacing its coding sequence with the coding sequence of a second gene using homologous recombination technologies (see Bolon,B. Basic Clin. Pharmacol. Toxicol. 95:4,12, 154-61 (2004); Matsuda and Alba, A., Methods Mol. Bio. 259:379-90 (2004); Forlino, et. al., J. Biol. Chem. 274:53, 37923-30 (1999)).

The polynucleotide expressed by the expression cassette is catalytic RNA or has ribozyme activity specific for the messenger RNA of a protein of interest. Thus, the polynucleotide causes the degradation of the endogenous messenger RNA, resulting in reduced expression of one or more proteins. This method is described, for example, in U.S. Patent No. 4,987,071.

The polynucleotide can comprise an inhibitory sequence that encodes an antibody that binds to at least one isoform of a seed protein, and reduces the level of the seed protein. The binding of the antibody can also result in increased turnover of the antibody-antigen complex by cellular quality control mechanisms. The expression of antibodies in plant cells and the inhibition of molecular pathways by expression and binding of antibodies to proteins in plant cells are well known in the art. See, for example, Conrad and Sonnewald (2003) Nature Biotech. 21:35-36.

Methods of biosynthetic competition with other high-sulfur-containing proteins may be used to reduce the levels of at least one seed protein in plant seed. The methods of biosynthetic competition comprise transforming plant cells with at least one expression cassette comprising a promoter that drives expression in the plant cell operably linked to at least one nucleotide sequence encoding a protein selected from the group consisting of delta-zeins, hordothionin 12, and other naturally occurring or engineered high-sulfur-containing proteins. In some cases the competing protein may possess a high lysine content in addition to a high sulfur content to further increase the nutritional value of the grain.

Biosynthetic competition of seed proteins with other sulfur-rich proteins occurs naturally. This natural process can be manipulated to reduce the levels of certain seed proteins, because the synthesis of some seed proteins is transcriptionally and/or translationally controlled by the nitrogen and/or sulfur supply in the developing seed. The expression of recombinant polypeptides, including the ectopic (transgenic) expression of seed proteins or other high-sulfur-, high-nitrogen-containing proteins, can have a substantial impact on intracellular nitrogen and sulfur pools. Thus, the expression of these proteins can result in suppression of the expression of other seed proteins.

Plant transformants containing a desired genetic modification as a result of any of the above described methods resulting in increased, decreased or eliminated expression of the seed protein tangeted in the invention can be selected by various methods known in the art. These methods include, but are not limited to, methods such as SDS-PAGE analysis, immunoblotting using antibodies which bind to the seed protein of interest, single nucleotide polymorphism (SNP) analysis, or assaying for the products of a reporter or marker gene, and the like.

As disclosed herein specific phenotypic traits are conferred by the expression, or lack thereof, of known corn seed proteins, such as the 50 kD gamma-zein, the 18 kD alpha-globulin, or the 50 kD legumin 1 polypeptides. The specific phenotypic traits for which this method finds use include, but are not limited to, all of those traits listed herein. Maize lines can be screened for a particular phenotypic trait conferred by the presence or absence of known corn seed proteins and the 50 kD gamma-zein, the 18 kD alpha-globulin, or the 50 kD legumin 1 protein using an antibody that binds selectively to one of these polypeptides. In this method, tissue from the maize line of interest is contacted with an antibody that selectively binds the seed-protein polypeptide for which the screen is designed. The development and use of antibodies for the detection of know corn seed proteins and of the 50 kD gamma-zein, the 18 kD alpha-globulin, or the 50 kD legumin 1 proteins is described in Woo, et al, *et seq.* The amount of antibody binding is then quantified and is a measure of the amount of the seed-protein polypeptide present in the maize line. Methods of quantifying polypeptides by immunodetection in this manner are well known in the art.

In the practice of certain specific embodiments of the present invention, a plant is genetically manipulated to have a suppressed or increased level of one or more seed proteins in seed and/or to ectopically express one or more seed or other high-sulfur, high-lysine-containing protein. Those of ordinary skill in the art realize that this can be accomplished in any one of a number of ways. For example, each of the respective coding sequences for such proteins can be operably linked to a promoter and then joined together in a single continuous fragment of DNA comprising a multigenic expression cassette. Such a multigenic expression cassette can be used to transform a plant to produce the desired outcome utilizing any of the methods of the invention including sense and antisense suppression and biosynthetic competition. Alternatively, separate plants can be transformed with expression cassettes containing one of the desired set of coding sequences. Transgenic plants resulting from any or a combination of methods including any method to modulate protein levels, can be selected by standard methods available in the art. These methods include, but are not limited to, methods such as immunoblotting using antibodies which bind to the proteins of interest, SNP analysis, or assaying for the products of a reporter or marker gene, and the like. Then, all of the desired coding sequences and/or transposon tagged sequences can be brought together into a single plant through one or more rounds of cross pollination utilizing the previously selected transformed plants as parents.

The nucleotide sequences for use in the present invention are provided in expression cassettes for transcription in the plant of interest. Such expression cassettes are provided with a plurality of restriction sites for insertion of sequence used in the present invention to be placed under the transcriptional regulation of the regulatory regions. The expression cassettes may additionally contain selectable marker genes.

The expression cassette can include in the 5'-3' direction of transcription, a transcriptional and translational initiation region, any seed protein sequence of the invention, and optionally, a transcriptional and translational termination region functional in plants. The transcriptional initiation region, may be native or analogous or foreign or heterologous to the plant host. Additionally, the promoter may be the natural sequence or alternatively a synthetic sequence. By "foreign" is intended that the transcriptional initiation region is not found in the native plant into which the transcriptional initiation region is introduced. As used herein, a gene comprises a coding sequence operably linked to a transcription initiation region that is heterologous to the coding sequence. Alternatively, a gene comprises fragments of at least two independent transcripts that are linked in a single transcription unit.

While it may be preferable to express the sequences using heterologous promoters, the native promoter sequences may be used. Such constructs would alter expression levels of the proteins in the plant or plant cell. Thus, the phenotype of the plant or plant cell is altered. Alternatively, the promoter sequence may be used to alter expression. For example, the promoter (or fragments thereof) of 27 kD gamma-zein can modulate expression of the native 27 kD gamma-zein protein or other closely related proteins.

Use of a termination region is not necessary for proper transcription of plant genes but may be used as part of an expression construct. The termination region may be native with the transcriptional initiation region, may be native with the operably linked DNA sequence of interest, or may be derived from another source. Convenient termination regions are available from the Ti-plasmid of *A*. *tumefaciens,* such as the octopine synthase and nopaline synthase termination regions. See also Guerineau et al. (1991) Mol. Gen. Genet. 262:141-144; Proudfoot (1991) Cell 64:671-674; Sanfacon et al. (1991) Genes Dev. 5:141-149; Mogen et al. (1990) Plant Cell 2:1261-1272; Munroe et al. (1990) Gene 91:151-158; Ballas et al. (1989) Nucleic Acids Res. 17:7891-7903; and Joshi et al. (1987). Nucleic Acid Res. 15:9627-9639.

Where appropriate, for example, as in the case of engineered high-sulfur-containing proteins for the method of biosynthetic competition, the gene(s) may be optimized for increased expression in the transformed plant. That is, the genes can be synthesized using plant-preferred codons for improved expression. See, for example, Campbell and Gowri (1990) Plant Physiol. 92:1-11 for a discussion of host-preferred codon usage. Methods are available in the art for synthesizing plant-preferred genes. See, for example, U.S. Patent Nos. 5,380,831, and 5,436,391, and Murray et al. (1989) Nucleic Acids Res. 17:477-498.

Additional sequence modifications are known to enhance gene expression in a cellular host. These include elimination of sequences encoding spurious polyadenylation signals, exon-intron splice site signals, transposon-like repeats, and other such well-characterized sequences that may be deleterious to gene expression. The G-C content of the sequence may be adjusted to levels average for a given cellular host, as calculated by reference to known genes expressed in the host cell. When possible, the sequence is modified to avoid predicted hairpin secondary mRNA structures.

The expression cassettes may additionally contain 5' leader sequences in the expression cassette construct. Such leader sequences can act to enhance translation. Translation leaders are known in the art and include: picornavirus leaders, for example, EMCV leader (Encephalomyocarditis 5' noncoding region) (Elroy-Stein et al. (1989) Proc. Natl. Acad. Sci. USA 86:6126-6130); potyvirus leaders, for example, TEV leader (Tobacco Etch Virus) (Gallie et al. (1995) Gene 165(2):233-238), MDMV leader (Maize Dwarf Mosaic Virus) (Virology 154:9-20), and human immunoglobulin heavy-chain binding protein (BiP) (Macejak et al. (1991) Nature 353:90-94); untranslated leader from the coat protein mRNA of alfalfa mosaic virus (AMV RNA 4) (Jobling et al. (1987) Nature 325:622-625); tobacco mosaic virus leader (TMV) (Gallie et al. (1989) in Molecular Biology of RNA, ed. Cech (Liss, New York), pp. 237-256); and maize chlorotic mottle virus leader (MCMV) (Lommel et al. (1991) Virology 81:382-385). See also, Della-Cioppa et al. (1987) Plant Physiol. 84:965-968. Other methods known to enhance translation can also be utilized, for example, introns, and the like.

In preparing the expression cassette, the various DNA fragments may be manipulated, so as to provide for the DNA sequences in the proper orientation and, as appropriate, in the proper reading frame. Toward this end, adapters or linkers may be employed to join the DNA fragments or other manipulations may be involved to provide for convenient restriction sites, removal of superfluous DNA, removal of restriction sites, or the like. For this purpose, *in vitro* mutagenesis, primer repair, restriction, annealing, resubstitutions, e.g., transitions and transversions, may be involved.

Generally, the expression cassette will comprise a selectable marker gene for the selection of transformed cells. Selectable marker genes are utilized for the selection of transformed cells or tissues. Marker genes include genes encoding antibiotic resistance, such as those encoding neomycin phosphotransferase II (NEO) and hygromycin phosphotransferase (HPT), as well as genes conferring resistance to herbicidal compounds, such as glufosinate ammonium, bromoxynil, imidazolinones, and 2,4-dichlorophenoxyacetate (2,4-D). See generally, Yarranton (1992) Curr. Opin. Biotech. 3:506-511; Christopherson et al. (1992) Proc. Natl. Acad. Sci. USA 89:6314-6318; Yao et al. (1992) Cell 71:63-72; Reznikoff (1992) Mol. Microbiol. 6:2419-2422; Barkley et al. (1980) in The Operon, pp. 177-220; Hu et al. (1987) Cell 48:555-566; Brown et al. (1987) Cell 49:603-612; Figge et al. (1988) Cell 52:713-722; Deuschle et al. (1989) Proc. Natl. Acad. Aci. USA 86:5400-5404; Fuerst et al. (1989) Proc. Natl. Acad. Sci. USA 86:2549-2553; Deuschle et al. (1990) Science 248:480-483; Gossen (1993) Ph.D. Thesis, University of Heidelberg; Reines et al. (1993) Proc. Natl. Acad. Sci. USA 90:1917-1921; Labow et al. (1990) Mol. Cell. Biol. 10:3343-3356; Zambretti et al. (1992) Proc. Natl. Acad. Sci. USA 89:3952-3956; Baim et al. (1991) Proc. Natl. Acad. Sci. USA 88:5072-5076; Wyborski et al. (1991) Nucleic Acids Res. 19:4647-4653; Hillenand-Wissman (1989) Topics Mol. Struc. Biol. 10:143-162; Degenkolb et al. (1991) Antimicrob. Agents Chemother. 35:1591-1595; Kleinschnidt et al. (1988) Biochemistry 27:1094-1104; Bonin (1993) Ph.D. Thesis, University of Heidelberg; Gossen et al. (1992) Proc. Natl. Acad. Sci. USA 89:5547-5551; Oliva et al. (1992) Antimicrob. Agents Chemother. 36:913-919; Hlavka et al. (1985) Handbook of Experimental Pharmacology, Vol. 78 (Springer-Verlag, Berlin); Gill et al. (1988) Nature 334:721-724.

The above list of selectable marker genes is not meant to be limiting. Any selectable marker gene can be used in the present invention.

The use of the term "nucleotide constructs" herein is not intended to limit the present invention to nucleotide constructs comprising DNA. Those of ordinary skill in the art will recognize that nucleotide constructs, particularly polynucleotides and oligonucleotides, comprised of ribonucleotides and combinations of ribonucleotides and deoxyribonucleotides may also be employed in the methods disclosed herein. Thus, the nucleotide constructs used in the present invention encompass all nucleotide constructs that can be employed in the methods of the present invention for transforming plants including, but not limited to, those comprised of deoxyribonucleotides, ribonucleotides, and combinations thereof. Such deoxyribonucleotides and ribonucleotides include both naturally occurring molecules and synthetic analogues. The nucleotide construct used in the invention also encompass all forms of nucleotide constructs including, but not limited to, single-stranded forms, double-stranded forms, hairpins, stem-and-loop structures, and the like.

Furthermore, it is recognized that the invention may employ a nucleotide construct that is capable of directing, in a transformed plant, the expression of at least one protein, or at least one RNA, such as, for example, an antisense RNA that is complementary to at least a portion of an mRNA. Alternatively, it is also recognized that the invention may employ a nucleotide construct that is not capable of directing, in a transformed plant, the expression of a protein or an RNA.

In addition, it is recognized that methods of the present invention do not depend on the incorporation of the entire nucleotide construct into the genome, only that the plant or cell thereof is altered as a result of the introduction of the nucleotide construct into a cell. The genome may be altered following the introduction of the nucleotide construct into a cell. For example, the nucleotide construct, or any part thereof, may incorporate into the genome of the plant. Alterations to the genome include, but are not limited to, additions, deletions, and substitutions of nucleotides in the genome. While the methods do not depend on additions, deletions, or substitutions of any particular number of nucleotides, it is recognized that such additions, deletions, or substitutions comprise at least one nucleotide.

The nucleotide constructs used in the invention also encompass nucleotide constructs that may be employed in methods for altering or mutating a genomic nucleotide sequence in an organism, including, but not limited to, chimeric vectors, chimeric mutational vectors, chimeric repair vectors, mixed-duplex oligonucleotides, self-complementary chimeric oligonucleotides, and recombinogenic oligonucleobases. Such nucleotide constructs and methods of use, such as, for example, chimeraplasty, are known in the art. Chimeraplasty involves the use of such nucleotide constructs to introduce site-specific changes into the sequence of genomic DNA within an organism. See U.S. Patent Nos. 5,565,350; 5,731,181; 5,756,325; 5,760,012; 5,795,972; and 5,871,984. See also, WO 98/49350, WO 99/07865, WO 99/25821, and Beetham et al. (1999) Proc. Natl. Acad. Sci. USA 96:8774-8778.

A number of promoters can be used in the practice of the invention. The promoters can be selected based on the desired outcome. The nucleic acids can be combined with constitutive, tissue-preferred, or other promoters for expression in plants, more preferably a promoter functional during seed development.

Such constitutive promoters include, for example, the core promoter of the Rsyn7 promoter and other constitutive promoters disclosed in WO 99/43838; the core CaMV 35S promoter (Odell et al. (1985) Nature 313:810-812); rice actin (McElroy et al. (1990) Plant Cell 2:163-171); ubiquitin (Christensen et al. (1989) Plant Mol. Biol. 12:619-632 and Christensen et al. (1992) Plant Mol. Biol. 18:675-689); pEMU (Last et al. (1991) Theor. Appl. Genet. 81:581-588); MAS (Velten et al. (1984) EMBO J. 3:2723-2730); ALS promoter (U.S. Patent No. 5,659,026), and the like. Other constitutive promoters include, for example, U.S. Patent Nos. 5,608,149; 5,608,144; 5,604,121; 5,569,597; 5,466,785; 5,399,680; 5,268,463; and 5,608,142.

Chemical-regulated promoters can be used to modulate the expression of a gene in a plant through the application of an exogenous chemical regulator. Depending upon the objective, the promoter may be a chemical-inducible promoter, where application of the chemical induces gene expression, or a chemical-repressible promoter, where application of the chemical represses gene expression. Chemical-inducible promoters are known in the art and include, but are not limited to, the maize In2-2 promoter, which is activated by benzenesulfonamide herbicide safeners, the maize GST promoter, which is activated by hydrophobic electrophilic compounds that are used as pre-emergent herbicides, and the tobacco PR-1 a promoter, which is activated by salicylic acid. Other chemical-regulated promoters of interest include steroid-responsive promoters (see, for example, the glucocorticoid-inducible promoter in Schena et al. (1991) Proc. Natl. Acad. Sci. USA 88:10421-10425 and McNellis et al. (1998) Plant J. 14(2):247-257) and tetracycline-inducible and tetracycline-repressible promoters (see, for example, Gatz et al. (1991) Mol. Gen. Genet. 227:229-237, and U.S. Patent Nos. 5,814,618 and 5,789,156).

Tissue-preferred promoters can be utilized to target enhanced protein expression within a particular plant tissue. Tissue-preferred promoters include, but are not limited to: Yamamoto et al. (1997) Plant J. 12(2)255-265; Kawamata et al. (1997) Plant Cell Physiol. 38(7):792-803; Hansen et al. (1997) Mol. Gen Genet. 254(3):337-343; Russell et al. (1997) Transgenic Res. 6(2):157-168; Rinehart et al. (1996) Plant Physiol. 112(3):1331-1341; Van Camp et al. (1996) Plant Physiol. 112(2):525-535; Canevascini et al. (1996) Plant Physiol. 112(2):513-524; Yamamoto et al. (1994) Plant Cell Physiol. 35(5):773-778; Lam (1994) Results Probl. Cell Differ. 20:181-196; Orozco et al. (1993) Plant Mol Biol. 23(6):1129-1138; Matsuoka et al. (1993) Proc Natl. Acad. Sci. USA 90(20):9586-9590; and Guevara-Garcia et al. (1993) Plant J. 4(3):495-505. Such promoters can be modified, if necessary, for weak expression.

"Seed-preferred" promoters include both "seed-specific" promoters (those promoters active during seed development such as promoters of seed storage proteins) as well as "seed-germinating" promoters (those promoters active during seed germination). See Thompson et al. (1989) BioEssays 10:108 . Such seed-preferred promoters include, but are not limited to, Cim1 (cytokinin-induced message); cZ19B1 (maize 19 kD zein); and milps (myo-inositol-1-phosphate synthase; see US Pat. No 6,225,529 ). The 27kD gamma-zein is a preferred endosperm-specific promoter. Glb-1 is a preferred embryo-specific promoter. For dicots, seed-specific promoters include, but are not limited to, bean β-phaseolin, napin, β-conglycinin, soybean lectin, cruciferin, and the like. For monocots, seed-specific promoters include, but are not limited to, maize 15 kD zein, 22 kD zein, 27 kD zein, 10kD delta-zein, waxy, shrunken 1, shrunken 2, globulin 1, etc.

In certain embodiments the nucleic acid sequences used in the present invention can be combined with any combination of polynucleotide sequences of interest or mutations in order to create plants with a desired phenotype. For example, the polynucleotides disclosed herein can be combined with any other polynucleotides disclosed herein such as any combination of SEQ ID NOS: 1, 3, 5, or with other seed storage protein genes or variants or fragments thereof such as: zeins, fatty acid desaturases, lysine ketoglutarate, *lec1*, or *Agp.* The combinations generated can also include multiple copies of any one of the polynucleotides of interest. The polynucleotides or mutations used in the present invention can also be combined with any other gene or combination of genes to produce plants with a variety of desired trait combinations including, but not limited to, traits desirable for animal feed such as high oil genes (*e.g*., U.S. Patent No. 6,232,529); balanced amino acids (*e.g*. hordothionins (U.S. Patent Nos. 5,990,389; 5,885,801; 5,885,802; 5,703,409 and 6,800,726); high lysine (Williamson et al. (1987) Eur. J. Biochem. 165:99-106; and WO 98/20122); and high methionine proteins (Pedersen et al. (1986) J. Biol. Chem. 261:6279; Kirihara et al. (1988) Gene 71:359; and Musumura et al. (1989) Plant Mol. Biol. 12: 123)); and thioredoxins (U.S. Application Serial No. 10/005,429, filed December 3, 2001)). The polynucleotides used in the present invention can also be combined with traits desirable for insect, disease or herbicide resistance (*e.g., Bacillus thuringiensis* toxic proteins (U.S. Patent Nos. 5,366,892; 5,747,450; 5,737,514; 5723,756; 5,593,881; Geiser et al. (1986) Gene 48:109); lectins (Van Damme et al. (1994) Plant Mol. Biol. 24:825); fumonisin detoxification genes (U.S. Patent No. 5,792,931); avirulence and disease resistance genes (Jones et al. (1994) Science 266:789; Martin et al. (1993) Science 262:1432; Mindrinos et al. (1994) Cell 78:1089); acetolactate synthase (ALS) mutants that lead to herbicide resistance such as the S4 and/or Hra mutations; inhibitors of glutamine synthase such as phosphinothricin or basta (*e.g*., bar gene); and glyphosate resistance (EPSPS gene)); and traits desirable for processing or process products such as high oil (*e.g*., U.S. Patent No. 6,232,529 ); modified oils (*e.g*., fatty acid desaturase genes (U.S. Patent No. 5,952,544; WO 94/11516)): modified starches (*e.g*., ADPG pyrophosphorylases (AGPase), starch synthases (SS), starch branching enzymes (SBE) and starch debranching enzymes (SDBE)); and polymers or bioplastics (*e.g*., U.S. patent No. 5.602,321; beta-ketothiolase, polyhydroxybutyrate synthase, and acetoacetyl-CoA reductase (Schubert et al. (1988) J. Bacteriol. 170:5837-5847) facilitate expression of polyhydroxyalkanoates (PHAs)) . One could also combine the polynucleotides or mutations used in the present invention with polynucleotides providing agronomic traits such as male sterility (*e*.*g*., see U.S. Patent No. 5.583,210), stalk strength, flowering time, or transformation technology traits such as cell cycle regulation or gene targeting (*e.g*. WO 99/61619; WO 00/17364; WO 99/25821).

These combinations can be created by any method including, but not limited to, cross breeding plants by any conventional or TopCross methodology, by homologous recombination, site specific recombination, or other genetic modification. If the traits are combined by genetically transforming the plants, the polynucleotide sequences of interest can be combined at any time and in any order. For example, a transgenic plant comprising one or more desired traits can be used as the target to introduce further traits by subsequent transformation. The traits can be introduced simultaneously in a co-transformation protocol with the polynucleotides of interest provided by any combination of transformation cassettes. For example, if two sequences will be introduced, the two sequences can be contained in separate transformation cassettes (trans) or contained on the same transformation cassette (cis). Expression of the sequences can be driven by the same promoter or by different promoters. In certain cases, it may be desirable to introduce a transformation cassette that will suppress the expression of the polynucleotide of interest. This may be combined with any combination of other suppression cassettes or overexpression cassettes to generate the desired combination of traits in the plant. Traits may also be combined by transformation and mutation by any known method.

Suitable methods of introducing nucleotide sequences into plant cells and subsequent insertion into the plant genome include, but are not limited to: microinjection (Crossway et al. (1986) Biotechniques 4:320-334), electroporation (Riggs et al. (1986) Proc. Natl. Acad. Sci. USA 83:5602-5606, Agrobacterium-mediated transformation (Townsend et al., U.S. Patent No. 5,563,055; Zhao et al., U.S. Patent No. 5,981,840; Cai et al., US Patent Application No. 09/056,418), direct gene transfer (Paszkowski et al. (1984) EMBO J. 3:2717-2722), and ballistic particle acceleration (see; for example, Sanford et al., U.S. Patent No. 4,945,050; Tomes et al., U.S. Patent No. 5,879,918; Tomes et al., U.S. Patent No. 5,886,244; Bidney et al., U.S. Patent No. 5,932,782; Tomes et al. (1995) "Direct DNA Transfer into Intact Plant Cells via Microprojectile Bombardment," in Plant Cell, Tissue, and Organ Culture: Fundamental Methods, ed. Gamborg and Phillips (Springer-Verlag, Berlin); and McCabe et al. (1988) Biotechnology 6:923-926). Also see Weissinger et al. (1988) Ann. Rev. Genet. 22:421-477; Sanford et al. (1987) Particulate Science and Technology 5:27-37 (onion); Christou et al. (1988) Plant Physiol. 87:671-674 (soybean); McCabe et al. (1988) Bio/Technology 6:923-926 (soybean); Finer and McMullen (1991) In Vitro Cell Dev. Biol. 27P:175-182 (soybean); Singh et al. (1998) Theor. Appl. Genet. 96:319-324 (soybean); Datta et al. (1990) Biotechnology 8:736-740 (rice); Klein et al. (1988) Proc. Natl. Acad. Sci. USA 85:4305-4309 (maize); Klein et al. (1988) Biotechnology 6:559-563 (maize); Tomes, U.S. Patent No. 5,240,855; Buising et al., U.S. Patent Nos. 5,322,783 and 5,324,646; Tomes et al. (1995) "Direct DNA Transfer into Intact Plant Cells via Microprojectile Bombardment," in Plant Cell, Tissue, and Organ Culture: Fundamental Methods, ed. Gamborg (Springer-Verlag, Berlin) (maize); Klein et al. (1988) Plant Physiol. 91:440-444 (maize); Fromm et al. (1990) Biotechnology 8:833-839 (maize); Hooykaas-Van Slogteren et al. (1984) Nature (London) 311:763-764; Bowen et al., U.S. Patent No. 5,736,369 (cereals); Bytebier et al. (1987) Proc. Natl. Acad. Sci. USA 84:5345-5349 (Liliaceae); De Wet et al. (1985) in The Experimental Manipulation of Ovule Tissues, ed. Chapman et al. (Longman, New York), pp. 197-209 (pollen); Kaeppler et al. (1990) Plant Cell Reports 9:415-418 and Kaeppler et al. (1992) Theor. Appl. Genet. 84:560-566 (whisker-mediated transformation); D'Halluin et al. (1992) Plant Cell 4:1495-1505 (electroporation); Li et al. (1993) Plant Cell Reports 12:250-255 and Christou and Ford (1995) Annals of Botany 75:407-413 (rice); Osjoda et al. (1996) Nature Biotechnology 14:745-750 (maize via *Agrobacterium tumefaciens*) .

The invention involves introducing a nucleotide construct into a plant. By "introducing" is intended presenting to the plant the nucleotide construct in such a manner that the construct gains access to the interior of a cell of the plant. The methods do not depend on a particular method for introducing a nucleotide construct to a plant, only that the nucleotide construct gains access to the interior of at least one cell of the plant. Methods for introducing nucleotide constructs into plants are known in the art including, but not limited to, stable transformation methods, transient transformation methods, and virus-mediated methods.

By "stable transformation" is intended that the nucleotide construct introduced into a plant integrates into the genome of the plant and is capable of being inherited by progeny thereof. By "transient transformation" is intended that a nucleotide construct introduced into a plant does not integrate into the genome of the plant.

The nucleotide constructs used in the invention may be introduced into plants by contacting plants with a virus or viral nucleic acids. Generally, such methods involve incorporating a nucleotide construct of the invention within a viral DNA or RNA molecule. It is recognized that the protein of interest may be initially synthesized as part of a viral polyprotein, which later may be processed by proteolysis *in vivo* or *in vitro* to produce the desired recombinant protein. Further, it is recognized that promoters used in the invention also encompass promoters utilized for transcription by viral RNA polymerases. Methods for introducing nucleotide constructs into plants and expressing a protein encoded therein, involving viral DNA or RNA molecules, are known in the art. See, for example, U.S. Patent Nos. 5,889,191, 5,889,190, 5,866,785, 5,589,367 and 5,316,931.

The cells that have been transformed may be grown into plants in accordance with conventional ways, under plant forming conditions. See, for example, McCormick et al. (1986) Plant Cell Reports 5:81-84. These plants may then be grown, and either pollinated with the same transformed strain or different strains, and the resulting hybrid having expression of the desired phenotypic characteristic identified. Two or more generations may be grown to ensure that expression of the desired phenotypic characteristic is stably maintained and inherited and then seeds harvested to ensure expression of the desired phenotypic characteristic has been achieved.

In addition, the desired genetically altered trait can be bred into other plant lines possessing desirable agronomic characteristics using conventional breeding methods (see Example 3) and/or top-cross technology. The top-cross method is taught in US Pat. No. 5,704,160.

Methods for cross pollinating plants are well known to those skilled in the art, and are generally accomplished by allowing the pollen of one plant, the pollen donor, to pollinate a flower of a second plant, the pollen recipient, and then allowing the fertilized eggs in the pollinated flower to mature into seeds. Progeny containing the entire complement of heterologous coding sequences of the two parental plants can be selected from all of the progeny by standard methods available in the art as described infra for selecting transformed plants. If necessary, the selected progeny can be used as either the pollen donor or pollen recipient in a subsequent cross pollination.

It has been shown that the response in digestibility to the treatment of grain with DTT is inversely related to the digestibility of untreated grain (Boisen and Eggum, Nutrition Research Reviews 4:141-162 (1991)).

Digestibility of immature grain (grain at late dough or silage maturity stage) is equally improved by pretreatment with reducing agents (DTT) as mature grain. The same can be expected for low gamma-zein corn as the effects of DTT pretreatment, and low gamma-zein corn, on digestibility are virtually the same. Improvements in digestibility of immature grain through the present invention can be extrapolated to improvements in digestibility of silage - about half of which consists of immature grain. The improvements in digestibility with DTT pretreatment is inversely related to the intrinsic digestibility of untreated grain. For this reason, corn lines of low intrinsic digestibility can be expected to be more amenable to genetic modification through the methods disclosed herein than those of higher digestibility. This enables those of skill in the art of breeding to make rapid advances in introgressing a low gamma-zein trait into the appropriate elite germplasm.

This invention allows for the improvement of grain properties such as increased digestibility/nutrient availability, nutritional value, silage quality, and efficiency of wet or dry milling in maize strains already possessing other desirable characteristics.

Corn grain with reduced gamma-zein protein content offers the following advantages:
First, ground corn grain with a reduced gamma-zein protein content offer increased energy availability and protein digestibility to monogastric livestock (see Example 2). "Monogastric animals" include but are not limited to: pigs, poultry, horses, dogs, cats, rabbits and rodents.
   It can be deduced from analysis of the *in vitro* experimental data provided herein that the corn grain from maize genetically altered to contain reduced gamma-zein protein levels will have a 5% increase in metabolizable energy for poultry and pigs. Using this assumption the following replacement value can be assigned to the high energy availability trait in grain resulting from the reduction of gamma-zein protein. Five percent of 1665 kcal/lb (15,400kj/kg) equals about 83 kcal/lb (766kj/kg). Taking into account that a bushel of corn contains 56-60 lbs (25.4 - 27.2 kg), the 83 kcal/lb (766kj/kg) difference amounts to a gain of 4650-5000 kcal/bu (19,500 - 20,900kj per bushel). This difference in available energy is equivalent to 1.3-1.4 lb fat (0.59 - 0.63kg), which, at 12cts/lb ($0.26/kg), is worth 15-17 cents ($0.15 - 0.17) per bushel.
Second, corn grain with a reduced gamma-zein protein content possess improved ruminant (e.g.: cattle sheep, and goats) feed quality through increased digestibility (see Example 2). Grain is fed to ruminants in minimally processed form, and the rigid protein structure of corn endosperm has been shown to constitute a large impediment to microbial digestion in the rumen, which can be partly overcome by predigestion with protease (McAllister et al. (1993) J. Anim. Sci. 71:205-212). A reduced gamma-zein protein content imparts a similar or even larger improvement to ruminal digestion of whole corn.
Third, corn grain with reduced levels of gamma-zein proteins has an increased response to feed processing. The nutrient availability from whole corn grain can be increased by extensive processing (steam-flaking or extrusion) resulting in starch gelatinization and protein disulfide bond reduction (Blackwood and Richardson, 1994). The response to processing is sometimes lower than expected. The heat and/or shearing force applied during processing causes rearrangements of protein disulfide bonds, which may partly counteract the improvement in digestibility resulting from starch gelatinization. The response to steam-flaking of corn and sorghum grain is negatively correlated with protein disulfide content (Blackwood and Richardson, 1994). For low gamma-zein corn or low kafarin sorghum the extent of disulfide rearrangements during processing is reduced, which allows for a more uniform response to steam-flaking, and which can be expected to reduce the energy required in steam-flaking or grinding processes.
Fourth, corn grain with a lower gamma-zein protein content has improved silage quality for dairy cattle, especially for silage harvested at late maturity. Although silage is harvested at earlier maturity than grain, a certain degree of dry-down (and protein disulfide formation) has already occurred by the time the crop is ensiled, especially under dry and hot conditions. Our work has shown that pretreatment with a reducing agent of immature, dough-stage corn kernels, sampled at silage maturity, resulted in drastically improved *in vitro* digestibility, a strong indication that the protein disulfide imposed barriers to digestion had already been established. (data in Example 2). Hence, the digestibility of the "yellow portion" of corn silage can be expected to be higher for grain with a reduced gamma-zein protein content. Increased digestibility will be especially notable in the case of silage made from mature corn and for high-yielding dairy cows in which high passage rates do not allow for extensive ruminal digestion.
Fifth, corn grain with a reduced gamma-zein protein content will have an increased efficiency of wet milling. An increase in wet-milling efficiency and starch recovery can be expected due to the lower disulfide content of grain with reduced gamma-zein protein content. Efficiencies in the processes of wet milling also include reduced steeping time and/or reduced need for chemical reductants such as sulfur dioxide and sodium bisulfite. The use of fewer chemicals will improve wet-milling economics and reduce environmental pollution.

### Table of Sequence ID Nos.

| SEQ ID NO: Acid/Nucleotide | Gene Name | Amino |
|---|---|---|
| 1 | 50 kD gamma-zein | Nucleotide |
| 2 | 50 kD gamma-zein | Amino Acid |
| 3 | 18 kD alpha-globulin | Nucleotide |
| 4 | 18 kD alpha-globulin | Amino Acid |
| 5 | 50 kD corn legumin1 | Nucleotide |
| 6 | 50 kD corn legumin1 | Amino Acid |
| 7 | 50 kD gamma-zein, B73 | Nucleotide |
| 8 | 50 kD gamma-zein, Mo17 | Nucleotide |
| 9 | 18 kD alpha-globulin, B73 | Nucleotide |
| 10 | 18 kD alpha-globulin, Mo17 | Nucleotide |
| 11 | TUSC primer 170 | Nucleotide |
| 12 | TUSC primer 296 | Nucleotide |
| 13 | MU primer 9242 | Nucleotide |
| 14 | TUSC primer 008 | Nucleotide |
| 15 | TUSC primer 009 | Nucleotide |
| 16 | Chimeric alpha-zein | Nucleotide |
| 17 | Chimeric silencing seq. | Nucleotide |
| 18 | 15kD beta-zein | Nucleotide |
| 19 | 15kD beta-zein | Amino Acid |
| 20 | High sulfur zein | Nucleotide |
| 21 | High sulfur zein | Amino Acid |
| 22 | Sorghum legumin1 | Nucleotide |
| 23 | Sorghum legumin1 | Amino Acid |
| 24 | Sugarcane legumin1 | Nucleotide |
| 25 | Sugarcane legumin1 | Amino Acid |
| 26 | GZ-W64A promoter | Nucleotide |
| 27 | GZ-W64A terminator | Nucleotide |

### EXAMPLES

Maize lines (transgenic and transposon-mutagenized) have been developed with increased or decreased levels of specific endosperm proteins. For several of the obtained lines, experimental evidence indicates that the introduced changes result in improved grain properties.

### Example 1: Cloning and Transgenic Co-suppression of a Novel Maize 50 kD Gamma-Zein.

A 50 kD gamma-zein nucleotide sequence was cloned from a maize endosperm cDNA library (mid and late development). Based on EST numbers 50 kD gamma-zein transcripts are relatively abundant (compared to other seed protein transcripts) and represent approximately 0.5% of the endosperm mRNA during mid development. A large variation in the abundance of 50 kD gamma-zein transcripts has been observed between different inbred lines (transcript profiling results). The 50 kD gamma-zein gene has been located on chromosome 7, bin 7.03

The 50kD gamma-zein cDNA sequences isolated from different inbred lines show an unusually low level of polymorphism. Only one SNP (a 3bp insertion) was detected along the entire cDNA sequence from DNA isolated from the inbred lines
Mo17 and B73 (the SNP is bold and in lower case). See also SEQ ID NOS: 7 and 8.
50 kD gamma-zein, B73 50 kD gamma-zein, Mo17 partial

The 50-kD gamma-zein transformation event described herein was one of various high-digestibility events produced. The event was generated with a construct containing the 27kD gamma-zein promoter, 50 kD gamma-zein ORF in sense orientation, and 27-kD gamma zein terminator using particle bombardment. It was found to be reduced in all known gamma zein proteins, i.e., 50 kD-, 27 kD-, and 16 kD gamma-zein. Protein gel & 50 kD gamma-zein Western blots of segregating CS50 events were performed to confirm co-suppression. The kernel phenotype of the transgenic seed was normal (i.e., vitreous).

Segregating kernels from transgenic corn co-suppressed in 50 kD gamma-zein were ground to a fine meal and subjected to the monogastric in vitro digestibility assay as described in Example 2 to determine Enzyme Digestible Dry Matter (EDDM). EDDM of 50 kD gamma-zein co-suppressed grain was improved by 3.0 percentage units. An overnight soak in 10 mM of the strong reducing agent dithiothreietol (DTT), known to maximize *in vitro* digestibility, improved digestibility slightly beyond that reached with 50kD gamma-zein co-suppression (by 1.4 percentage units).

### Example 2: In Vitro Enzyme Digestible Dry Matter (EDDM) Assay.

Corn grain was ground in a micro Wiley Mill (Thomas Scientific, Swedesboro, NJ) through a 1 mm screen; 0.5 g of ground corn sample was placed in a pre-weighed nylon bag (50 micron pore size) and heat sealed. Approximately 40 bags were placed in an incubation bottle with 2L of 0.2M phosphate buffer (pH 2.0) containing pepsin (0.25 mg/ml). Samples were incubated in a Daisy II incubator (ANKOM Technology, Fairport, NY) at 39°C for 2 hours. After 2 hours, samples were placed in a mesh bag and washed for 2 minutes with cold water in a washer (Whirlpool) using delicate cycle. Samples were then transferred into 2L of 0.2M phosphate buffer (pH 6.8) containing pancreatin (5.0 mg/ml) and incubated at 39°C for 4 or 6 hours. Samples were washed for 2 minutes as described earlier. Samples were then dried overnight at 55°C and weighed. The difference in sample weight before and after incubation was expressed as percentage of enzyme digestible dry matter digestibility (EDDM). EDDM data generated by in vitro digestibility assay could vary with genetic backgrounds, field conditions and locations in which the plants are grown. Hence the absolute EDDM values could vary for the same transgene with different genetic backgrounds, field conditions and locations in which they are grown.

### Example 3: Transgenic Co-suppression of 27 kD Gamma-Zein.

Events in which the expression level of 27 kD gamma-zein protein was reduced to less than 5% of wild-type as determined by SDS-PAGE and immunoblotting were obtained with three different transgenic constructs.

One event was generated with a construct containing the 27kD gamma-zein promoter, 27 kD gamma-zein ORF in sense orientation (GenBank Accession No: AF371261), and the 27-kD gamma-zein terminator using Agrobacterium-mediated transformation (see Example 12). It was found to be reduced in 27 kD-, and 16 kD-gamma-zein. The kernel phenotype of the transgenic seed was normal (i.e., vitreous).

A second event was generated with a construct containing the CZ19B1 1 promoter (US Pat No:6,225,529), 27 kD gamma-zein ORF in sense orientation, and the 27-kD gamma-zein terminator using Agrobacterium-mediated transformation (see Example 12). It also was found to be reduced in 27 kD-, and 16 kD-gamma-zein. The kernel phenotype of the transgenic seed was normal (i.e., vitreous).

Finally, several events were produced with a construct containing the CZ19B1 promoter, an inverted repeat comprised of 303 bp of the 27 kD gamma-zein cDNA in sense orientation and 303 bp of the 27-kD gamma-zein cDNA in anti-sense orientation. The sequence of this hairpin is found at positions 1-303 of SEQ ID NO:17. The construct contained no terminator and was transformed using Agrobacterium-mediated transformation (see Example 12). About 90% of the transgenic maize events generated with this construct were found to be reduced in 27 kD-, and 16 kD-gamma-zein. The kernel phenotype of the transgenic seed was normal (i.e., vitreous).

The endosperm protein profiles of grain in which the 27 kD gamma-zein gene was co-suppressed showed more than a 95% suppression of the protein and an additional reduction of more than 60% in the level of the 16 kD gamma-zein protein, and an approximate three- to five-fold increase in the level of the 15 kD beta-zein protein. Even with the significant decrease in high disulfide containing gamma-zein proteins, grain from these events showed a normal (vitreous) phenotype and were of unaltered test weight and hardness. This result was unexpected as the decrease in the disulfide content, and specifically the decrease in 27kD gamma-zein, might have been expected to result in grain with a soft or opaque phenotype (see Lopez and Larkins, 1991).

Assays for seed hardness are well known in the art and include such methods as those used in the present invention, described in Pomeranz et al. Cereal Chemistry 61(2):147-150 (1984). In essence, breaking susceptibility and grain hardness are measured by density, near-infrared reflectance or average particle size of ground material. The three measure of hardness are highly, linearly, and positively correlated provided the maize samples are homogenous in terms of starch composition (such as waxy, regular or high amylose), and in protein, oil, and ash content.

Assays for the vitreous phenotype are well known in the art and include such methods as those used in the present invention, described in: Erasmus and Taylor (2004). J. Science of Food and Agriculture, 84 (9): 920-930. Briefly, the intensity of translucency in maize is linearly correlated to the percentage of kernel illumination (r=0.99, p<0.001) when placed on a round illuminated area smaller than the projected area of the kernel, allowing light to shine through. Translucency as a percentage of the whole kernel and vitreous endosperm (mass%), was r=0.77. Translucency as a percentage of the whole kernel and opaque endosperm (mass%), was r= -0.72. Translucency varied by 29.5% between the lowest and highest values, and vitreous endosperm (mass%) varied by 16.8% between the lowest and highest values.

The co-suppression trait was shown to be dominant. Various normal and transgenic maize lines, as well as commercial hybrids, were pollinated with pollen from the gamma-zein co-suppressing events with the result of total suppression of gamma-zein protein in the hemizygous endosperm as determined by SDS-PAGE and immunoblotting. Therefore, the gamma-zein gene co-suppression trait can be introduced into specific pollinators (i.e., high oil corn) using conventional methods and/or the top-cross technology found in US Patent No. 5,704,160.

T₃-segregating grain was phenotyped for gamma-zein protein levels and were divided into two samples, one with wild-type gamma-zein protein levels and a second with reduced gamma-zein protein levels (less than 10% of wild-type). Ground corn from both samples was subjected to an *in vitro* energy availability assay. The enzyme digestible dry matter (EDDM) assay used in these experiments as an indicator of *in vivo* digestibility, is known in the art and was performed using enzymes, buffers, and digestion conditions described in Example 2 and Boisen and Fernandez (1997) Animal Feed Science and Technology 68: 277-286); and Boisen and Fernandez (1995) Animal Feed Science and Technology 51:29. The results clearly indicated that ground corn from gamma-zein co-suppressed grain were more rapidly and extensively digested than corn with normal gamma-zein protein levels, by as much as 20% at the 4 hour time point and as much as an additional 6% at the 6 hr time point.

The role of disulfide bridges in the digestion of corn was investigated in co-suppressed gamma-zein versus control grain. As expected, pretreatment with a strong reducing agent (10 mM DTT) increased the enzyme digestible dry matter (EDDM) level (4 hour digestion) of control grain by 16% but not that of the gamma-zein gene co-suppressed grain. A similar result was observed for various low gamma-zein TopCross hybrids (e.g.: with public grain hybrids 3394 and 32J55). Hence, the impact of DTT on digestibility apparently involves the reduction of disulfides of cysteine residues in gamma-zein proteins. Phenotyped kernel samples (those with normal levels of 27 kD gamma-zein protein and those with low levels of 27 kD gamma-zein protein) from segregating ears from the same events were analyzed using a small-scale simulated wet-milling process incorporating or leaving out a reducing agent (bisulfite) in the steep water (Eckhoff et al., (1996) Cereal Chem. 73:54-57). Similar to the digestibility assay, the reductant had a lesser impact on starch extractability in grain containing low levels of 27 kD gamma-zein protein compared to wild-type grain.

Eighty-three Pioneer inbred lines and 34 Pioneer hybrid lines, representing a wide spectrum of germplasm, were pollinated with pollen from a gamma-zein co-suppressing event with the result of greater than 95% suppression of gamma-zein protein in the hemizygous endosperm as determined by SDS-PAGE and immunoblotting. The same inbred and hybrid lines were grown at the same locations and self-pollinated to provide trait controls. Ears from the crossed inbred and hybrid plants as well as from the control plants were harvested and analyzed for EDDM at the four-hour time point (see Example 2). The results clearly indicated that ground corn from gamma-zein co-suppressed grain were more rapidly and extensively digested than corn with normal gamma-zein protein levels, by as much as 20% at the 4 hour time point. Moreover the grain digestibility of the crossed conversions obtained generally a similar high level of digestibility, i.e. grain conversions derived from poor digestible inbred and hybrid lines generally improved with a larger margin than grain from crosses with lines of higher digestibility (for example, at the 4 hour time point an improvement from 40% to 60% EDDM digestibility in a low digestible line and from 57% to 60% in a high digestible line).

Rumen *in situ* dry matter digestibility of co-suppressed (i.e. low) 27 kD gamma-zein corn in a top-cross onto three Pioneer grain hybrids: 3394, 35N05, and 32K61, were compared with a control top-cross and with the grain parent. Coarsely ground mature grain samples were weighed into pre-tared nylon bags (4 replicates each). The bags were sealed and placed in the rumen of a fistulated steer for 18hrs, then washed to remove microbial mass, ovendried, and weighed. Ruminal digestibility of the low gamma-zein grain was on average, 9% higher than the control top-cross and 5% higher than that of the grain parent. (See also: Nocek, J. E. 1988. In situ and other methods to estimate ruminal protein and energy digestibility. J. Dairy Sci. 71:2051-2069).

The same samples subjected to the in situ digestion procedure were also evaluated by an automated *in vitro* gas production method as described by Pell and Schofield (1993, *supra*). Coarsely ground mature grain samples were weighed into fermentation flasks (9 replicates each). The flasks were inoculated with buffered rumen fluid and incubated at 38°C for 24 hrs, during which the volume of the fermentation gas was automatically recorded. Average gas production was 2.5 and 3 gas volume (ml) higher for low gamma-zein topcrosses as compared to control top-cross and the grain parent at 15 hours after incubation with rumen fluid.

Immature kernels of various wild-type inbreds & hybrids, sampled at various stages of seed development and maturation, consistently respond to DTT pretreatment in the monogastric in vitro assay when sampled 1 month after pollination or later. The improvement in digestibility with DTT (20% at the 4 hour timepoint) points at a consistent inhibitory role of protein disulfide bonds on digestibility of wild-type kernels from about 28 DAP onwards. From these results one can conclude that kernels harvested at dough stage or silage maturity (approximately 40-45 DAP) would benefit from reduced gamma- zein levels. We also applied DTT pretreatment prior to monogastric in vitro digestion of 27 kD gamma-zein co-suppressed immature kernels (33 DAP), with no apparent effect, similar to our observations for low gamma-zein mature grain. Given the response to DTT for wild-type immature kernels from 28 DAP through maturity, the lack of DTT response for low gamma-zein kernels of any maturity, and the observed improvements in ruminal digestibility of mature low gamma-zein grain, one can deduce, with very high likelihood, that ruminal digestibility of silage maturity kernels will be improved with gamma-zein reduction.

Co-suppressed (i.e. low) 27 kD gamma-zein corn produced as a top-cross onto Pioneer grain hybrid 3394 was compared with a control top-cross and with the grain parent in a chicken feeding trial. A 21-day chick growth trial was performed with digestibility measurements, which demonstrated increased (by 2 percentage units) in vivo dry matter digestibility and increased energy conversion efficiency (by 2%) for the low gamma-zein topcross. In addition, in vivo protein digestibility was improved by 9 percentage units (from 69 to 78%), representing a 13% increase. The increase in protein digestibility resulted in a 29% decrease in nitrogen excretion into the environment.

The same low gamma-zein topcross was also compared with the control topcross in a pig in vivo digestion trial. Metabolizable Energy content of the low gamma zein topcross amounted to 3646 kcal/kg (15,300 kJ/kg), 73 kcal (305 kJ) (or 2%) higher than the control topcross. Protein digestibility was improved from 75.8 to 79.8% for the low gamma-zein topcross. This represents a 4% improvement in protein digestibility, and a 15% reduction in nitrogen excretion into the environment.

### Example 4: Suppression of 27 kD Gamma-Zein through Interruption of the 27 kD Gamma-Zein Gene by Transposon Tagging. (for reference purposed only)

A maize line containing a Mu-insertion in the 27kD gamma-zein coding region was isolated using the method of US Patent No. 5,962,764.

Briefly, a population of F1 maize plants produced by crossing *Mutator-*active lines (see Bennetzen et. al., Curr Top Microbiol Immunol 1996, 204:195-229 and Chandler, et al., Adv Genet 1992, 30:77-122) with inbred/hybrid lines and a collection of the F2 seed from each plant are available for screening of mutations (*Mu* insertion alleles) in genes of known sequence.

Prospective insertion alleles are identified by successive rounds of PCR/DNA dot blot hybridization (using gene-specific primers) first on DNA pools, subsequently on DNA from individuals, as described below:

Pool screening was initiated with 27kD gamma-zein primers 296 (SEQ ID NO:11) and 170 (SEQ ID NO:12) in combination with Mu TIR primer 9242 (SEQ ID NO:13). Pools were selected for fragment sizing based on signal intensity and reproducibility.

Bands were detected in fifteen of the sixteen pools selected for fragment sizing with primer 170.

In pool screening with primer 296 in conjunction with Mu TIR primer 9242, several intense, reproducible signals were detected. Sixteen pools were selected for fragment sizing from these signals; bands were detected by hybridization in three pools.

An individual master plate was constructed with the nine best pools for which bands were detected. The nine pools were selected based on the putative insertion location of mutator in the gene and possibly the promoter region.

Screening of individual alleles with primers 296 and 170 conjointly with *Mu* TIR primer 9242 was initiated. Several strong, reproducible signals were detected with both primers 296 and 170. Four individual alleles were cross confirmed with both primers. Seed was advanced for analysis based on pedigree relationship, signal intensity, and reproducibility. Non-mutant seed was used for negative controls.

Many Mu insertions detected by PCR are false-positives due to somatic insertion activity of Mu elements.

In confirmatory PCR, one Mu insertion allele in family PV03 80 B-06 appeared heritable. That is, the insertion was proven to be a germinal event into the 27kD gamma-zein gene that was able to transmit through the germline into F2 progeny. The heritable TUSC Mu insertion allele into the 27kD gamma zein gene was renamed "TUSC27"

The material originating from this source was advanced genetically, predominately via backcrossing strategies, to create material suitable for feeding trials, energy availability studies, and product development applications.

Grain from progeny of this TUSC27 line was tested in the *in vitro* digestibility assay (EDDM) with essentially similar results as observed with the 27 kD gamma-zein gene co-suppressed lines (see Example 3). Seed from this progeny is represented by, but not limited to, ATCC Dep. No:PTA-6323.

Four F2 hybrid grain lines containing TUSC27 were assayed for dry matter digestibility (EDDM) using the procedure of Example 2 against four wild-type lines. Two of the F2 hybrids in PHN46 background (PHP38 x PHN46, and PH09B x PHN46) showed 2-5% improvement in digestibility over the wild-type lines. The F2 hybrid in PH581 backgrounds (PH705 x PH581) showed 9% improvement and the hybrid in a PH3KP background (PH705 x PH3KP) showed 3% improvement over its wild-type line. All F2 hybrids showed 2-8% improvement in EDDM over the control hybrid 3335 - a commercially available "high digestibility" line not altered in seed storage protein content. All hybrid maize lines used have been deposited with the Patent Depository of the American Type Culture Collection (ATCC), Manassas, Virginia.

The trait was semi-dominant rather than recessive: that is the 27 kD gamma-zein level in endosperm showed a strong gene-dosage effect.

### Example 5: Suppression of 27 kD Gamma-Zein by Over-Expression of High-Sulfur Proteins through Competition for Biosynthetically Available Pools of Sulfur Amino Acids. (for reference purposes only)

Transgenic plants expressing the 18 kD delta-zein protein or the engineered high-lysine, high-sulfur protein hordothionin 12 (US Patent 5,990,389) in the endosperm showed an 80% decrease in gamma-zein protein levels, possibly due to limitations of free sulfur-amino acid pools. Seed from these events were tested essentially under the same conditions as seed from gamma-zein gene co-suppressing events (see Example 2) using the *in vitro* digestibility assay in both the presence and absence of disulfide reducing agents. The results obtained were similar to those described in the previous two Examples. The reduced levels of gamma-zein protein had a large positive impact on dry matter digestibility in the absence of DTT. Plants expressing high-sulfur protein showed 6% improvement in EDDM dry matter digestibility. The improvement in digestibility in this case is not as high as in CS27 (example 3). This could be due to the higher residual level of gamma zein in high-sulfur protein plants as compared to CS27. Maize plants ectopically expressing 18 kD delta-zein protein or hordothionin 12 protein in corn endosperm were both produced using the top-cross technology. Comparable results were also obtained using hemizygous seed from top-crossed elite inbreds and hybrids with hordothionin 12 corn as the male parent. Grain from these plants showed improved digestibility, and therefore improved energy availability.

### Example 6: Cloning of a Novel Maize 18 kD Alpha-globulin. (for reference)

An 18 kD alpha-globulin full-length cDNA (B73 allele) was cloned from a maize endosperm library (mid and late development). Based on EST numbers alpha-globulin transcripts are relatively rare (compared to other seed protein transcripts) and represent approximately 0.1 % of the endosperm mRNA during mid development. Different maize inbred lines showed considerable allelism including several SNP's. The 18 kD alpha-globulin gene has been located on chromosome 6, bin 6.05.

The coding region of the B73 allele is 618 bp. The encoded 206 amino acid sequence of the pro-polypeptide contains a predicted N-terminal ER import signal peptide of 23 amino acids. Remarkable is the string of tryptophane (W) residues ("tryptophane box"), which has been also observed in puroindolins from wheat. Puroindolins in wheat have been associated with grain hardness. The 18 kD alpha-globulin of the present invention and the puroindolins are distantly evolutionary related and belong both to the 2S albumin gene superfamily.

### SNP's and Alleles

Different corn inbred lines show considerable allelism. For example, sequence fragments isolated from B73 and Mo17 are shown below. The two alleles differ by mostly insertions '*' and a few SNP's (lower case bold). (See also SEQ ID NOS: 9 and 10).
18 kD alpha-globulin, B73 allele, partial 18 kD alpha-globulin_{.} Mo17 allele, partial

### Example 7: Transgenic Expression of 18 kD Alpha-globulin in Maize. (for reference)

The cDNA encoding the maize 18 kD alpha-globulin was placed under the control of the strong endosperm specific 27kD gamma-zein promoter and introduced into maize plants by Agrobacterium-mediated transformation. Several transgenic events were identified that had increased levels alpha-globulin protein as demonstrated by SDS-PAGE and staining of gels with Coomassie blue. A prominent band was visible at a molecular weight corresponding to the 18 kD protein extracted from transgenic seed, but absent from protein extracted similarly from wild type seed. The seed of transformants and progeny overexpressing 18kD alpha-globulin is phenotypically normal (vitreous).

The identity of the polypeptide migrating at 18 kD in the polyacrylamide gel was confirmed by immune blotting using 18 kD alpha-globulin protein specific antibodies. In seed of transgenic plants, the 18 kD alpha-globulin protein accumulated to levels of between 2-5% of the SDS-sample buffer (60 mM Tris, pH 6.8, 100 mM DTT, 2% SDS) extractable seed protein. Seed expressing these amounts of omega zein protein contained 0.162% tryptophan per dry weight compared to No. 2 yellow corn having 0.06% tryptophan amounting to a greater-than 100% increase in tryptophan levels. Also, sulfur amino acid content was increased by about 80%.

In vitro dry matter digestibility of corn overexpressing 18kD alpha-globulin was determined using the monogastric EDDM assay.

18kD Alpha-globulin overexpression resulted in improved 4 hr EDDM by 10.6 percentage units. An overnight soak in 10 mM of the strong reducing agent dithiothreietol (DTT), known to maximize in vitro digestibility. DTT treatment improved digestibility beyond that reached with 18kD alpha-globulin overexpression (by 3.4 percentage units), indicated that the improvement in digestibility attainable with removing digestion-limiting disulfide bonds is partially additive to the improvement obtained with alpha-globulin over-expression. Similarly, improvements made by combining gamma- zein co-suppression and 18 kD alpha-globulin overexpression can be expected to be partially additive.

### Example 8: Preparation of Maize 18 kD Alpha-globulin-Specific Antibodies. (for reference)

Standard methods for the production of antibodies were used such as those described in Harlow and Lane (1988) Antibodies: A Laboratory Manual (Cold Spring Harbor, NY: Cold Spring Harbor Laboratory. Specifically, antibodies for 18 kD alpha-globulin polypeptides were produced by injecting female New Zealand white rabbits (Bethyl Laboratory, Montgomery, Tex.) six times with homogenized polyacrylamide gel slices containing 100 micrograms of PAGE purified alpha-globulin polypeptide. The alpha-globulin polypeptide was purified by sub-cloning into a pET28 vector resulting in an insert encoding a His-tag fusion of the alpha-globulin polypeptide. The fusion protein was expressed in *E*. *coli* BL21 (DE3) cells and purified from the lysate by Nickel chelation chromatography. The denatured purified fusion protein was used for immunization.

Animals were then bled at two week intervals. The antibodies were further purified by affinity-chromatography with Affigel 15 (BioRad)-immobilized antigen as described by Harlow and Lane (1988) Antibodies: A Laboratory Manual, Cold Spring Harbor, N.Y. The affinity column was prepared with purified 18 kD alpha-globulin protein essentially as recommended by BioRad RTM. Immune detection of antigens on PVDF blots was carried out following the protocol of Meyer et al. (1988) J. Cell. Biol. 107:163, using the ECL kit from Amersham (Arlington Heights, III).

### Example 9: Cloning and Sequencing of Maize 50 kD Legumin 1 Protein. (for reference)

A 50 kD legumin 1 nucleotide sequence was cloned from a maize endosperm cDNA library (mid and late development). Based on EST numbers 50 kD legumin 1 transcripts are relatively abundant (compared to other seed protein transcripts) and represent approximately 0.5% of the endosperm mRNA during mid development. The 50 kD legumin 1 DNA sequences isolated from different inbred lines showed a considerable level of polymorphism. The 50 kD legumin 1 gene has been mapped to chromosome 6, Bin 6.01.

Corn legumin 1 has the unique property of missing an evolutionary conserved 11S globulin pro-protein cleavage site.

### Example 10: Preparation of Maize 50 kD Legumin 1-Specific Antibodies.

Antibodies to this protein were prepared essentially as described for the 18 kD alpha-globulin polypeptide. (for reference)

### Example 11: Transgenic Expression of 50 kD Legumin 1 in Maize. (for reference purposes only)

Additional copies of the 50 kD legumin 1 cDNA under control of the strong endosperm specific 27kD gamma-zein promoter were introduced into transgenic corn plants. Several maize lines were identified that over-express the 50 kD legumin 1 protein. Over-expression was demonstrated by SDS-PAGE and staining of the gels with Coomassie blue. A prominent band was visible at 50 kD in protein extracted from transgenic seed but absent in protein from wild type seed. The identity of the polypeptide band was confirmed to be the 50 kD legumin 1 protein by immune blotting using the 50 kD legumin 1 protein specific antibodies. In the seed of transgenic maize plants over-expressing the 50 kD legumin 1 protein, this protein accumulates to levels of between 2-5% of the SDS-sample buffer (60 mM Tris, pH 6.8, 100 mM DTT, 2% SDS) extractable seed protein. The seed over-expressing the 50 kD legumin 1 protein showed a normal (vitreous) phenotype. In addition to overexpression of the 50 kD legumin 1, independent transformants were also obtained in which the legumin 1 gene was silenced as evidenced by reduced protein level using immune blotting. These events were also silenced for the 27 kD gamma-zein, by apparent promoter-induced silencing. Finally, one event was obtained in which the 27 kD gamma-zein was silenced, but the 50 KD legumin 1 clearly overexpressed as assessed by SDS-PAGE/Coomassie blue staining and immune blotting. Seed from all these events were phenotypically normal (vitreous).

Two segregating events, both silenced for 27 kD gamma-zein, but only one overexpressing the corn legumin 1, were evaluated in the monogastric EDDM assay. 50 kD legumin 1 overexpression in low gamma-zein background resulted in improved grain digestibility by about 3.2 percentage units.

These results showed not only that overexpression of corn 50 kD legumin1 improves digestibility, but that these improvements are additive to those obtained with 27kD gamma-zein co-suppression.

### Example 12: Agrobacterium-Mediated Transformation of Maize.

For Agrobacterium-mediated transformation of maize, a nucleotide sequence encoding a protein tangeted in the present invention was operably linked to either the 27 kD gamma-zein promoter or the maize CZ19B1 promoter, and the method of Zhao was employed (U.S. Patent No. 5,981,840, and PCT patent publication WO98/32326). Briefly, immature embryos were isolated from maize and the embryos contacted with a suspension of *Agrobacterium,* where the bacteria are capable of transferring the nucleotide sequence of interest to at least one cell of at least one of the immature embryos (step 1: the infection step). In this step the immature embryos were immersed in an *Agrobacterium* suspension for the initiation of inoculation. The embryos were co-cultured for a time with the *Agrobacterium* (step 2: the co-cultivation step). The immature embryos were cultured on solid medium following the infection step. Following this co-cultivation period an optional "resting" step is contemplated. In this resting step, the embryos were incubated in the presence of at least one antibiotic known to inhibit the growth of *Agrobacterium* without the addition of a selective agent for plant transformants (step 3: resting step). The immature embryos were cultured on solid medium with antibiotic, but without a selecting agent, for elimination of *Agrobacterium* and for a resting phase for the infected cells. Next, inoculated embryos were cultured on medium containing a selective agent and growing transformed callus was recovered (step 4: the selection step). The immature embryos were cultured on solid medium with a selective agent resulting in the selective growth of transformed cells. The callus was then regenerated into plants (step 5: the regeneration step), and calli grown on selective medium were cultured on solid medium to regenerate the plants.

### Example 13. Transformation of Maize Embryos by Particle Bombardment.

Immature maize embryos from greenhouse donor plants are bombarded with a plasmid containing the nucleotide sequence encoding a protein targeted in present invention operably linked to a selected promoter plus a plasmid containing the selectable marker gene PAT (Wohlleben et al. (1988) Gene 70:25-37) that confers resistance to the herbicide Bialaphos. Transformation is performed as follows.
Preparation of Target Tissue The ears are surface sterilized in 30% Clorox bleach plus 0.5% Micro detergent for 20 minutes, and rinsed two times with sterile water. The immature embryos are excised and placed embryo axis side down (scutellum side up), 25 embryos per plate, on 560Y medium for 4 hours and then aligned within the 2.5-cm target zone in preparation for bombardment.

### Preparation of DNA

A plasmid vector comprising the nucleotide sequence encoding a protein targeted in the present invention operably linked to a promoter is made. This plasmid DNA plus plasmid DNA containing a PAT selectable marker is precipitated onto 1.1 µm (average diameter) tungsten pellets using a CaCl₂ precipitation procedure as follows:
100 µl prepared tungsten particles in water
10 µl (1 µg) DNA in Tris EDTA buffer (1 µg total)
100 µl 2.5 M CaC1₂
10 µl 0.1 M spermidine

Each reagent is added sequentially to the tungsten particle suspension, while maintained on the multitube vortexer. The final mixture is sonicated briefly and allowed to incubate under constant vortexing for 10 minutes. After the precipitation period, the tubes are centrifuged briefly, liquid removed, washed with 500 ml 100% ethanol, and centrifuged for 30 seconds. Again the liquid is removed, and 105 µl 100% ethanol is added to the final tungsten particle pellet. For particle gun bombardment, the tungsten/DNA particles are briefly sonicated and 10 µl spotted onto the center of each macrocarrier and allowed to dry about 2 minutes before bombardment.

### Particle Gun Treatment

The sample plates are bombarded at level #4 in particle gun #HE34-1 or #HE34-2. All samples receive a single shot at 650 PSI, with a total of ten aliquots taken from each tube of prepared particles/DNA.

### Subsequent Treatment

Following bombardment, the embryos are kept on 560Y medium for 2 days, then transferred to 560R selection medium containing 3 mg/liter Bialaphos, and subcultured every 2 weeks. After approximately 10 weeks of selection, selection-resistant callus clones are transferred to 288J medium to initiate plant regeneration. Following somatic embryo maturation (2-4 weeks), well-developed somatic embryos are transferred to medium for germination and transferred to the lighted culture room. Approximately 7-10 days later, developing plantlets are transferred to 272V hormone-free medium in tubes for 7-10 days until plantlets are well established. Plants are then transferred to inserts in flats (equivalent to 2.5" pot) containing potting soil and grown for 1 week in a growth chamber, subsequently grown an additional 1-2 weeks in the greenhouse, then transferred to classic 600 pots (1.6 gallon) and grown to maturity. Plants are monitored and scored for the desired phenotypic trait.

### Bombardment and Culture Media

Bombardment medium (560Y) comprises 4.0 g/l N6 basal salts (SIGMA C-1416), 1.0 ml/l Eriksson's Vitamin Mix (1000X SIGMA-1511), 0.5 mg/l thiamine HCl, 120.0 g/l sucrose, 1.0 mg/l 2.4-D, and 2.88 g/l L-proline (brought to volume with D-I H₂0 following adjustment to pH 5.8 with KOH); 2.0 g/l Gelrite (added after bringing to volume with D-I H₂0); and 8.5 mg/l silver nitrate (added after sterilizing the medium and cooling to room temperature). Selection medium (560R) comprises 4.0 g/l N6 basal salts (SIGMA C-1416), 1.0 ml/l Eriksson's Vitamin Mix (1000X SIGMA-1511), 0.5 mg/l thiamine HCl, 30.0 g/l sucrose, and 2.0 mg/l 2,4-D (brought to volume with D-I H₂0 following adjustment to pH 5.8 with KOH); 3.0 g/l Gelrite (added after bringing to volume with D-I H₂0); and 0.85 mg/l silver nitrate and 3.0 mg/l bialaphos(both added after sterilizing the medium and cooling to room temperature).

Plant regeneration medium (288J) comprises 4.3 g/l MS salts (GIBCO 11117-074), 5.0 ml/l MS vitamins stock solution (0.100 g nicotinic acid, 0.02 g/l thiamine HCL, 0.10 g/l pyridoxine HCL, and 0.40 g/l glycine brought to volume with polished D-I H₂0) (Murashige and Skoog (1962) *Physiol. Plant. 15*:473), 100 mg/l myo-inositol, 0.5 mg/l zeatin, 60 g/l sucrose, and 1.0 ml/l of 0.1 mM abscisic acid (brought to volume with polished D-I H₂0 after adjusting to pH 5.6); 3.0 g/l Gelrite (added after bringing to volume with D-I H₂0); and 1.0 mg/l indoleacetic acid and 3.0 mg/l bialaphos (added after sterilizing the medium and cooling to 60°C). Hormone-free medium (272V) comprises 4.3 g/l MS salts (GIBCO 11117-074), 5.0 ml/l MS vitamins stock solution (0.100 g/l nicotinic acid, 0.02 g/l thiamine HCL, 0.10 g/l pyridoxine HCL, and 0.40 g/l glycine brought to volume with polished D-I H₂0), 0.1 g/l myo-inositol, and 40.0 g/l sucrose (brought to volume with polished D-I H₂0 after adjusting pH to 5.6); and 6 g/l bacto-agar (added after bringing to volume with polished D-I H₂0), sterilized and cooled to 60°C.

### Example 14: Breeding crosses made with transgenic or mutant high-digestible-grain corn lines. (for reference)

A transgenic line that segregated for co-suppressed 4-coumarate ligase (4CL) was planted and the segregating progeny was either self-fertilized or pollinated with the transgenic low gamma-zein line. Ground grain samples were subjected to a two-stage *in*-*vitro*-mimicking small intestinal digestion procedure as described in Example 2 followed by an additional step that included viscozyme that mimics large-intestinal fermentation (Boisen and Fernandez, 1997, Animal Feed Science and Technology 68:277-286). The improvement in digestibility for low gamma-zein corn in this two-stage assay, and the independent improvement obtained with 4CL co-suppression were additive as indicated by an approximately 2% increase in EDDM value for 4CL grains that was top-crossed with CS27 pollen as compared to 4CL grains top-crossed with control pollen.

### Example 15: Transgenic Down Regulation of Alpha-Zein (CS19,CSAZ). (for reference)

Expression cassettes were made comprising a chimeric maize alpha-zein fusion of polynucleotide fragments from the coding regions of 19KD alpha-zein clone D1 (563 bp), 19KD alpha-zein clone B1 (536 bp), and 22KD alpha-zein (610 bp) (SEQ ID NO: 16). The cassette included a selectable marker gene such as PAT (Wohlleben et al (1988) Gene 70:25-37, or BAR for resistance to Basta/phosphinothricin were constructed. The polynucleotides were operably linked to the CZ19B1 promoter to direct expression to maize endosperm. The construction of such expression cassettes is well known to those of skill in the art in light of the present disclosure. See, for example, e.g., Sambrook et al. (1989) Molecular Cloning: A Laboratory Manual (Cold Spring Harbor, Laboratory Press, Plainview, NY; Gelvin et al. Plant Molecular Biology Manual (1990) .

Corn was transformed as described in Example 13 using particle bombardment. Seeds from 30 transgenic events were analyzed for protein profiles by SDS-PAGE and more than 20% of the events showed segregating kernels with suppressed (>90% suppression of the alpha-zein protein when compared to non-transgenic control seed) alpha- zein protein levels (designated as CS-AZ). One event showed suppression of only the 19kD alpha-zein (designated as CS19).

Total alpha-zein suppression (CS-AZ) resulted in improved 4 hr EEDM by 7 percentage units. Similarly, 19 kD alpha-zein suppression (CS19) resulted in improved 4 hr EDDM by 4.9 percentage units. An overnight soak in 10 mM of the strong reducing agent dithiothreietol (DTT) is known to maximize *in vitro* digestibility. Digestibility improved beyond that reached with 19 kD alpha-zein co-suppression (by 4.3 percentage units), indicated that the improvement in digestibility attainable with reduction of alpha-zein protein is partially additive to the improvement obtained with 19 kD alpha-zein co-suppression. Similarly, improvements made by combining 27kD gamma-zein co-suppression and total alpha-zein suppression or with 19 kD alpha-zein co-suppression over-expression have been found to be to be partially additive (See Example 20).

### Example 16: Combination of over-expressed corn legumin and co-suppressed 27kD gamma-zein.

A construct is made that links in tandem a selectable marker gene, a corn legumin over-expression cassette and a 27KD gamma-zein co-suppression cassette. The selectable marker gene consists of PAT for resistance to Basta/phosphinothricin. The corn legumin coding sequence is placed under the transcriptional control of the maize GZ-W64A promoter and terminator (SEQ ID NOs:26 and 27) (Reina, M. et al, (1990), Nucleic Acids Res., 18:6426) to drive expression in maize endosperm as described in Example 11. The 27KD gamma-zein co-suppression cassette contains the CZ19B1 promoter, and an inverted repeat with 27 kD gamma-zein cDNA fragments as described in Example 3. The entire construct of linked cassettes is transformed into maize by Agrobacterium-mediated transformation and resulting T0 plants are pollinated by a non-transgenic male parent corn plant.

Seed from transgenic events are analyzed for their seed protein profile by SDS-PAGE and Coomassie-staining of gels. In the protein profiles of kernels, about 90% of events indicate a 1:1 segregation of wildtype seed protein profiles and altered seed protein profile phenotypes. About 90% of the transgenic maize events generated with this construct are found to contain segregating kernels that are reduced in 27 kD-, and 16 kD-gamma-zein by at least 50% (dry weight; i.e.: "low gamma-zein phenotype") and about 30% of the transgenic events are found to contain segregating kernels that are increased in corn legumin by at least 200% (dry weight; i.e.: "high corn legumin protein phenotype") in addition to the reduced gamma-zein protein phenotype. The kernel phenotype of the transgenic seed is normal (i.e., vitreous).

Segregating seed from low gamma-zein/ high corn legumin protein transgenic events are analyzed by SDS-PAGE for their seed protein phenotypes and sorted into two batches of 50 seed each. One batch contains seed with wild type seed protein phenotype (controls) and the second batch contains seed with low gamma- zein/ high corn legumin protein phenotypes. The seed of both batches are ground and analyzed by the EDDM assay. The analysis shows improved EDDM digestibility of the seed sample with low gamma-zein/ high corn legumin protein phenotypes compared to the seed sample with the wild type seed protein phenotype.

Moreover, the EDDM improvement in the sample derived from low gamma-zein/ high corn legumin seed is greater than in seed samples with only a low 27kD gamma-zein content (Example 3) or in seed samples with only a high corn legumin protein content (Example 11); showing a partially additive effect of the suppression of gamma-zein and the increased expression of corn legumin on grain digestibility.

### Example 17: Combination of over-expressed corn alpha-globulin and co-suppressed 27kD gamma-zein.

A construct is made comprising a tandem-linked corn alpha-globulin over-expression cassette, a 27KD gamma-zein co-supression cassette, and selectable marker cassette. The selectable marker cassette consists of the ubiquitin promoter, PAT selectable marker (for resistance to Basta/phosphinothricin) and PINII terminator. The corn alpha-globulin coding sequence (SEQ ID NO:3) is placed under the transcriptional control of the maize GZ-W64A promoter and terminator (SEQ ID NOs:26 and 27) to drive expression in maize endosperm as described in Example 11. The 27KD gamma-zein co-suppression cassette contains the CZ19B1 promoter, and an inverted repeat with 27 kD gamma-zein cDNA fragments as described in Example 3. The entire construct of linked cassettes is transformed into maize by Agrobacterium-mediated transformation and resulting T0 plants are pollinated by a nontransgenic male parent corn plant.

Seed from transgenic events are analyzed for their seed protein profile by SDS-PAGE and Coomassie-staining of gels. The protein profiles of kernels in about 90% of events indicate a 1:1 segregation of wildtype seed protein profiles to altered seed protein profile phenotypes. About 90% of the transgenic maize events generated with this construct are found to contain segregating kernels that are reduced in 27 kD-, and 16 kD-gamma-zein by at least 50% (dry weight; e.g.: "low gamma-zein phenotype") and about 30% of the transgenic events are found to contain segregating kernels that are increased in corn alpha-globulin by at least 500% (dry weight; e.g.: "high corn alpha-globulin protein phenotype") in addition to the reduced gamma-zein protein phenotype. The kernel phenotype of the transgenic seed is normal (i.e., vitreous).

Segregating seed from low gamma-zein/ high corn alpha-globulin protein transgenic events are analyzed by SDS-PAGE for their seed protein phenotypes and sorted into two batches of 50 seed each. One batch contains seed with wild type seed protein phenotype (controls) and the second batch contains seed with low gamma-zein/ high corn alpha-globulin protein phenotypes.

The seed of both batches are ground and analyzed by the EDDM assay. The analysis shows improved EDDM digestibility of the sample with seed with low gamma-zein/ high corn alpha-globulin protein phenotypes compared to the EDDM digestibility of the wild type seed sample. Moreover, the EDDM improvement in the low gamma-zein/ high corn alpha-globulin seed is greater than in seed samples with only a low 27kD gamma-zein content (Example 3), or in seed samples with only a high corn alpha-globulin protein content (Example 7), demonstrating a partially additive effect on grain digestibility of the suppression of gamma-zein and the increased expression of corn alpha-globulin.

### Example 18: Transgenic Over-Expression of Combination of Alpha-Globulins and Corn Legumin 1. (for reference)

A construct is made comprising a tandem-linked corn alpha-globulin over-expression cassette, a corn legumin over-expression cassette and selectable-marker cassette. The selectable marker cassette consists of the ubiquitin promoter, PAT selectable marker (for resistance to Basta/phosphinothricin) and PINII terminator. The corn alpha-globulin coding sequence is placed under the transcriptional control of the maize floury2 22kD alpha-zein promoter and terminator to drive expression in maize endosperm. The corn legumin over-expression cassette contains the corn legumin coding sequence placed under the transcriptional control of the maize GZ-W64A promoter and terminator to drive expression in maize endosperm as described in Example 11. The entire construct of linked cassettes is transformed into maize by Agrobacterium-mediated transformation and resulting T0 plants are pollinated by a nontransgenic male parent corn plant.

Seed from transgenic events are analyzed for their seed protein profile by SDS-PAGE and Coomassie-staining of gels. The protein profiles of kernels in about 90% of events indicate a 1:1 segregation of wild-type seed protein profiles to altered seed protein profile phenotypes. About 50% of the transgenic maize events generated with this construct are found to contain segregating kernels that are increased in corn alpha-globulin by at least 500% (dry weight; e.g.: "high corn alpha-globulin protein phenotype") and in corn legumin by at least 200% (dry weight; e.g.: "high corn legumin phenotype"). The kernel phenotype of the transgenic seed is normal (i.e., vitreous).

Segregating seed from high corn legumin/ high corn alpha-globulin protein transgenic events are analyzed by SDS-PAGE for their seed protein phenotypes and sorted into two batches of 50 seed each. One batch contains seed with wild type seed protein phenotype (controls) and the second batch contains seed with high corn legumin/ high corn alpha-globulin protein phenotypes. The seed of both batches are ground and analyzed by the EDDM assay. The analysis shows improved EDDM digestibility of the seed sample with high corn legumin/ high corn alpha-globulin protein phenotypes compared to the EDDM digestibility of the seed sample with wild- type seed protein phenotype. Moreover, the EDDM improvement in the sample derived from high corn legumin/ high corn alpha-globulin seed is greater than in samples from seed with only a high corn legumin content (Example 11) or in samples from seed with only a high corn alpha-globulin protein content (Example 7), demonstrating a partially additive effect of the increased expression of corn legumin and the increased expression of corn alpha-globulin on grain digestibility.

### Example 19: Down Regulation of 27kD gamma-zein and alpha-zeins.

A construct is made comprising a tandem-linked selectable marker cassette, and a 27KD gamma-zein/ 22kD alpha-zein/19kD alpha-zein clone B/19kD alpha-zein clone D co-supression cassette. The selectable marker cassette consists of the ubiquitin promoter, PAT selectable marker (for resistance to Basta/phosphinothricin) and PINII terminator. The co-suppression cassette contains the CZ19B1 promoter and an inverted repeat with fragments of the 27 kD gamma-zein cDNA, the 19kD alpha-zein B1 cDNA, the 19kD alpha-zein D1 cDNA and the 22kD alpha-zein 1 cDNA (SEQ ID NO:17). The transcriptional fusion is arranged as an inverted repeat around a spliceable ADH1 INTRON1 to effect silencing of all genes and genes highly similar to genes represented in the fusion.

The entire construct of linked cassettes is transformed into maize by Agrobacterium-mediated transformation and resulting T0 plants are pollinated by a nontransgenic male parent corn plant.

Seed from transgenic events are analyzed for their seed protein profile by SDS-PAGE and Coomassie-staining of gels. The protein profiles of kernels in about 90% of events indicate a 1:-1 segregation of wild-type seed protein profiles to altered seed protein profile phenotypes. About 90% of the transgenic maize events generated with this construct are found to contain segregating kernels that are reduced in 27 kD- and 16 kD-gamma-zein, all 22kD alpha-zein proteins, and all 19kD alpha-zein proteins. These zeins are suppressed by at least 90% (dry weight; e.g.: "low gamma-zein/low alpha-zein phenotype") in about 90% of the altered seed protein events. Segregating seed from low gamma-zein/ low alpha-zein transgenic events are analyzed by SDS-PAGE for their seed protein phenotypes and sorted into two batches of 50 seed each. One batch contains seed with wild type seed protein phenotype (controls) and the second batch contains seed with low gamma-zein/ low alpha-zein protein phenotypes.

The seed of both batches are ground and analyzed by the EDDM assay. The analysis shows improved EDDM digestibility of the seed sample with low gamma-zein/ low alpha-zein protein phenotypes compared to the wild-type seed sample. Moreover, the EDDM improvement in the low gamma-zein/low alpha-zein seeds sample is greater than in seed samples with only a low 27kD gamma-zein content (Example 3) or in seed samples with only a low alpha-zein protein content (Example 15); demonstrating a partially additive effect of the suppression of gamma-zein and the suppression of alpha-zeins on grain digestibility.

### Example 20: Combined Down-regulation of 27kD and 16kD gamma-zein, 22kD and 19kD alpha-zein and over-expression of alpha-globulin, 15kD beta-zein, and 18kD delta zein.

A stacked construct (SEQ ID NO: 17) was constructed which included the following expression cassettes: floury2 promoter::18KD alpha-globulin coding sequence::floury2 terminator; ZM-LEG1A PRO (the promoter from the maize legumin gene): HSZ (high sulfur zein) coding sequence::ZM-LEG1 terminator; GZ-W64A Pro::15KD Beta Zein coding sequence::GZ-W64A terminator; and the CZ19B1 promoter driving a transcriptional fusion of fragments of 27KD gamma-zein, both 19KD alpha-zeins, 22KD alpha-zein, and Lysine Ketoglutarate Reductase (LKR; Arruda P, et al, (2000), Trends Plant Sci. 5:324-330). The transcriptional fusion was arranged as an inverted repeat around a spliceable ADH1 INTRON1 to effect silencing of all five genes represented in the fusion.

The entire construct of linked cassettes was transformed into maize by Agrobacterium-mediated transformation. 361 T0 plants derived from 252 independent events were obtained and pollinated by a nontransgenic male parent corn plant. From those 361 ears, 302 ears contained more than 50 kernels per ear.

Four to six seed from each of these ears were analyzed by SDS-PAGE for their protein profiles. This analysis identified 273 ears (90%) that contained seeds segregating for altered seed protein profiles: that is the 27kD- and 16kD gamma-zeins, all 22kD- and all 19kD alpha-zein proteins were suppressed by at least 90% (dry weight) Strong protein bands migrating in SDS-PAGE gels with apparent molecular weights of 15kD, 18kD and 50kD were observed.

The identity of the 15kD protein as the 15kD beta-zein, of the 18kD protein as the 18kD corn alpha-globulin and 18kD delta-zein proteins and of the 50kD protein as the 50kD corn legumin was confirmed by immuno-blotting using beta-zein, alpha- globulin, delta-zein and corn legumin specific antibodies (Woo et al) with seed extracts from 9 selected events. Moreover, the suppression of LKR was also confirmed in these events by immuno-blotting. These results demonstrated a very high rate of combined efficacy of the co-suppression cassette and the linked over-expression cassettes.

Segregating seed from 9 events showing the low gamma-zein/ low alpha-zein/high corn legumin/high alpha-globulin/high 18kD delta-zein/high beta-zein phenotype ("altered seed protein phenotype") were analyzed by SDS-PAGE for their seed protein phenotypes. Each event was sorted into two batches of 50 seeds each. The seed weights of each pair of batches were determined and showed no significant difference.

Meal was prepared from each sample (nine events, two batches each) and analyzed for its amino acid composition and protein content. For all events, the average protein content was reduced in the altered seed protein phenotype samples by 10% (dry weight), the lysine content was increased by >70% (dry weight) and the tryptophane content was increased by >60% (dry weight).

Meal was prepared from each of the pairs of batches of six events and analyzed for EDDM at the 6hr. time point. The altered seed protein phenotype samples showed, on average, an improvement of 9.8 percentage points compared to the segregating control samples.

In the segregating control, an overnight soak in 10 mM of dithiothreietol (DTT), improved digestibility by 3.7 percentage units. In transgenic corn there was no additional improvement in digestibility. The effect of DTT on digestibility mimics the impact of the suppression of gamma-zein on grain digestibility. Thus the difference between the improved digestibility of 3.7 percentage units due to DTT treatment and the improved digestibility in the altered seed protein phenotype samples (9.8 percentage units) was an indicator for the added digestibility improvement to EDDM digestibility attributable to the suppression of alpha-zein proteins, overexpression of alpha-globulin and overexpression of corn legumin. This demonstrated that digestibility of the transgenic, altered seed protein phenotype, grains improved beyond that attainable by only removing digestion-limiting disulfide bonds (associated with gamma-zein), or by only removing alpha-zeins or by only over-expressing either alpha-globulin or corn legumin as single transgene events. The digestibility trait of the combination of co-suppression and over-expression cassettes was partially additive to digestibility effects obtained with the non-combined, co-suppression or over- expression cassettes in independent transgenic events.

All publications and patent applications mentioned in the specification are indicative of the level of those skilled in the art to which this invention pertains.

## Claims

1. A genetically modified maize plant wherein said genetic modification is selected from:
(a) co-suppression or homology-dependent gene silencing of a 27 kD gamma-zein and said plant has a stably decreased level of a 27 kD and 16 kD gamma-zein protein in the grain of the modified maize plant due to said modification; and
(b) co-suppression or homology-dependent gene silencing of a 50 kD gamma-zein and said plant has a stably decreased level of a 27 kD, 50 kD and 16 kD gamma-zein protein in the grain of the modified maize plant due to said modification;
and wherein the grain of the genetically modified maize plant has a hard, vitreous endosperm.

2. A plant according to claim 1 having a genetic modification targeting 27 kD and 50 kD gamma-zein.

3. A plant according to claim 1 or 2 further comprising a genetic modification targeting 16 kD gamma-zein.

4. A plant according to any one of the preceding claims further comprising a genetic modification targeting 15 kD beta-zein.

5. A plant according to any one of the preceding claims further comprising a genetic modification targeting one or more alpha-zeins.

6. A plant according to preceding claim 5 wherein the alpha-zein in selected from 22 kD alpha-zein and 19 kD alpha-zein.

7. A plant according to any one of the preceding claims further comprising increased expression, compared to the corresponding unmodified plant, of a nucleotide sequence that encodes the maize 18 kD alpha-globulin or a 50 kD legumin protein.

8. A plant according to any one of the preceding claims further comprising increased expression, compared to the corresponding unmodified plant, of a nucleotide sequence that encodes the maize 18 kD delta zein protein.

9. Seed or grain of the plant of claim 1 having decreased level of a 27 kD and 16 kD gamma-zein protein compared to seed or grain of the corresponding unmodified plant.

10. Seed or grain of the plant of claim 1 having decreased level of 27 kD, 50 kD gamma-zein protein, and 16 kD gamma-zein protein compared to seed or grain of the corresponding unmodified plant.

11. Seed or grain of claim 9 or 10 further having a decrease in one or more alpha-zein protein compared to seed or grain of the corresponding unmodified plant.

12. Seed or grain of the plant of claim 7 having increased levels of maize 18 kD alpha-globulin or maize 50 kD legumin protein compared to seed or grain of the corresponding unmodified plant.

13. Seed or grain of the plant of claim 8 having increased levels of maize 18 kD delta zein protein compared to seed or grain of the corresponding unmodified plant.

## Patentansprüche

1. Genetisch modifizierte Maispflanze, wobei die genetische Modifizierung ausgewählt ist aus:
(a) Cosuppression oder Homologie-abhängige Genabschaltung von einem 27-kD-gamma-Zein, und die Pflanze hat ein stabil vermindertes Niveau von einem 27-kD-und 16-kD-gamma-Zein-Protein in dem Korn der modifizierten Maispflanze auf Grund der Modifizierung; und
(b) Cosuppression oder Homologie-abhängige Genabschaltung von einem 50-kD-gamma-Zein, und die Pflanze hat ein stabil vermindertes Niveau von einem 27-kD-, 50-kD- und 16-kD-gamma-Zein-Protein in dem Korn der modifizierten Maispflanze auf Grund der Modifizierung;
und wobei das Korn der genetisch modifizierten Maispflanze ein hartes glasartiges Endosperm hat.

2. Pflanze gemäß Anspruch 1 mit einer genetischen Modifizierung, die auf 27-kD-und 50-kD-gamma-Zein zielt.

3. Pflanze gemäß Anspruch 1 oder 2, weiterhin umfassend eine genetische Modifizierung, die auf 16-kD-gamma-Zein zielt.

4. Pflanze gemäß einem der vorhergehenden Ansprüche, weiterhin umfassend eine genetische Modifizierung, die auf 15-kD-beta-Zein zielt.

5. Pflanze gemäß einem der vorhergehenden Ansprüche, weiterhin umfassend eine genetische Modifizierung, die auf ein oder mehrere alpha-Zeine zielt.

6. Pflanze gemäß dem vorhergehenden Anspruch 5, wobei das alpha-Zein aus 22-kD-alpha-Zein und 19-kD-alpha-Zein ausgewählt ist.

7. Pflanze gemäß einem der vorhergehenden Ansprüche, weiterhin umfassend erhöhte Expression, verglichen mit der entsprechenden unmodifizierten Pflanze, von einer Nucleotidsequenz, die das Mais-18-kD-alpha-Globulin- oder ein -50-kD-Legumin-Protein codiert.

8. Pflanze gemäß einem der vorhergehenden Ansprüche, weiterhin umfassend erhöhte Expression, verglichen mit der entsprechenden unmodifizierten Pflanze, von einer Nucleotidsequenz, die das Mais-18-kD-delta-Zein-Protein codiert.

9. Samen oder Korn von der Pflanze nach Anspruch 1 mit vermindertem Niveau von einem 27-kD- und 16-kD-gamma-Zein-Protein, verglichen mit Samen oder Korn der entsprechenden unmodifizierten Pflanze.

10. Samen oder Korn von der Pflanze nach Anspruch 1 mit vermindertem Niveau von 27-kD-, 50-kD-gamma-Zein-Protein und 16-kD-gamma-Zein-Protein, verglichen mit Samen oder Korn von der entsprechenden unmodifizierten Pflanze.

11. Samen oder Korn nach Anspruch 9 oder 10, weiterhin mit einer Verminderung in einem oder mehreren alpha-Zein-Proteinen, verglichen mit Samen oder Korn von der entsprechenden unmodifizierten Pflanze.

12. Samen oder Korn von der Pflanze nach Anspruch 7 mit erhöhten Niveaus von Mais-18-kD-alpha-Globulin- oder Mais-50-kD-Legumin-Protein, verglichen mit Samen oder Korn von der entsprechenden unmodifizierten Pflanze.

13. Samen oder Korn von der Pflanze nach Anspruch 8 mit erhöhten Niveaus von Mais-18-kD-delta-Zein-Protein, verglichen mit Samen oder Korn von der entsprechenden unmodifizierten Pflanze.

## Revendications

1. Plante de maïs génétiquement modifiée, dans laquelle ladite modification génétique est choisie parmi:
(a) une co-suppression ou une atténuation de gène dépendant de l'homologie d'une gamma-zéine de 27 kD et ladite plante possède un niveau diminué de façon stable d'une protéine de gamma-zéine de 27 kD et de 16 kD dans la graine de la plante de maïs modifiée dû à ladite modification; et
(b) une co-suppression ou une atténuation de gène dépendant de l'homologie d'une gamma-zéine de 50 kD et ladite plante possède un niveau diminué de façon stable d'une protéine de gamma-zéine de 27 kD, de 50 kD et de 16 kD dans la graine de la plante de maïs modifiée dû à ladite modification;
et dans laquelle la graine de la plante de maïs génétiquement modifiée présente un endosperme dur, vitreux.

2. Plante selon la revendication 1, ayant une modification génétique ciblant une gamma-zéine de 27 kD et de 50 kD.

3. Plante selon la revendication 1 ou 2, comprenant en outre une modification génétique ciblant une gamma-zéine de 16 kD.

4. Plante selon l'une quelconque des revendications précédentes, comprenant en outre une modification génétique ciblant une bêta-zéine de 15 kD.

5. Plante selon l'une quelconque des revendications précédentes, comprenant en outre une modification génétique ciblant une ou plusieurs alpha-zéines.

6. Plante selon la revendication 5 précédente, dans laquelle l'alpha-zéine est choisie parmi une alpha-zéine de 22 kD et une alpha-zéine de 19 kD.

7. Plante selon l'une quelconque des revendications précédentes, comprenant en outre une expression accrue, comparée à la plante non modifiée correspondante, d'une séquence de nucléotides qui code pour l'alpha-globuline de 18 kD de maïs ou une protéine de légumine de 50 kD.

8. Plante selon l'une quelconque des revendications précédentes, comprenant en outre une expression accrue, comparée à la plante non modifiée correspondante, d'une séquence de nucléotides qui code pour la protéine de delta-zéine de 18 kD de maïs.

9. Semence ou graine de la plante selon la revendication 1, possédant un niveau diminué d'une protéine de gamma-zéine de 27 kD et de 16 kD comparé à celui de la semence ou de la graine de la plante non modifiée correspondante.

10. Semence ou graine de la plante selon la revendication 1, possédant un niveau diminué d'une protéine de gamma-zéine de 27 kD, de 50 kD et d'une protéine de gamma-zéine de 16 kD comparé à celui de la semence ou de la graine de la plante non modifiée correspondante.

11. Semence ou graine selon la revendication 9 ou 10, possédant une diminution dans une ou plusieurs protéines d'alpha-zéines comparée à celle de la semence ou de la graine de la plante non modifiée correspondante.

12. Semence ou graine de la plante selon la revendication 7, possédant des niveaux accrus d'alpha-globuline de 18 kD de maïs ou de protéine de légumine de 50 kD de maïs comparés à ceux de la semence ou de la graine de la plante non modifiée correspondante.

13. Semence ou graine de la plante selon la revendication 8, possédant des niveaux accrus de protéine de delta-zéine de 18 kD de maïs comparés à ceux de la semence ou de la graine de la plante non modifiée correspondante.


### SEQUENCE LISTING

<110> Pioneer Hi-Bred International, Inc.
<120> Improved Grain Quality Through Altered
   Expression of Seed Proteins
<130> 1276-PCT
<160> 27
<170> FastSEQ for Windows Version 4.0
<210> 1
   <211> 1129
   <212> DNA
   <213> Zea mays
<220>
   <221> CDS
   <222> (120)...(1004)
   <223> 50 kD gamma-zein prolamin/PTA 2272
<400> 1
   gtgtatttgc actcatgcat cacaaaacat ccttctatca gtaccatcaa tcatcattca    60
   tcttagtagt ataggcacca aatcaaatct gcaacatcaa ttatctaact ccaaaaacc    119
   atg aag ctg gtg ctt gtg gtt ctt gct ttc att gct tta gta tca agt     167
   Met Lys Leu Val Leu Val Val Leu Ala Phe Ile Ala Leu Val Ser Ser
    1               5                  10                  15
   gtt tct tgt aca cag aca ggc ggc tgc agc tgt ggt caa caa caa agc     215
   Val Ser Cys Thr Gln Thr Gly Gly Cys Ser Cys Gly Gln Gln Gln Ser
               20                  25                  30
   cat gag cag caa cat cat cca caa caa cat cat cca caa aaa caa caa     263
   His Glu Gln Gln His His Pro Gln Gln His His Pro Gln Lys Gln Gln
           35                  40                  45
   cat caa cca cca cca caa cat cac cag cag cag caa cac caa caa caa     311
   His Gln Pro Pro Pro Gln His His Gln Gln Gln Gln His Gln Gln Gln
       50                  55                  60
   caa gtt cac atg caa cca caa aaa cat cag caa caa caa gaa gtt cat     359
   Gln Val His Met Gln Pro Gln Lys His Gln Gln Gln Gln Glu Val His
   65                  70                  75                  80
   gtt caa caa caa caa caa caa ccg cag cac caa caa caa caa caa caa     407
   Val Gln Gln Gln Gln Gln Gln Pro Gln His Gln Gln Gln Gln Gln Gln
                   85                  90                  95
   caa cag cac caa caa caa cat caa tgt gaa ggc caa caa caa cat cac     455
   Gln Gln His Gln Gln Gln His Gln Cys Glu Gly Gln Gln Gln His His
               100                 105                 110
   caa caa tca caa ggc cat gtg caa caa cac gaa cag agc cat gag caa     503
   Gln Gln Ser Gln Gly His Val Gln Gln His Glu Gln Ser His Glu Gln
           115                 120                 125
   cac caa gga cag agc cat gag caa caa cat caa caa caa ttc cag ggt     551
   His Gln Gly Gln Ser His Glu Gln Gln His Gln Gln Gln Phe Gln Gly
       130                 135                 140
   cat gac aag cag caa caa cca caa cag cct cag caa tat cag cag ggc      599
   His Asp Lys Gln Gln Gln Pro Gln Gln Pro Gln Gln Tyr Gln Gln Gly
   145                 150                 155                 160
   cag gaa aaa tca caa cag caa caa tgt cat tgc cag gag cag caa cag      647
   Gln Glu Lys Ser Gln Gln Gln Gln Cys His Cys Gln Glu Gln Gln Gln
                   165                 170                 175
   act aca agg tgc agc tat aac tac tat agc agt agc tca aat cta aaa      695
   Thr Thr Arg Cys Ser Tyr Asn Tyr Tyr Ser Ser Ser Ser Asn Leu Lys
               180                 185                 190
   aat tgt cat gaa ttc cta agg cag cag tgc agc cct ttg gta atg cct      743
   Asn Cys His Glu Phe Leu Arg Gln Gln Cys Ser Pro Leu Val Met Pro
           195                 200                 205
   ttt ctc caa tca cgt ttg ata caa cca agt agc tgc cag gta ttg cag      791
   Phe Leu Gln Ser Arg Leu Ile Gln Pro Ser Ser Cys Gln Val Leu Gln
       210                 215                 220
   caa caa tgt tgt cat gat ctt agg cag att gag cca caa tac att cac      839
   Gln Gln Cys Cys His Asp Leu Arg Gln Ile Glu Pro Gln Tyr Ile His
   225                 230                 235                 240
   caa gca atc tac aac atg gtt caa tcc ata atc cag gag gag caa caa      887
   Gln Ala Ile Tyr Asn Met Val Gln Ser Ile Ile Gln Glu Glu Gln Gln
                   245                 250                 255
   caa caa cca tgt gag tta tgt gga tct caa caa gct act cca aag tgc      935
   Gln Gln Pro Cys Glu Leu Cys Gly Ser Gln Gln Ala Thr Pro Lys Cys
               260                 265                 270
   ggt ggc aat ctt gac agc agc aca ata cct acc atc aat gtg cgg ctt      983
   Gly Gly Asn Leu Asp Ser Ser Thr Ile Pro Thr Ile Asn Val Arg Leu
           275                 280                 285
   gta cca ctc ata cta cca aaa taatccatgc agcagcaatg acattagtgg        1034
   Val Pro Leu Ile Leu Pro Lys
       290                 295
   tgtttgcaat tgaagaattg tgtctaccta gccgttatac tcatataacg gtgttaagca   1094
   ataaagtacc atacattatg atgttaaaaa aaaaa                               1129
<210> 2
   <211> 295
   <212> PRT
   <213> Zea mays
<400> 2
   Met Lys Leu Val Leu Val Val Leu Ala Phe Ile Ala Leu Val Ser Ser
   1               5                   10                  15
   Val Ser Cys Thr Gln Thr Gly Gly Cys Ser Cys Gly Gln Gln Gln Ser
               20                  25                  30
   His Glu Gln Gln His His Pro Gln Gln His His Pro Gln Lys Gln Gln
           35                  40                  45
   His Gln Pro Pro Pro Gln His His Gln Gln Gln Gln His Gln Gln Gln
       50                  55                  60
   Gln Val His Met Gln Pro Gln Lys His Gln Gln Gln Gln Glu Val His
   65                  70                  75                  80
   Val Gln Gln Gln Gln Gln Gln Pro Gln His Gln Gln Gln Gln Gln Gln
                       85                  90                  95
   Gln Gln His Gln Gln Gln His Gln Cys Glu Gly Gln Gln Gln His His
                   100                 105                 110
   Gln Gln Ser Gln Gly His Val Gln Gln His Glu Gln Ser His Glu Gln
               115                 120                 125
   His Gln Gly Gln Ser His Glu Gln Gln His Gln Gln Gln Phe Gln Gly
       130                 135                 140
   His Asp Lys Gln Gln Gln Pro Gln Gln Pro Gln Gln Tyr Gln Gln Gly
   145                 150                 155                 160
   Gln Glu Lys Ser Gln Gln Gln Gln Cys His Cys Gln Glu Gln Gln Gln
                   165                 170                 175
   Thr Thr Arg Cys Ser Tyr Asn Tyr Tyr Ser Ser Ser Ser Asn Leu Lys
               180                 185                 190
   Asn Cys His Glu Phe Leu Arg Gln Gln Cys Ser Pro Leu Val Met Pro
           195                 200                 205
   Phe Leu Gln Ser Arg Leu Ile Gln Pro Ser Ser Cys Gln Val Leu Gln
       210                 215                 220
   Gln Gln Cys Cys His Asp Leu Arg Gln Ile Glu Pro Gln Tyr Ile His
   225                 230                 235                 240
   Gln Ala Ile Tyr Asn Met Val Gln Ser Ile Ile Gln Glu Glu Gln Gln
                   245                 250                 255
   Gln Gln Pro Cys Glu Leu Cys Gly Ser Gln Gln Ala Thr Pro Lys Cys
               260                 265                 270
   Gly Gly Asn Leu Asp Ser Ser Thr Ile Pro Thr Ile Asn Val Arg Leu
           275                 280                 285
   Val Pro Leu Ile Leu Pro Lys
       290                 295
<210> 3
   <211> 950
   <212> DNA
   <213> Zea mays
<220>
   <221> CDS
   <222> (111)...(728)
   <223> 18 kD alpha-globulin/PTA 2274
<400> 3
   aaaaaaaccc cctcgtcgat caccaccaaa gaacacagta actagcagct agcacatcaa      60
   acaagtggcg acagacaaag atttgtgagg gtgatccgcg ctgagaagag atg gct        116
                                                          Met Ala
                                                          1
   aag atc gcc gcg gcg gcg gcg gcg gcg ctg tgc ttc gcg gcc ctg gtg       164
   Lys Ile Ala Ala Ala Ala Ala Ala Ala Leu Cys Phe Ala Ala Leu Val
           5                   10                  15
   gcc gtg gcc gtc tgc caa ggc gag gtc gag cgg cag agg ctc agg gac       212
   Ala Val Ala Val Cys Gln Gly Glu Val Glu Arg Gln Arg Leu Arg Asp
       20                  25                  30
   ctg cag tgc tgg cag gag gtc cag gag agc ccg ctc gac gcg tgc cgc       260
   Leu Gln Cys Trp Gln Glu Val Gln Glu Ser Pro Leu Asp Ala Cys Arg
   35                  40                  45                  50
   cag gtc ctc gac cgg cag cta acc ggc ggc ggc ggc ggc ggc ggc gtt       308
   Gln Val Leu Asp Arg Gln Leu Thr Gly Gly Gly Gly Gly Gly Gly Val
                    55                  60                  65
   ggc ccg ttc cgg tgg ggc acc ggg ctc cgg atg cgg tgc tgc cag cag       356
   Gly Pro Phe Arg Trp Gly Thr Gly Leu Arg Met Arg Cys Cys Gln Gln
               70                  75                  80
   ctc cag gac gtg agc cgc gag tgc cgc tgc gcc gcc atc cgg agc atg       404
   Leu Gln Asp Val Ser Arg Glu Cys Arg Cys Ala Ala Ile Arg Ser Met
           85                  90                  95
   gtc agg ggc tac gag gag gcc atg ccg ccg ctg gag aaa ggc tgg tgg       452
   Val Arg Gly Tyr Glu Glu Ala Met Pro Pro Leu Glu Lys Gly Trp Trp
       100                 105                 110
   cca tgg ggg cgg cag cag cag ccg ccg ccg cag gga gga gga gga gga       500
   Pro Trp Gly Arg Gln Gln Gln Pro Pro Pro Gln Gly Gly Gly Gly Gly
   115                 120                 125                 130
   cag ggg ggc tac tac tac ccc tgc agc cgg cca gga gag gga tac ggc       548
   Gln Gly Gly Tyr Tyr Tyr Pro Cys Ser Arg Pro Gly Glu Gly Tyr Gly
                   135                 140                 145
   tac ggt cag ggt ggc cag cgg cag atg tat cca ccg tgt cgt ccc ggc       596
   Tyr Gly Gln Gly Gly Gln Arg Gln Met Tyr Pro Pro Cys Arg Pro Gly
               150                 155                 160
   acc acc ggc ggc ggg cca agg atc ggc cgc gtg agg ctt acg aag gcc       644
   Thr Thr Gly Gly Gly Pro Arg Ile Gly Arg Val Arg Leu Thr Lys Ala
           165                 170                 175
   cgg gag tac gcc gcg ggg ttg ccg atg atg tgc cgg ctg tcg gag ccc       692
   Arg Glu Tyr Ala Ala Gly Leu Pro Met Met Cys Arg Leu Ser Glu Pro
       180                 185                 190
   cag gag tgc agc atc ttc tcc ggc ggc gac cag tac tagctaccat            738
   Gln Glu Cys Ser Ile Phe Ser Gly Gly Asp Gln Tyr
   195                 200                 205
   ggttaaagcg agtcggcgcg aggtgcaaga cgcagcatgt gtactgtgcg cgtgcaaatc     798
   cagaatgacg tagctctgac gtgggctcgc aatattgtcg cgtgttcgtt acaataatga     858
   taataactat gaggaataaa tatgggaatg ttgccagata gtactggcgc cggttcttca     918
   aaaaaaaaaa aaaaaaaaaa aaaaaaaaaa aa                                   950
<210> 4
   <211> 206
   <212> PRT
   <213> Zea mays
<400> 4
   Met Ala Lys Ile Ala Ala Ala Ala Ala Ala Ala Leu Cys Phe Ala Ala
   1               5                   10                  15
   Leu Val Ala Val Ala Val Cys Gln Gly Glu Val Glu Arg Gln Arg Leu
               20                  25                  30
   Arg Asp Leu Gln Cys Trp Gln Glu Val Gln Glu Ser Pro Leu Asp Ala
           35                  40                  45
   Cys Arg Gln Val Leu Asp Arg Gln Leu Thr Gly Gly Gly Gly Gly Gly
       50                  55                  60
   Gly Val Gly Pro Phe Arg Trp Gly Thr Gly Leu Arg Met Arg Cys Cys
   65                  70                  75                  80
   Gln Gln Leu Gln Asp Val Ser Arg Glu Cys Arg Cys Ala Ala Ile Arg
                   85                  90                  95
   Ser Met Val Arg Gly Tyr Glu Glu Ala Met Pro Pro Leu Glu Lys Gly
               100                 105                 110
   Trp Trp Pro Trp Gly Arg Gln Gln Gln Pro Pro Pro Gln Gly Gly Gly
           115                 120                 125
   Gly Gly Gln Gly Gly Tyr Tyr Tyr Pro Cys Ser Arg Pro Gly Glu Gly
       130                 135                 140
   Tyr Gly Tyr Gly Gln Gly Gly Gln Arg Gln Met Tyr Pro Pro Cys Arg
   145                 150                 155                 160
   Pro Gly Thr Thr Gly Gly Gly Pro Arg Ile Gly Arg Val Arg Leu Thr
                   165                 170                 175
   Lys Ala Arg Glu Tyr Ala Ala Gly Leu Pro Met Met Cys Arg Leu Ser
               180                 185                 190
   Glu Pro Gln Glu Cys Ser Ile Phe Ser Gly Gly Asp Gln Tyr
           195                 200                 205
<210> 5
   <211> 1679
   <212> DNA
   <213> Zea mays
<220>
   <221> CDS
   <222> (34) ... (1485)
   <223> 50 kD legumin-1 prolamin/PTA 2273
<400> 5
   gcacgaggag cgagcgagca gaggcagcgc aca atg gcg gcg gca ata gta ctc      54
                                        Met Ala Ala Ala Ile Val Leu
                                        1               5
   tcc ggc cag gtg cgg ccg ctt ccc tcg tcg ctg ccc ctg tcc ctg ctg      102
   Ser Gly Gln Val Arg Pro Leu Pro Ser Ser Leu Pro Leu Ser Leu Leu
           10                  15                  20
   ctg ctc ctc ctc ctg tgc tgc tcc ggc acc tcg tgg gga tgg agc acg      150
   Leu Leu Leu Leu Leu Cys Cys Ser Gly Thr Ser Trp Gly Trp Ser Thr
       25                  30                  35
   tcc cgg gga gga gcc gcc agg gag tgc ggc ttc gat ggc aag ctg gag      198
   Ser Arg Gly Gly Ala Ala Arg Glu Cys Gly Phe Asp Gly Lys Leu Glu
   40                  45                  50                  55
   gcc ctg gag ccg cgc cac aag gtg cag tct gag gcc ggc tcc gtc cag      246
   Ala Leu Glu Pro Arg His Lys Val Gln Ser Glu Ala Gly Ser Val Gln
                   60                  65                  70
   tac ttc agc cgg ttc aac gaa gcc gac cgg gag ctc acc tgc gcc ggc      294
   Tyr Phe Ser Arg Phe Asn Glu Ala Asp Arg Glu Leu Thr Cys Ala Gly
               75                  80                  85
   atc ttc gcc gtc cgc gtc gtc gtc gac gcc atg ggc ctc ctg ctc cct      342
   Ile Phe Ala Val Arg Val Val Val Asp Ala Met Gly Leu Leu Leu Pro
           90                  95                  100
   cga tac tcc aac gtc cat tcg ctt gtc tac atc gtc caa ggg aga ggg      390
   Arg Tyr Ser Asn Val His Ser Leu Val Tyr Ile Val Gln Gly Arg Gly
       105                 110                 115
   atc att ggg ttc tcg ttt ccg gga tgc caa gag gag acc cag cag cag      438
   Ile Ile Gly Phe Ser Phe Pro Gly Cys Gln Glu Glu Thr Gln Gln Gln
   120                 125                 130                 135
   cag tat gga tac gga tat gga tat gga cac cat cac cat cag cat gac     486
   Gln Tyr Gly Tyr Gly Tyr Gly Tyr Gly His His His His Gln His Asp
                   140                 145                 150
   cac cac aag atc cac cga ttc gag cag ggc gac gtg gtg gcc atg ccg     534
   His His Lys Ile His Arg Phe Glu Gln Gly Asp Val Val Ala Met Pro
               155                 160                 165
   gcc ggc gcc cag cac tgg ctg tac aac gac ggc gac gcg ccg ctt gtg     582
   Ala Gly Ala Gln His Trp Leu Tyr Asn Asp Gly Asp Ala Pro Leu Val
           170                 175                 180
   gcg gtc tac gtc ttc gac gag aac aac aac atc aac cag ctc gag cct     630
   Ala Val Tyr Val Phe Asp Glu Asn Asn Asn Ile Asn Gln Leu Glu Pro
       185                 190                 195
   tcc atg agg aaa ttt ttg ctg gct ggg ggc ttc agc aag ggg cag ccc     678
   Ser Met Arg Lys Phe Leu Leu Ala Gly Gly Phe Ser Lys Gly Gln Pro
   200                 205                 210                 215
   cac ttc gcc gag aac atc ttc aaa ggg atc gac gcc cgg ttc ctg agc     726
   His Phe Ala Glu Asn Ile Phe Lys Gly Ile Asp Ala Arg Phe Leu Ser
                   220                 225                 230
   gaa gcc ctg ggc gtc agc atg cac gtc gcc gag aag ctg cag agc cgg     774
   Glu Ala Leu Gly Val Ser Met His Val Ala Glu Lys Leu Gln Ser Arg
               235                 240                 245
   cgt gac cag cga ggc gag atc gtc cgc gtg gag ccg gag cac ggc ttt     822
   Arg Asp Gln Arg Gly Glu Ile Val Arg Val Glu Pro Glu His Gly Phe
           250                 255                 260
   cac cag ctg aat ccg tcg ccg tcg tcg tcg tcg ttt tcg ttc cca tcg     870
   His Gln Leu Asn Pro Ser Pro Ser Ser Ser Ser Phe Ser Phe Pro Ser
       265                 270                 275
   tca caa gtc cag tac caa acg tgc cag cgc gac gtc gac agg cac aac     918
   Ser Gln Val Gln Tyr Gln Thr Cys Gln Arg Asp Val Asp Arg His Asn
   280                 285                 290                 295
   gtc tgc gcc atg gag gtg agg cac agc gtc gaa cgg ctg gac cag gcc     966
   Val Cys Ala Met Glu Val Arg His Ser Val Glu Arg Leu Asp Gln Ala
                   300                 305                 310
   gac gtc tac agc cct ggg gct ggg agg atc aca cgc ctc acc agc cac    1014
   Asp Val Tyr Ser Pro Gly Ala Gly Arg Ile Thr Arg Leu Thr Ser His
               315                 320                 325
   aag ttc ccc gtc ctc aac ctc gta cag atg agc gcg gtg cgg gta gac    1062
   Lys Phe Pro Val Leu Asn Leu Val Gln Met Ser Ala Val Arg Val Asp
           330                 335                 340
   ctg tac cag gac gcc atc atg tcg ccg ttc tgg aac ttc aac gcc cac    1110
   Leu Tyr Gln Asp Ala Ile Met Ser Pro Phe Trp Asn Phe Asn Ala His
       345                 350                 355
   agc gcc atg tac ggc atc agg ggc agt gca agg gtc cag gtc gcc agc    1158
   Ser Ala Met Tyr Gly Ile Arg Gly Ser Ala Arg Val Gln Val Ala Ser
   360                 365                 370                 375
   gac aac ggg acc acg gtg ttc gac gac gtg ctc cgt gcg ggg cag ctg      1206
   Asp Asn Gly Thr Thr Val Phe Asp Asp Val Leu Arg Ala Gly Gln Leu
                   380                 385                 390
   ctc atc gta ccc cag ggc tac ctc gtc gcc acc aag gcg cag gga gaa      1254
   Leu Ile Val Pro Gln Gly Tyr Leu Val Ala Thr Lys Ala Gln Gly Glu
               395                 400                 405
   ggc ttc cag tac atc gcc ttc gag acg aac cct gac acc atg gtc agc      1302
   Gly Phe Gln Tyr Ile Ala Phe Glu Thr Asn Pro Asp Thr Met Val Ser
           410                 415                 420
   cac gtc gcc ggg aag aac tcc gtc ctg agc gac ttg ccg gcc gcc gtc      1350
   His Val Ala Gly Lys Asn Ser Val Leu Ser Asp Leu Pro Ala Ala Val
       425                 430                 435
   atc gcc agc tcg tat gcc atc tcc atg gag gaa gct gca gag ctc aag      1398
   Ile Ala Ser Ser Tyr Ala Ile Ser Met Glu Glu Ala Ala Glu Leu Lys
   440                 445                 450                 455
   aac ggt agg aag cat gag ctg gct gtg ctt act cct gct ggc agt ggc      1446
   Asn Gly Arg Lys His Glu Leu Ala Val Leu Thr Pro Ala Gly Ser Gly
                   460                 465                 470
   agc tac caa caa ggt caa gct ggc agc gcc caa cag tag gcacaacctc       1495
   Ser Tyr Gln Gln Gly Gln Ala Gly Ser Ala Gln Gln  *
               475                 480
   agagtgatct gcctgaataa gtactcgtag actgtaataa ttaaacaaag cttgctcatg    1555
   gttaaactgc gtgttgatta gtctttcaac tacatagctc taaagttttt gatacaccga    1615
   gtgatttgcc agggaaaaaa tgagcagatt gttgtaagca aaaaaaaaaa aaaaaaaaaa    1675
   aaaa                                                                 1679
<210> 6
   <211> 483
   <212> PRT
   <213> Zea mays
<400> 6
   Met Ala Ala Ala Ile Val Leu Ser Gly Gln Val Arg Pro Leu Pro Ser
   1               5                   10                  15
   Ser Leu Pro Leu Ser Leu Leu Leu Leu Leu Leu Leu Cys Cys Ser Gly
               20                  25                  30
   Thr Ser Trp Gly Trp Ser Thr Ser Arg Gly Gly Ala Ala Arg Glu Cys
           35                  40                  45
   Gly Phe Asp Gly Lys Leu Glu Ala Leu Glu Pro Arg His Lys Val Gln
       50                  55                  60
   Ser Glu Ala Gly Ser Val Gln Tyr Phe Ser Arg Phe Asn Glu Ala Asp
   65                  70                  75                  80
   Arg Glu Leu Thr Cys Ala Gly Ile Phe Ala Val Arg Val Val Val Asp
                   85                  90                  95
   Ala Met Gly Leu Leu Leu Pro Arg Tyr Ser Asn Val His Ser Leu Val
               100                 105                 110
   Tyr Ile Val Gln Gly Arg Gly Ile Ile Gly Phe Ser Phe Pro Gly Cys
           115                 120                 125
   Gln Glu Glu Thr Gln Gln Gln Gln Tyr Gly Tyr Gly Tyr Gly Tyr Gly
       130                 135                 140
   His His His His Gln His Asp His His Lys Ile His Arg Phe Glu Gln
   145                 150                 155                 160
   Gly Asp Val Val Ala Met Pro Ala Gly Ala Gln His Trp Leu Tyr Asn
                   165                 170                 175
   Asp Gly Asp Ala Pro Leu Val Ala Val Tyr Val Phe Asp Glu Asn Asn
               180                 185                 190
   Asn Ile Asn Gln Leu Glu Pro Ser Met Arg Lys Phe Leu Leu Ala Gly
           195                 200                 205
   Gly Phe Ser Lys Gly Gln Pro His Phe Ala Glu Asn Ile Phe Lys Gly
       210                 215                 220
   Ile Asp Ala Arg Phe Leu Ser Glu Ala Leu Gly Val Ser Met His Val
   225                 230                 235                 240
   Ala Glu Lys Leu Gln Ser Arg Arg Asp Gln Arg Gly Glu Ile Val Arg
                   245                 250                 255
   Val Glu Pro Glu His Gly Phe His Gln Leu Asn Pro Ser Pro Ser Ser
               260                 265                 270
   Ser Ser Phe Ser Phe Pro Ser Ser Gln Val Gln Tyr Gln Thr Cys Gln
           275                 280                 285
   Arg Asp Val Asp Arg His Asn Val Cys Ala Met Glu Val Arg His Ser
       290                 295                 300
   Val Glu Arg Leu Asp Gln Ala Asp Val Tyr Ser Pro Gly Ala Gly Arg
   305                 310                 315                 320
   Ile Thr Arg Leu Thr Ser His Lys Phe Pro Val Leu Asn Leu Val Gln
                   325                 330                 335
   Met Ser Ala Val Arg Val Asp Leu Tyr Gln Asp Ala Ile Met Ser Pro
               340                 345                 350
   Phe Trp Asn Phe Asn Ala His Ser Ala Met Tyr Gly Ile Arg Gly Ser
           355                 360                 365
   Ala Arg Val Gln Val Ala Ser Asp Asn Gly Thr Thr Val Phe Asp Asp
       370                 375                 380
   Val Leu Arg Ala Gly Gln Leu Leu Ile Val Pro Gln Gly Tyr Leu Val
   385                 390                 395                 400
   Ala Thr Lys Ala Gln Gly Glu Gly Phe Gln Tyr Ile Ala Phe Glu Thr
                   405                 410                 415
   Asn Pro Asp Thr Met Val Ser His Val Ala Gly Lys Asn Ser Val Leu
               420                 425                 430
   Ser Asp Leu Pro Ala Ala Val Ile Ala Ser Ser Tyr Ala Ile Ser Met
           435                 440                 445
   Glu Glu Ala Ala Glu Leu Lys Asn Gly Arg Lys His Glu Leu Ala Val
       450                 455                 460
   Leu Thr Pro Ala Gly Ser Gly Ser Tyr Gln Gln Gly Gln Ala Gly Ser
   465                 470                 475                 480
   Ala Gln Gln
<210> 7
   <211> 324
   <212> DNA
   <213> Zea mays
<220>
   <221> allele
   <222> (0) ...(0)
   <223> 50kD gamma-zein, B73 partial
<400> 7
   ccagcagcag caacaccaac aacaacaagt tcacatgcaa ccacaaaaac atcagcaaca      60
   acaagaagtt catgttcaac aacaacaaca acaaccgcag caccaacaac aacaacaaca     120
   acaacagcac caacaacaac atcaatgtga aggccaacaa caacatcacc aacaatcaca     180
   aggccatgtg caacaacacg aacagagcca tgagcaacac caaggacaga gccatgagca     240
   acaacatcaa caacaattcc agggtcatga caagcagcaa caaccacaac agcctcagca     300
   atatcagcag ggccaggaaa aatc                                            324
<210> 8
   <211> 321
   <212> DNA
   <213> Zea mays
<220>
   <221> allele
   <222> (0)...(0)
   <223> 50kD gamma-zein, Mo17 partial
<400> 8
   ccagcagcag caacaccaac aacaacaagt tcacatgcaa ccacaaaaac atcagcaaca      60
   acaagaagtt catgttcaac aacaacaaca acaaccgcag caccaacaac aacaacaaca     120
   acagcaccaa caacaacatc aatgtgaagg ccaacaacaa catcaccaac aatcacaagg     180
   ccatgtgcaa caacacgaac agagccatga gcaacaccaa ggacagagcc atgagcaaca     240
   acatcaacaa caattccagg gtcatgacaa gcagcaacaa ccacaacagc ctcagcaata     300
   tcagcagggc caggaaaaat c                                               321
<210> 9
   <211> 561
   <212> DNA
   <213>. Zea mays
<220>
   <221> allele
   <222> (0)...(0)
   <223> 18 kD alpha-globulin, B73, partial
<400> 9
   aattcgccct tgtcattctg gatttgcacg cgcacagtac acatgctgcg tcttgcacgt      60
   cgcgccgact cgctttaacc atggtagcta gtactggtcg ccgccggaga agatgctgca     120
   ctcctggggc tccgacagcc ggcacatcat cggcaacccc gcggcgtact cccgggcctt     180
   cgtaagcctc acgcggccga tccttggccc gccgccggtg gtgccgggac gacacggtgg     240
   atacatctgc cgctggccac cctgaccgta gccgtatccc tctcctggcc ggctgcaggg     300
   gtagtagtag cccccctgtc ctcctcctcc tccctgcggc ggcggctgct gctgccgccc    360
   ccatggccac cagcctttct ccagcggcgg catggcctcc tcgtagcccc tgaccatgct     420
   ccggatggcg gcgcagcggc actcgcggct cacgtcctgg agctgctggc agcaccgcat     480
   ccggagcccg gtgccccacc ggaacgggcc aacgccgccg ccgccgccgc cgccggttag     540
   ctgccggtcg aggaaagggc g                                               561
<210> 10
   <211> 537
   <212> DNA
   <213> Zea mays
<220>
   <221> allele
   <222> (0)...(0)
   <223> 18 kD alpha-globulin, Mo17 partial
<400> 10
   aattcgccct tgtcattctg gatttgcacg cgcacagtac acatgctgcg ccttgcacgt 60
   cgcgccgact cactcttttt tttttaaccc tggtagctag tactggtcgc cgccggagaa 120
   gatgctgcac tcctggggct ccgacagccg gcacatcatc ggcaaccccg cggcgtactc 180
   ccgggccttc gtaagcctca cgcggccgat ccttggcccg gtggtgccgg gacgacacgg 240
   tggatacatc tgcgtttggt atccctctcc tgcccggctg caggggtagt agtagccccc 300
   ctgtcctcct cctcctccct gcggcggcgg ctgctgctgc cgcccccatg gccaccagcc 360
   tttctccaga ggcggcatgg cctcctcgta gcccctgacc atgctccgga tggcggcgca 420
   gcggcactcg cggctcacgt cctggagctg ctggcagcac cgcatccgga gcccggtgcc 480
   ccaccggaac gggccgccga cgccgccgcc ggttagctgc cggtcgagga aagggcg 537
<210> 11
   <211> 23
   <212> DNA
   <213> Artificial Sequence
<220>
   <223> primer 170
<400> 11
   agcgccacct ccacgcatac aag 23
<210> 12
   <211> 26
   <212> DNA
   <213> Artificial Sequence
<220>
   <223> primer 296
<400> 12
   ctagctagcc agcggctata ctacag 26
<210> 13
   <211> 32
   <212> DNA
   <213> Artificial Sequence
<220>
   <223> Mu primer 9242
<400> 13
   agagaagcca acgccawcgc ctcyatttcg tc 32
<210> 14
   <211> 28
   <212> DNA
   <213> Artificial Sequence
<220>
   <223> primer 008
<400> 14
   gtcgaaccag aacagcatga agatggtc 28
<210> 15
   <211> 26
   <212> DNA
   <213> Artificial Sequence
<220>
   <223> primer 009
<400> 15
   gtactggtac tggtagagtc caccca 26
<210> 16
   <211> 1704
   <212> DNA
   <213> Artificial Sequence
<220>
   <221> CDS
   <222> (1) ... (563)
   <223> 19kD alpha-zein D1
<221> CDS
   <222> (564) ... (1101)
   <223> 19kD alpha-zein B1
<221> CDS
   <222> (1102) ... (1711)
   <223> 22kD alpha-zein
<223> fusion silencing construct
<400> 16
   atg gta caa gag gcc atc caa gca agc atc tta cgg tca tta gca tta     48
   Met Val Gln Glu Ala Ile Gln Ala Ser Ile Leu Arg Ser Leu Ala Leu
    1               5                  10                  15
   acc ctc caa caa cca tat gct cta ttg caa cag cca tcc tta gtg cat     96
   Thr Leu Gln Gln Pro Tyr Ala Leu Leu Gln Gln Pro Ser Leu Val His
               20                  25                  30
   ctg tat ctc caa aga atc gcg gca caa caa cta caa caa cag ttg cta    144
   Leu Tyr Leu Gln Arg Ile Ala Ala Gln Gln Leu Gln Gln Gln Leu Leu
           35                  40                  45
   cca aca atc aat caa gta gtt gca gcg aac ctt gct gct tac ctc cag    192
   Pro Thr Ile Asn Gln Val Val Ala Ala Asn Leu Ala Ala Tyr Leu Gln
       50                  55                  60
   caa caa cag ttt ctt cca ttc aat caa cta gct ggg gtg aac cct gct    240
   Gln Gln Gln Phe Leu Pro Phe Asn Gln Leu Ala Gly Val Asn Pro Ala
   65                  70                  75                  80
   atc tac ttg cag gca caa cag cta cta cca ttt aac caa ctt gtc ggg    288
   Ile Tyr Leu Gln Ala Gln Gln Leu Leu Pro Phe Asn Gln Leu Val Gly
                   85                  90                  95
   agc cct tat gcc ttc tta ctg caa caa cag ctt ctg cca ttc cat ctg    336
   Ser Pro Tyr Ala Phe Leu Leu Gln Gln Gln Leu Leu Pro Phe His Leu
               100                 105                 110
   caa gct gtg gca aac att gtt gct ttc ttg aga caa caa cat ttg ttg    384
   Gln Ala Val Ala Asn Ile Val Ala Phe Leu Arg Gln Gln His Leu Leu
           115                 120                 125
   cca ttt tac cca caa gtt gtg gga aac att aat gcc ttc ttg caa cag    432
   Pro Phe Tyr Pro Gln Val Val Gly Asn Ile Asn Ala Phe Leu Gln Gln
       130                 135                 140
   caa caa ttg cta cca ttc tac cca cag aat gtg gca aac att gtt gcc    480
   Gln Gln Leu Leu Pro Phe Tyr Pro Gln Asn Val Ala Asn Ile Val Ala
   145                 150                 155                 160
   ttc tta caa caa caa caa ttg ctg cca ttt agc caa cat gct ttg acg    528
   Phe Leu Gln Gln Gln Gln Leu Leu Pro Phe Ser Gln His Ala Leu Thr
                   165                 170                 175
   aat cct acc acc tta ttg caa cct cga gtc caa ca g gca atc gca gct   576
   Asn Pro Thr Thr Leu Leu Gln Pro Arg Val Gln Gln   Ala Ile Ala Ala
               180                   185                    190
   ggc atc tta cct tta tca ccc ttg ttc ctc caa caa tca tca gcc cta     624
   Gly Ile Leu Pro Leu Ser Pro Leu Phe Leu Gln Gln Ser Ser Ala Leu
           195                 200                 205
   tta cag cag tta cct ttg gtg cat tta ttg gca caa aac atc agg gca     672
   Leu Gln Gln Leu Pro Leu Val His Leu Leu Ala Gln Asn Ile Arg Ala
       210                 215                 220
   caa caa cta caa caa ctt gtg cta gca aac ctt gct gcc tac tct cag     720
   Gln Gln Leu Gln Gln Leu Val Leu Ala Asn Leu Ala Ala Tyr Ser Gln
   225                 230                 235                 240
   caa caa cag ttt ctt cca ttc aac caa cta gct gca ttg aac tct gct     768
   Gln Gln Gln Phe Leu Pro Phe Asn Gln Leu Ala Ala Leu Asn Ser Ala
                   245                 250                 255
   tct tat ttg caa caa caa caa cta cca ttc agc cag cta tct gct gcc     816
   Ser Tyr Leu Gln Gln Gln Gln Leu Pro Phe Ser Gln Leu Ser Ala Ala
               260                 265                 270
   tac ccc cag caa ttt ctt cca ttc aac caa ctg aca gct ttg aac tct     864
   Tyr Pro Gln Gln Phe Leu Pro Phe Asn Gln Leu Thr Ala Leu Asn Ser
           275                 280                 285
   cct gct tat tta cag cag caa caa cta cta cca ttc agc cag cta gct     912
   Pro Ala Tyr Leu Gln Gln Gln Gln Leu Leu Pro Phe Ser Gln Leu Ala
       290                 295                 300
   ggt gtg agc cct gct acc ttc ttg aca caa cca caa ttg ttg ccg ttc     960
   Gly Val Ser Pro Ala Thr Phe Leu Thr Gln Pro Gln Leu Leu Pro Phe
   305                 310                 315                 320
   tac cag cac gct gcg cct aac gct ggc acc ctc tta caa ctg caa caa    1008
   Tyr Gln His Ala Ala Pro Asn Ala Gly Thr Leu Leu Gln Leu Gln Gln
                   325                 330                 335
   ttg ctg cca ttc aac caa ctt gct ttg aca aac cca aca gca ttc tac    1056
   Leu Leu Pro Phe Asn Gln Leu Ala Leu Thr Asn Pro Thr Ala Phe Tyr
               340                 345                 350
   caa caa ccc atc att ggt ggt gcc ctc ttt tac ccg ctt gcg gcg        1101
   Gln Gln Pro Ile Ile Gly Gly Ala Leu Phe Tyr Pro Leu Ala Ala
           355                 360                 365
   agc gcc tta caa caa cca att gcc caa ttg caa caa caa tcc ttg gca    1149
   Ser Ala Leu Gln Gln Pro Ile Ala Gln Leu Gln Gln Gln Ser Leu Ala
           370                 375                 380
   cat cta acc cta caa acc att gca acg caa caa caa caa caa cag ttt    1197
   His Leu Thr Leu Gln Thr Ile Ala Thr Gln Gln Gln Gln Gln Gln Phe
       385                 390                 395
   ctg cca tca ctg agc cac cta gcc gtg gtg aac cct gtc acc tac ttg    1245
   Leu Pro Ser Leu Ser His Leu Ala Val Val Asn Pro Val Thr Tyr Leu
   400                 405                 410                 415
   caa cag cag ctg ctt gca tcc aac cca ctt gct ctg gcg aac gta gct    1293
   Gln Gln Gln Leu Leu Ala Ser Asn Pro Leu Ala Leu Ala Asn Val Ala
                   420                 425                 430
   gca tac cag caa caa caa cag ctg caa cag ttt atg cca gtg ctc agt    1341
   Ala Tyr Gln Gln Gln Gln Gln Leu Gln Gln Phe Met Pro Val Leu Ser
               435                     440                     445
   caa cta gcc atg gtg aac cct gcc gtc tac cta caa cta ctt tca tct    1389
   Gln Leu Ala Met Val Asn Pro Ala Val Tyr Leu Gln Leu Leu Ser Ser
           450                     455                     460
   agc ccg ctc gcg gtg ggc aat gca cct acg tac cta caa caa cag ttg    1437
   Ser Pro Leu Ala Val Gly Asn Ala Pro Thr Tyr Leu Gln Gln Gln Leu
       465                     470                     475
   ctg caa caa att gta cca gct ctg act cag cta gct gtg gca aac cct    1485
   Leu Gln Gln Ile Val Pro Ala Leu Thr Gln Leu Ala Val Ala Asn Pro
   480                     485                     490                     495
   gct gcc tac tta caa cag ttg ctt cca ttc aac caa ctg gct gtg tca    1533
   Ala Ala Tyr Leu Gln Gln Leu Leu Pro Phe Asn Gln Leu Ala Val Ser
                   500                     505                     510
   aac tct gct gcg tac cta caa cag cga caa cag tta ctt aat cca ttg    1581
   Asn Ser Ala Ala Tyr Leu Gln Gln Arg Gln Gln Leu Leu Asn Pro Leu
               515                     520                     525
   gca gtg gct aac cca ttg gtc gct acc ttc ctg cag cag caa caa caa    1629
   Ala Val Ala Asn Pro Leu Val Ala Thr Phe Leu Gln Gln Gln Gln Gln
           530                     535                     540
   ttg ctg cca tac aac cag ttc tct ttg atg aac cct gcc ttg cag caa    1677
   Leu Leu Pro Tyr Asn Gln Phe Ser Leu Met Asn Pro Ala Leu Gln Gln
       545                     550                     555
   ccc atc gtt gga ggt gcc atc ttt tag                                1704
   Pro Ile Val Gly Gly Ala Ile Phe  *   *   *
   560                     565
<210> 17
   <211> 1637
   <212> DNA
   <213> Artificial Sequence
<220>
   <223> 27kD gamma-zein
<223> 22kD alpha-zein
<223> 19kD alpha-zein B1
<223> 19kD alpha-zein D1
<223> LKR
<400> 17
   aacagccgca tccaagcccg tgccagctgc agggaacctg cggcgttggc agcaccccga 60
   tcctgggcca gtgcgtcgag ttcctgaggc atcagtgcag cccgacggcg acgccctact 120
   gctcgcctca gtgccagtcg ttgcggcagc agtgttgcca gcagctcagg caggtggagc 180
   cgcagcaccg gtaccaggcg atcttcggct tggtcctcca gtccatcctg cagcagcagc 240
   cgcaaagcgg ccaggtcgcg gggctgttgg cggcgcagat agcgcagcaa ctgacggcga 300
   tgtagcggcc ccaattgcaa caacaatcct tggcacatct aaccctacaa accattgcaa 360
   cgcaacaaca acaacaacag tttctgccat cactgagcca cctagccgtg gtgaaccctg 420
   tcacctactt gcaacagcag ctgcttgcat ccaacccact tgctctggcg aacgtagctg 480
   cataccagca acaacaacag ctgcaacagt ttatgccagt gctcagtcaa ctagccatgg 540
   tgaaccctgc cgtctaccta caactacttt catctagccg tcgagactac cattcagcca 600
   gctatctgct gcctaccccc agcaatttct tccattcaac caactgacag ctttgaactc 660
   tcctgcttat ttacagcagc aacaactact accattcagc cagctagctg gtgtgagccc 720
   tgctaccttc ttgacacaac cacaattgtt gccgttctac cagcacgctg cgcctaacgc 780
   tggcaccctc ttacaactgc aacaattgca ctagcggagc caagattttt gccctccttg 840
   ccctccttgc tctttcagca agcgctgcta cctcgacttt tattccacaa tgctcacaac 900
   aatacctctc tccggtgaca gccgcgggat ttcaataccc aactatacaa tcctacatgg 960
   tacaagaggc catccaagca agcatcttac ggtcattagc attaaccctc caacaaccat 1020
   atgctctatt gcaacagcca tccttagtgc atctgtatct ccaaagaatc gccccggttt 1080
   agagcacaag gaggatccat catgacgctc agtatgagga tgcaggatgc gagatttcag 1140
   aagacctgtc agaatgcggc cttatcatag gcatcaaaca acccaagctg cagatgattc 1200
   tttcagatag agcgtacgct ttcttttcac acacacacaa agcccaaaaa gagaatatgc 1260
   cactgttaga caagatcctt gaagaagggg tgtccttgtt tgattatgag ctaattgttg 1320
   gagatgatgg gaaaagatca ctagcatttg ggaaatttgc tggtagagct ggactgatag 1380
   atttcttaca tggtctcgga cagcgatatt tgagccttgg atactcgact ccatttctct 1440
   ctctgggaca atctcatatg tatccttcgc tcgctgcagc caaggctgca gtcattgtcg 1500
   ttgcagaaga gatagcaaca tttggacttc catccggaat ttgtccgata gtgtttgtgt 1560
   tcactggagt tggaaacgtc tctcagggtg cgcaggagat attcaagtta ttgccccata 1620
   cctttgttga tgctgag                                                1637
<210> 18
   <211> 537
   <212> DNA
   <213> Zea mays
<220>
   <221> CDS
   <222> (1)...(537)
<400> 18
   atg aag atg gtc atc gtt ctc gtc gtg tgc ctg gct ctg tca gct gcc     48
   Met Lys Met Val Ile Val Leu Val Val Cys Leu Ala Leu Ser Ala Ala
    1               5                  10                  15
   agc gcc tct gca atg cag atg ccc tgc ccc tgc gcg ggg ctg cag ggc     96
   Ser Ala Ser Ala Met Gln Met Pro Cys Pro Cys Ala Gly Leu Gln Gly
               20                  25                  30
   ttg tac ggc gct ggc gcc ggc ctg acg acg atg atg ggc gcc ggc ggg    144
   Leu Tyr Gly Ala Gly Ala Gly Leu Thr Thr Met Met Gly Ala Gly Gly
           35                  40                  45
   ctg tac ccc tac gcg gag tac ctg agg cag ccg cag tgc agc ccg ctg    192
   Leu Tyr Pro Tyr Ala Glu Tyr Leu Arg Gln Pro Gln Cys Ser Pro Leu
       50                  55                  60
   gcg gcg gcg ccc tac tac gcc ggg tgt ggg cag ccg agc gcc atg ttc    240
   Ala Ala Ala Pro Tyr Tyr Ala Gly Cys Gly Gln Pro Ser Ala Met Phe
   65                  70                  75                  80
   cag ccg ctc cgg caa cag tgc tgc cag cag cag atg agg atg atg gac    288
   Gln Pro Leu Arg Gln Gln Cys Cys Gln Gln Gln Met Arg Met Met Asp
                   85                  90                  95
   gtg cag tcc gtc gcg cag cag ctg cag atg atg atg cag ctt gag cgt    336
   Val Gln Ser Val Ala Gln Gln Leu Gln Met Met Met Gln Leu Glu Arg
               100                 105                 110
   gcc gct gcc gcc agc agc agc ctg tac gag cca gct ctg atg cag cag    384
   Ala Ala Ala Ala Ser Ser Ser Leu Tyr Glu Pro Ala Leu Met Gln Gln
           115                 120                 125
   cag cag cag ctg ctg gca gcc cag ggt ctc aac ccc atg gcc atg atg    432
   Gln Gln Gln Leu Leu Ala Ala Gln Gly Leu Asn Pro Met Ala Met Met
       130                 135                 140
   atg gcg cag aac atg ccg gcc atg ggt gga ctc tac cag tac cag ctg    480
   Met Ala Gln Asn Met Pro Ala Met Gly Gly Leu Tyr Gln Tyr Gln Leu
   145                 150                 155                 160
   ccc agc tac cgc acc aac ccc tgt ggc gtc tcc gct gcc att ccg ccc    528
   Pro Ser Tyr Arg Thr Asn Pro Cys Gly Val Ser Ala Ala Ile Pro Pro
                   165                 170                 175
   tac tac tga                                                        537
   Tyr Tyr  *
<210> 19
   <211> 178
   <212> PRT
   <213> Zea mays
<400> 19
   Met Lys Met Val Ile Val Leu Val Val Cys Leu Ala Leu Ser Ala Ala
    1               5                  10                  15
   Ser Ala Ser Ala Met Gln Met Pro Cys Pro Cys Ala Gly Leu Gln Gly
               20                  25                  30
   Leu Tyr Gly Ala Gly Ala Gly Leu Thr Thr Met Met Gly Ala Gly Gly
           35                  40                  45
   Leu Tyr Pro Tyr Ala Glu Tyr Leu Arg Gln Pro Gln Cys Ser Pro Leu
       50                  55                  60
   Ala Ala Ala Pro Tyr Tyr Ala Gly Cys Gly Gln Pro Ser Ala Met Phe
   65                  70                  75                  80
   Gln Pro Leu Arg Gln Gln Cys Cys Gln Gln Gln Met Arg Met Met Asp
                   85                  90                  95
   Val Gln Ser Val Ala Gln Gln Leu Gln Met Met Met Gln Leu Glu Arg
               100                 105                 110
   Ala Ala Ala Ala Ser Ser Ser Leu Tyr Glu Pro Ala Leu Met Gln Gln
           115                 120                 125
   Gln Gln Gln Leu Leu Ala Ala Gln Gly Leu Asn Pro Met Ala Met Met
       130                 135                 140
   Met Ala Gln Asn Met Pro Ala Met Gly Gly Leu Tyr Gln Tyr Gln Leu
   145                 150                 155                 160
   Pro Ser Tyr Arg Thr Asn Pro Cys Gly Val Ser Ala Ala Ile Pro Pro
                   165                 170                 175
   Tyr Tyr
<210> 20
   <211> 636
   <212> DNA
   <213> Zea mays
<220>
   <221> CDS
   <222> (1)...(636)
<400> 20
   atg gca gcc aag atg ttt gca ttg ttt gcg ctc cta gct ctt tgt gca      48
   Met Ala Ala Lys Met Phe Ala Leu Phe Ala Leu Leu Ala Leu Cys Ala
   1               5                   10                  15
   acc gcc act agt gct acc cat atc cca ggg cac ttg tca cca cta ctg      96
   Thr Ala Thr Ser Ala Thr His Ile Pro Gly His Leu Ser Pro Leu Leu
               20                  25                  30
   atg cca ttg gct acc atg aac cct tgg atg cag tac tgc atg aag caa     144
   Met Pro Leu Ala Thr Met Asn Pro Trp Met Gln Tyr Cys Met Lys Gln
           35                  40                  45
   cag ggg gtt gcc aac ttg tta gcg tgg ccg acc ctg atg ctg cag caa     192
   Gln Gly Val Ala Asn Leu Leu Ala Trp Pro Thr Leu Met Leu Gln Gln
       50                  55                  60
   ctg ttg gcc tca ccg ctt cag cag tgc cag atg cca atg atg atg ccg     240
   Leu Leu Ala Ser Pro Leu Gln Gln Cys Gln Met Pro Met Met Met Pro
   65                  70                  75                  80
   ggt atg atg cca ccg atg acg atg atg ccg atg ccg agt atg atg cca     288
   Gly Met Met Pro Pro Met Thr Met Met Pro Met Pro Ser Met Met Pro
                   85                  90                  95
   tcg atg atg gtg ccg act atg atg tca cca atg acg atg gct agt atg     336
   Ser Met Met Val Pro Thr Met Met Ser Pro Met Thr Met Ala Ser Met
               100                 105                 110
   atg ccg ccg atg atg atg cca agc atg att tca cca atg acg atg ccg     384
   Met Pro Pro Met Met Met Pro Ser Met Ile Ser Pro Met Thr Met Pro
           115                 120                 125
   agt atg atg cct tcg atg ata atg ccg acc atg atg tca cca atg att     432
   Ser Met Met Pro Ser Met Ile Met Pro Thr Met Met Ser Pro Met Ile
       130                 135                 140
   atg ccg agt atg atg cca cca atg atg atg ccg agc atg gtg tca cca     480
   Met Pro Ser Met Met Pro Pro Met Met Met Pro Ser Met Val Ser Pro
   145                 150                 155                 160
   atg atg atg cca aac atg atg aca gtg cca caa tgt tac tct ggt tct     528
   Met Met Met Pro Asn Met Met Thr Val Pro Gln Cys Tyr Ser Gly Ser
                   165                 170                 175
   atc tca cac att ata caa caa caa caa tta cca ttc atg ttc agc ccc     576
   Ile Ser His Ile Ile Gln Gln Gln Gln Leu Pro Phe Met Phe Ser Pro
               180                 185                 190
   aca gca atg gcg atc cca ccc atg ttc tta cag cag ccc ttt gtt ggt     624
   Thr Ala Met Ala Ile Pro Pro Met Phe Leu Gln Gln Pro Phe Val Gly
           195                 200                 205
   gct gca ttc tag                                                     636
   Ala Ala Phe  *
       210
<210> 21
   <211> 211
   <212> PRT
   <213> Zea mays
<400> 21
   Met Ala Ala Lys Met Phe Ala Leu Phe Ala Leu Leu Ala Leu Cys Ala
    1               5                  10                  15
   Thr Ala Thr Ser Ala Thr His Ile Pro Gly His Leu Ser Pro Leu Leu
               20                  25                  30
   Met Pro Leu Ala Thr Met Asn Pro Trp Met Gln Tyr Cys Met Lys Gln
           35                  40                  45
   Gln Gly Val Ala Asn Leu Leu Ala Trp Pro Thr Leu Met Leu Gln Gln
       50                  55                  60
   Leu Leu Ala Ser Pro Leu Gln Gln Cys Gln Met Pro Met Met Met Pro
   65                  70                  75                  80
   Gly Met Met Pro Pro Met Thr Met Met Pro Met Pro Ser Met Met Pro
                   85                  90                  95
   Ser Met Met Val Pro Thr Met Met Ser Pro Met Thr Met Ala Ser Met
               100                 105                 110
   Met Pro Pro Met Met Met Pro Ser Met Ile Ser Pro Met Thr Met Pro
           115                 120                 125
   Ser Met Met Pro Ser Met Ile Met Pro Thr Met Met Ser Pro Met Ile
       130                 135                 140
   Met Pro Ser Met Met Pro Pro Met Met Met Pro Ser Met Val Ser Pro
   145                 150                 155                 160
   Met Met Met Pro Asn Met Met Thr Val Pro Gln Cys Tyr Ser Gly Ser
                   165                 170                 175
   Ile Ser His Ile Ile Gln Gln Gln Gln Leu Pro Phe Met Phe Ser Pro
               180                 185                 190
   Thr Ala Met Ala Ile Pro Pro Met Phe Leu Gln Gln Pro Phe Val Gly
           195                 200                 205
   Ala Ala Phe
       210
<210> 22
   <211> 1623
   <212> DNA
   <213> Sorghum bicolor
<220>
   <221> CDS
   <222> (91) ... (1610)
<400> 22
   ctccaccgcg gtggcggccg ctctagaact agtggatccc ccgggctgca ggcagcactc  60
   tgtcagtgaa gagagtgagt gagcagaagc aat ggc ggc cgc ggc gtc act ctc  114
                                    Asn Gly Gly Arg Gly Val Thr Leu
                                     1               5
   cgg caa gct gct gtt tcc ctc gtc gct gtg cct ctg cct tct cct cct   162
   Arg Gln Ala Ala Val Ser Leu Val Ala Val Pro Leu Pro Ser Pro Pro
       10                  15                  20
   gtg ctg ctc cgg cgc cgg cgg cgc agc agc cag cag ctc atg ggg ggc   210
   Val Leu Leu Arg Arg Arg Arg Arg Ser Ser Gln Gln Leu Met Gly Gly
    25                  30                  35                  40
   gtc ccg ggg agg agc cgc cag gga gtg cgg ctt cga cgg caa gct gga   258
   Val Pro Gly Arg Ser Arg Gln Gly Val Arg Leu Arg Arg Gln Ala Gly
                    45                  50                  55
   ggc cct gga gcc gcg cca caa ggc gca gtc cga ggc cgg ctc cgt cga   306
   Gly Pro Gly Ala Ala Pro Gln Gly Ala Val Arg Gly Arg Leu Arg Arg
               60                  65                  70
   gta ctt cag ccg gtt cac cga agc cga ccg gga gct cac ctg cgc tgg   354
   Val Leu Gln Pro Val His Arg Ser Arg Pro Gly Ala His Leu Arg Trp
           75                  80                  85
   cct ctt cgc cgt ccg tgt cgt cgt cga cgc ctt ggg cct cgt gct tcc   402
   Pro Leu Arg Arg Pro Cys Arg Arg Arg Arg Leu Gly Pro Arg Ala Ser
       90                  95                  100
   tcg cta ctc caa cct cca ttc gct tgt cta cat cgc cca agg gag agg   450
   Ser Leu Leu Gln Pro Pro Phe Ala Cys Leu His Arg Pro Arg Glu Arg
   105                 110                 115                 120
   gat tat tgg gtt ctc gtt tcc ggg atg cca aga aga gac cca cca tca   498
   Asp Tyr Trp Val Leu Val Ser Gly Met Pro Arg Arg Asp Pro Pro Ser
                   125                 130                 135
   gca gca gta tgg ata cgg ata tgg ata tga aca tca tca tca gcg ccc   546
   Ala Ala Val Trp Ile Arg Ile Trp Ile  *  Thr Ser Ser Ser Ala Pro
               140                 145                     150
   tga cga gca tca caa gat cca ccg att cca aca ggg aga tgt ggt cgc   594
    *  Arg Ala Ser Gln Asp Pro Pro Ile Pro Thr Gly Arg Cys Gly Arg
               155                 160                 165
   cat gcc cgc cgg tgc cca gca ctg gct gta caa cga cgg cga tac gcc   642
   His Ala Arg Arg Cys Pro Ala Leu Ala Val Gln Arg Arg Arg Tyr Ala
               170                 175                 180
   gct tgt ggc gat cta cgt ctt cga cac aaa caa caa cat caa cca gct   690
   Ala Cys Gly Asp Leu Arg Leu Arg His Lys Gln Gln His Gln Pro Ala
           185                 190                 195
   tga gcc ttc cat gag gaa gtt ctt gct ggc tgg ggg att cag cag ggg   738
    *  Ala Phe His Glu Glu Val Leu Ala Gly Trp Gly Ile Gln Gln Gly
           200                 205                 210
   gca gcc cca ctt cgc cga gaa cat ctt taa agg aat cga cgc ccg gtt   786
   Ala Ala Pro Leu Arg Arg Glu His Leu  *  Arg Asn Arg Arg Pro Val
       215                 220                 225
   cct gag cga agc ctt ggg tgt cag cat gca agt cgc tga gaa gct tca   834
   Pro Glu Arg Ser Leu Gly Cys Gln His Ala Ser Arg  *  Glu Ala Ser
       230                 235                 240
   gag ccg gcg tga aca gcg agg cga gat agt ccg tgt gga gct gga gca   882
   Glu Pro Ala  *  Thr Ala Arg Arg Asp Ser Pro Cys Gly Ala Gly Ala
       245                 250                 255
   tgg cct tca cct gct caa tcc acc acc gcc gtc gtt tcc atc act aca   930
   Trp Pro Ser Pro Ala Gln Ser Thr Thr Ala Val Val Ser Ile Thr Thr
       260                 265                 270
   aga cca gta cca gca tca cca aac atg cca acg cga caa cag tcg taa   978
   Arg Pro Val Pro Ala Ser Pro Asn Met Pro Thr Arg Gln Gln Ser  *
   275                 280                 285
   cat ctg cac cat gga ggt gag gca cag cgt cga acg cct gga tca ggc   1026
   His Leu His His Gly Gly Glu Ala Gln Arg Arg Thr Pro Gly Ser Gly
   290                 295                 300                 305
   cga tgt cta cag ccc tgg tgc tgg aag gat cac acg cct gac cag cca    1074
   Arg Cys Leu Gln Pro Trp Cys Trp Lys Asp His Thr Pro Asp Gln Pro
                   310                 315                 320
   caa gtt ccc aat tct caa cct cat aca gat gag cgc agt tcg agt aga    1122
   Gln Val Pro Asn Ser Gln Pro His Thr Asp Glu Arg Ser Ser Ser Arg
               325                 330                 335
   cct gta tca gga cgc cat cct gtc acc gtt ctg gaa ctt caa cgc cca    1170
   Pro Val Ser Gly Arg His Pro Val Thr Val Leu Glu Leu Gln Arg Pro
           340                 345                 350
   cag tgc cat gta cac cat cag agg ctg tgc cag ggt tca ggt cgc cag    1218
   Gln Cys His Val His His Gln Arg Leu Cys Gln Gly Ser Gly Arg Gln
       355                 360                 365
   cga caa cgg gac gac ggt gtt cga cgg cgt gct tcg tgc tgg gca gct    1266
   Arg Gln Arg Asp Asp Gly Val Arg Arg Arg Ala Ser Cys Trp Ala Ala
   370                 375                 380                 385
   gct cat cat acc cca ggg cta cct tgt cgc cac caa ggc gca agg aga    1314
   Ala His His Thr Pro Gly Leu Pro Cys Arg His Gln Gly Ala Arg Arg
                   390                 395                 400
   agg gtt tca gta cat ctc ctt cga gac gaa cca taa ctc cat ggt cag    1362
   Arg Val Ser Val His Leu Leu Arg Asp Glu Pro  *  Leu His Gly Gln
               405                 410                 415
   cca cat cgc cgg gaa gaa ctc cct ctt gag cga ttt gcc ggt cgg cgt    1410
   Pro His Arg Arg Glu Glu Leu Pro Leu Glu Arg Phe Ala Gly Arg Arg
               420                 425                 430
   cat cgc cag ctc cta tgg cgt ctc gat gga gga agc tgc aga gct gaa    1458
   His Arg Gln Leu Leu Trp Arg Leu Asp Gly Gly Ser Cys Arg Ala Glu
           435                 440                 445
   gaa cag tag gaa gca tga gct cgc tgt gtt tac tac tcc tcc tgg tgg    1506
   Glu Gln  *  Glu Ala  *  Ala Arg Cys Val Tyr Tyr Ser Ser Trp Trp
       450                 455                 460
   cag cta tga tca agg tca tgt tgg cag cgc cca aca gta ggc acc tga    1554
   Gln Leu  *  Ser Arg Ser Cys Trp Gln Arg Pro Thr Val Gly Thr  *
               465                 470                 475
   gag tga tct acc tga ata agt act cgt gga ctg taa taa aca aag ctt    1602
   Glu  *  Ser Thr  *  Ile Ser Thr Arg Gly Leu  *   *  Thr Lys Leu
                   480                 485
   gtt cat gg gtaaaaaaaa aaa                                          1623
   Val His
       490
<210> 23
   <211> 490
   <212> PRT
   <213> Sorghum bicolor
<400> 23
   Asn Gly Gly Arg Gly Val Thr Leu Arg Gln Ala Ala Val Ser Leu Val
   1               5                   10                  15
   Ala Val Pro Leu Pro Ser Pro Pro Val Leu Leu Arg Arg Arg Arg Arg
               20                  25                  30
   Ser Ser Gln Gln Leu Met Gly Gly Val Pro Gly Arg Ser Arg Gln Gly
           35                  40                  45
   Val Arg Leu Arg Arg Gln Ala Gly Gly Pro Gly Ala Ala Pro Gln Gly
       50                  55                  60
   Ala Val Arg Gly Arg Leu Arg Arg Val Leu Gln Pro Val His Arg Ser
   65                  70                  75                  80
   Arg Pro Gly Ala His Leu Arg Trp Pro Leu Arg Arg Pro Cys Arg Arg
                   85                  90                  95
   Arg Arg Leu Gly Pro Arg Ala Ser Ser Leu Leu Gln Pro Pro Phe Ala
               100                 105                 110
   Cys Leu His Arg Pro Arg Glu Arg Asp Tyr Trp Val Leu Val Ser Gly
           115                 120                 125
   Met Pro Arg Arg Asp Pro Pro Ser Ala Ala Val Trp Ile Arg Ile Trp
       130                 135                 140
   Ile Thr Ser Ser Ser Ala Pro Arg Ala Ser Gln Asp Pro Pro Ile Pro
   145                 150                 155                 160
   Thr Gly Arg Cys Gly Arg His Ala Arg Arg Cys Pro Ala Leu Ala Val
                   165                 170                 175
   Gln Arg Arg Arg Tyr Ala Ala Cys Gly Asp Leu Arg Leu Arg His Lys
               180                 185                 190
   Gln Gln His Gln Pro Ala Ala Phe His Glu Glu Val Leu Ala Gly Trp
           195                 200                 205
   Gly Ile Gln Gln Gly Ala Ala Pro Leu Arg Arg Glu His Leu Arg Asn
       210                 215                 220
   Arg Arg Pro Val Pro Glu Arg Ser Leu Gly Cys Gln His Ala Ser Arg
   225                 230                 235                 240
   Glu Ala Ser Glu Pro Ala Thr Ala Arg Arg Asp Ser Pro Cys Gly Ala
                   245                 250                 255
   Gly Ala Trp Pro Ser Pro Ala Gln Ser Thr Thr Ala Val Val Ser Ile
               260                 265                 270
   Thr Thr Arg Pro Val Pro Ala Ser Pro Asn Met Pro Thr Arg Gln Gln
           275                 280                 285
   Ser His Leu His His Gly Gly Glu Ala Gln Arg Arg Thr Pro Gly Ser
       290                 295                 300
   Gly Arg Cys Leu Gln Pro Trp Cys Trp Lys Asp His Thr Pro Asp Gln
   305                 310                 315                 320
   Pro Gln Val Pro Asn Ser Gln Pro His Thr Asp Glu Arg Ser Ser Ser
                   325                 330                 335
   Arg Pro Val Ser Gly Arg His Pro Val Thr Val Leu Glu Leu Gln Arg
               340                 345                 350
   Pro Gln Cys His Val His His Gln Arg Leu Cys Gln Gly Ser Gly Arg
           355                 360                 365
   Gln Arg Gln Arg Asp Asp Gly Val Arg Arg Arg Ala Ser Cys Trp Ala
       370                 375                 380
   Ala Ala His His Thr Pro Gly Leu Pro Cys Arg His Gln Gly Ala Arg
   385                 390                 395                 400
   Arg Arg Val Ser Val His Leu Leu Arg Asp Glu Pro Leu His Gly Gln
                   405                 410                 415
   Pro His Arg Arg Glu Glu Leu Pro Leu Glu Arg Phe Ala Gly Arg Arg
               420                 425                 430
   His Arg Gln Leu Leu Trp Arg Leu Asp Gly Gly Ser Cys Arg Ala Glu
           435                 440                 445
   Glu Gln Glu Ala Ala Arg Cys Val Tyr Tyr Ser Ser Trp Trp Gln Leu
       450                 455                 460
   Ser Arg Ser Cys Trp Gln Arg Pro Thr Val Gly Thr Glu Ser Thr Ile
   465                 470                 475                 480
   Ser Thr Arg Gly Leu Thr Lys Leu Val His
                   485                 490
<210> 24
   <211> 1712
   <212> DNA
   <213> Saccharum officinale
<220>
   <221> CDS
   <222> (100)...(1677)
<400> 24
   cgcctgcagg taccggtccg gaattcccgg gtcgacccac gcgtccgccc acgcgtccgc 60
   tgtcgtcagt cgtcactgaa gagagtgagc agaagcaac aat ggc ggc ggc act    114
                                             Asn Gly Gly Gly Thr
                                              1               5
   ctc cgg caa gct gct gcc gct tcc ctc ctc cct gtg cct gtg cct gct    162
   Leu Arg Gln Ala Ala Ala Ala Ser Leu Leu Pro Val Pro Val Pro Ala
                   10                  15                  20
   tct cct cct gtg ctg ctc cgg ctc tgg cgc cgg cgc agc cag cag ctc    210
   Ser Pro Pro Val Leu Leu Arg Leu Trp Arg Arg Arg Ser Gln Gln Leu
               25                  30                  35
   atg ggg ggc gtc ccg ggg agg agc cgc cag gga gtg cgg ttt cga cga    258
   Met Gly Gly Val Pro Gly Arg Ser Arg Gln Gly Val Arg Phe Arg Arg
           40                  45                  50
   caa gct gga ggc cct gga gcc gcg cca caa ggt gca gtc cga ggc cgg    306
   Gln Ala Gly Gly Pro Gly Ala Ala Pro Gln Gly Ala Val Arg Gly Arg
       55                  60                  65
   ctc cgt cga gta ctt cag ccg att cac cga agc cga ccg gga gct cac    354
   Leu Arg Arg Val Leu Gln Pro Ile His Arg Ser Arg Pro Gly Ala His
   70                  75                  80                  85
   ctg cgc cgg cat ctt cgc cgt ccg cgt cgt cgt gga cgc ctt ggg cct    402
   Leu Arg Arg His Leu Arg Arg Pro Arg Arg Arg Gly Arg Leu Gly Pro
                   90                  95                  100
   cct tct tcc tcg cta ctc caa cct cca ttc tct ggt cta cat cat aca    450
   Pro Ser Ser Ser Leu Leu Gln Pro Pro Phe Ser Gly Leu His His Thr
               105                 110                 115
   agg gag agg gat tat tgg gtt ctc gtt tcc ggg atg cca aga aga gac    498
   Arg Glu Arg Asp Tyr Trp Val Leu Val Ser Gly Met Pro Arg Arg Asp
           120                 125                 130
   cca cca tca gca gca gta tgc ata cgg ata tgg ata tga aca tca tca    546
   Pro Pro Ser Ala Ala Val Cys Ile Arg Ile Trp Ile  *  Thr Ser Ser
       135                 140                 145
   tca tca gcg ccc tga cga gca tca caa gat cca ccg att cga aca ggg    594
   Ser Ser Ala Pro  *  Arg Ala Ser Gln Asp Pro Pro Ile Arg Thr Gly
       150                 155                 160
   aga cgt ggt ggc cat gcc ggc cgg tgc tca gca ctg gct gta caa cga    642
   Arg Arg Gly Gly His Ala Gly Arg Cys Ser Ala Leu Ala Val Gln Arg
   165                  170                  175
   cgg caa tgc gcc gct tgt ggc gat cta cgt ctt cga cac aaa caa caa    690
   Arg Gln Cys Ala Ala Cys Gly Asp Leu Arg Leu Arg His Lys Gln Gln
   180                 185                 190                 195
   cat caa cca gct tga gcc ttc cat gag gaa gtt ctt gct ggc tgg ggg    738
   His Gln Pro Ala  *  Ala Phe His Glu Glu Val Leu Ala Gly Trp Gly
                       200                 205                 210
   att cag caa ggg gca gat cca ctt cgc cga gaa cat ctt taa agg aat    786
   Ile Gln Gln Gly Ala Asp Pro Leu Arg Arg Glu His Leu  *  Arg Asn
                   215                 220                 225
   cga cgc ccg gtt cct gag cga agc cct ggg tgt cag cat gaa tgt cac    834
   Arg Arg Pro Val Pro Glu Arg Ser Pro Gly Cys Gln His Glu Cys His
                   230                 235                 240
   taa gaa gct tca gag ccg aca tga cca gcg ggg cga aat agt ccg tgt    882
    *  Glu Ala Ser Glu Pro Thr  *  Pro Ala Gly Arg Asn Ser Pro Cys
                   245                 250                 255
   gga gct gga gca tgg cct tca cct cct gaa tcc acc atc gtc gtc gtc    930
   Gly Ala Gly Ala Trp Pro Ser Pro Pro Glu Ser Thr Ile Val Val Val
                   260                 265                 270
   att tcc atc act aca aga cca gta cca aca tca cca aac atg tca acg    978
   Ile Ser Ile Thr Thr Arg Pro Val Pro Thr Ser Pro Asn Met Ser Thr
               275                 280                 285
   cga cga cag cca taa cat ctg cgc cat ggc ggt gag gca cag cgt cga    1026
   Arg Arg Gln Pro  *  His Leu Arg His Gly Gly Glu Ala Gln Arg Arg
           290                 295                 300
   acg cct tga tca ggc cga cgt cta cag ccc tgg tgc tgg gag gat cac    1074
   Thr Pro  *  Ser Gly Arg Arg Leu Gln Pro Trp Cys Trp Glu Asp His
               305                 310                 315
   acg cct gac cag cca caa gtt ccc aat tct caa cct cat aca gat gag    1122
   Thr Pro Asp Gln Pro Gln Val Pro Asn Ser Gln Pro His Thr Asp Glu
           320                 325                 330
   cgc ggt gcg agt aga cct ata tca gga tgc cat cct gtc gcc gtt ctg    1170
   Arg Gly Ala Ser Arg Pro Ile Ser Gly Cys His Pro Val Ala Val Leu
       335                 340                 345
   gaa ctt caa cgc cca cag cgc cat gta cac cat cag agg ctg tgc cag    1218
   Glu Leu Gln Arg Pro Gln Arg His Val His His Gln Arg Leu Cys Gln
   350                 355                 360                 365
   ggt tca ggt cgc cag cga caa tgg gac gac cgt gtt cga cgg cgt gct    1266
   Gly Ser Gly Arg Gln Arg Gln Trp Asp Asp Arg Val Arg Arg Arg Ala
                   370                 375                 380
   tcg tcc tgg gca gct gtt cat cat acc cca ggg cta cct tgt cgc cac    1314
   Ser Ser Trp Ala Ala Val His His Thr Pro Gly Leu Pro Cys Arg His
               385                 390                 395
   caa ggc gca agg aga agg gtt cca gta cat atc cat cga gat gaa ccc    1362
   Gln Gly Ala Arg Arg Arg Val Pro Val His Ile His Arg Asp Glu Pro
           400                 405                 410
   caa ctc cat ggt cag cca cat tgc cgg gaa gaa ctc cgt ctt cag caa    1410
   Gln Leu His Gly Gln Pro His Cys Arg Glu Glu Leu Arg Leu Gln Gln
       415                 420                 425
   ttt gcc ggt cgg cat cat cgc cag ctc gta tgg cgt ctc cat gga gga    1458
   Phe Ala Gly Arg His His Arg Gln Leu Val Trp Arg Leu His Gly Gly
   430                 435                 440                     445
   agc tgc aga gct gaa gaa cag tag aaa gca tga gct tgc tgt gtt tac    1506
   Ser Cys Arg Ala Glu Glu Gln  *  Lys Ala  *  Ala Cys Cys Val Tyr
                   450                 455
   tcc tgg tgg cag cta tga tca agg tca tgt tgg cag cgc cca aca gta    1554
   Ser Trp Trp Gln Leu  *  Ser Arg Ser Cys Trp Gln Arg Pro Thr Val
   460                 465                 470
   ggc acc tta gag tga tct gct tga ata agt tat cgt gga ctg taa taa    1602
   Gly Thr Leu Glu  *  Ser Ala  *  Ile Ser Tyr Arg Gly Leu  *   *
   475                 480                     485
   aca aag ctt gtt cat ggt taa act gca tgt ctg cat gga tga atc ttt    1650
   Thr Lys Leu Val His Gly  *  Thr Ala Cys Leu His Gly  *  Ile Phe
               490                 495                     500
   caa cta cat agc tcg tca aat aaa aca actgaactga agtgagtaat          1697
   Gln Leu His Ser Ser Ser Asn Lys Thr
                   505
   gtttcaaaaa aaaaa                                                   1712
<210> 25
   <211> 509
   <212> PRT
   <213> Saccharum officinale
<400> 25
   Asn Gly Gly Gly Thr Leu Arg Gln Ala Ala Ala Ala Ser Leu Leu Pro
   1               5                   10                  15
   Val Pro Val Pro Ala Ser Pro Pro Val Leu Leu Arg Leu Trp Arg Arg
               20                  25                  30
   Arg Ser Gln Gln Leu Met Gly Gly Val Pro Gly Arg Ser Arg Gln Gly
           35                  40                  45
   Val Arg Phe Arg Arg Gln Ala Gly Gly Pro Gly Ala Ala Pro Gln Gly
       50                  55                  60
   Ala Val Arg Gly Arg Leu Arg Arg Val Leu Gln Pro Ile His Arg Ser
   65                  70                  75                  80
   Arg Pro Gly Ala His Leu Arg Arg His Leu Arg Arg Pro Arg Arg Arg
                   85                  90                  95
   Gly Arg Leu Gly Pro Pro Ser Ser Ser Leu Leu Gln Pro Pro Phe Ser
               100                 105                 110
   Gly Leu His His Thr Arg Glu Arg Asp Tyr Trp Val Leu Val Ser Gly
           115                 120                 125
   Met Pro Arg Arg Asp Pro Pro Ser Ala Ala Val Cys Ile Arg Ile Trp
       130                 135                 140
   Ile Thr Ser Ser Ser Ser Ala Pro Arg Ala Ser Gln Asp Pro Pro Ile
   145                 150                 155                 160
   Arg Thr Gly Arg Arg Gly Gly His Ala Gly Arg Cys Ser Ala Leu Ala
                   165                 170                 175
   Val Gln Arg Arg Gln Cys Ala Ala Cys Gly Asp Leu Arg Leu Arg His
               180                 185                 190
   Lys Gln Gln His Gln Pro Ala Ala Phe His Glu Glu Val Leu Ala Gly
   195 200 205
   Trp Gly Ile Gln Gln Gly Ala Asp Pro Leu Arg Arg Glu His Leu Arg
   210 215 220
   Asn Arg Arg Pro Val Pro Glu Arg Ser Pro Gly Cys Gln His Glu Cys
   225 230 235 240
   His Glu Ala Ser Glu Pro Thr Pro Ala Gly Arg Asn Ser Pro Cys Gly
   245 250 255
   Ala Gly Ala Trp Pro Ser Pro Pro Glu Ser Thr Ile Val Val Val Ile
   260 265 270
   Ser Ile Thr Thr Arg Pro Val Pro Thr Ser Pro Asn Met Ser Thr Arg
   275 280 285
   Arg Gln Pro His Leu Arg His Gly Gly Glu Ala Gln Arg Arg Thr Pro
   290 295 300
   Ser Gly Arg Arg Leu Gln Pro Trp Cys Trp Glu Asp His Thr Pro Asp
   305 310 315 320
   Gln Pro Gln Val Pro Asn Ser Gln Pro His Thr Asp Glu Arg Gly Ala
   325 330 335
   Ser Arg Pro Ile Ser Gly Cys His Pro Val Ala Val Leu Glu Leu Gln
   340 345 350
   Arg Pro Gln Arg His Val His His Gln Arg Leu Cys Gln Gly Ser Gly
   355 360 365
   Arg Gln Arg Gln Trp Asp Asp Arg Val Arg Arg Arg Ala Ser Ser Trp
   370 375 380
   Ala Ala Val His His Thr Pro Gly Leu Pro Cys Arg His Gln Gly Ala
   385 390 395 400
   Arg Arg Arg Val Pro Val His Ile His Arg Asp Glu Pro Gln Leu His
   405 410 415
   Gly Gln Pro His Cys Arg Glu Glu Leu Arg Leu Gln Gln Phe Ala Gly
   420 425 430
   Arg His His Arg Gln Leu Val Trp Arg Leu His Gly Gly Ser Cys Arg
   435 440 445
   Ala Glu Glu Gln Lys Ala Ala Cys Cys Val Tyr Ser Trp Trp Gln Leu
   450 455 460
   Ser Arg Ser Cys Trp Gln Arg Pro Thr Val Gly Thr Leu Glu Ser Ala
   465 470 475 480
   Ile Ser Tyr Arg Gly Leu Thr Lys Leu Val His Gly Thr Ala Cys Leu
   485 490 495
   His Gly Ile Phe Gln Leu His Ser Ser Ser Asn Lys Thr
   500 505
<210> 26
   <211> 1510
   <212> DNA
   <213> Zea mays
<220>
   <221> promoter
   <222> (1)...(1510)
   <223> GZ-W64A promoter
<400> 26
   ttatataatt tataagctga aacaacccgg ccctaaagca ctatcgtatc acctatctga 60
   aataagtcac gggtttcgaa cgtccacttg cgtcgcacgg aattgcatgt ttcttgttgg 120
   aagcatattc acgcaatctc cacacataaa ggtttatgta taaacttaca tttagctcag 180
   tttaattaca gtcttatttg gatgcatatg tatggttctc aatccatata agttagagta 240
   aaaaataagt ttaaatttta tcttaattca ctccaacata tatggattga gtacaatact 300
   catgtgcatc caaacaaact acttatattg aggtgaattt ggatagaaat taaactaact 360
   tacacactaa gccaatcttt actatattaa agcaccagtt tcaacgatcg tcccgcgtca 420
   atattattaa aaaactccta catttcttta taatcaaccc gcactcttat aatctcttct 480
   ctactactat aataagagag tttatgtaca aaataaggtg aaattatgta taagtgttct 540
   ggatattggt tgttggctcc atattcacac aacctaatca atagaaaaca tatgttttat 600
   taaaacaaaa tttatcatat atcatatata tatatataca tatatatata taaaccgtag 660
   caatgcacgg gcatataact agtgcaactt aatacatgtg tgtattaaga tgaataagag 720
   ggtatccaaa taaaaaactt gttcgcttac gtctggatcg aaaggggttg gaaacgatta 780
   aatctcttcc tagtcaaaat tgaatagaag gagatttaat ctctcccaat ccccttcgat 840
   catccaggtg caaccgtata agtcctaaag tggtgaggaa cacgaaacaa ccatgcattg 900
   gcatgtaaag ctccaagaat ttgttgtatc cttaacaact cacagaacat caaccaaaat 960
   tgcacgtcaa gggtattggg taagaaacaa tcaaacaaat cctctctgtg tgcaaagaaa 1020
   cacggtgagt catgccgaga tcatactcat ctgatataca tgcttacagc tcacaagaca 1080
   ttacaaacaa ctcatattgc attacaaaga tcgtttcatg aaaaataaaa taggccggac 1140
   aggacaaaaa tccttgacgt gtaaagtaaa tttacaacaa aaaaaaagcc atatgtcaag 1200
   ctaaatctaa ttcgttttac gtagatcaac aacctgtaga aggcaacaaa actgagccac 1260
   gcagaagtac agaatgattc cagatgaacc atcgacgtgc tacgtaaaga gagtgacgag 1320
   tcatatacat ttggcaagaa accatgaagc tgcctacagc cgtctcggtg gcataagaac 1380
   acaagaaatt gtgttaatta atcaaagcta taaataacgc tcgcatgcct gtgcacttct 1440
   ccatcaccac cactgggtct tcagaccatt agctttatct actccagagc gcagaagaac 1500
   ccgatcgaca                                                        1510
<210> 27
   <211> 492
   <212> DNA
   <213> Zea mays
<220>
   <221> terminator
   <222> (1)...(492)
   <223> GZ-W64A terminator
<400> 27
   gatccccggc ggtgtccccc actgaagaaa ctatgtgctg tagtatagcc gctgcccgct 60
   ggctagctag ctagttgagt catttagcgg cgatgattga gtaataatgt gtcacgcatc 120
   accatgcatg ggtggcagtg tcagtgtgag caatgacctg aatgaacaat tgaaatgaaa 180
   agaaaaaagt attgttccaa attaaacgtt ttaacctttt aataggttta tacaataatt 240
   gatatatgtt ttctgtatat gtctaatttg ttatcatcca tttagatata gacaaaaaaa 300
   atctaagaac taaaacaaat gctaatttga aatgaaggga gtatatattg ggataatgtc 360
   gatgagatcc ctcgtaatat caccgacatc acacgtgtcc agttaatgta tcagtgatac 420
   gtgtattcac atttgttgcg cgtaggcgta cccaacaatt ttgatcgact atcagaaagt 480
   caacggaagc ga                                                     492